(19) Europäisches Patentamt
European Patent Office
Office européen des brevets

(11) EP 1 830 841 B1

(12) **EUROPEAN PATENT SPECIFICATION**

(45) Date of publication and mention of the grant of the patent:
**02.07.2008 Bulletin 2008/27**

(21) Application number: **05854587.2**

(22) Date of filing: **16.12.2005**

(51) Int Cl.:
*A61K 31/4015* (2006.01) *C07D 207/27* (2006.01)
*C07D 211/76* (2006.01) *C07D 401/12* (2006.01)
*A61K 31/4025* (2006.01) *A61K 31/4412* (2006.01)
*A61P 3/04* (2006.01)

(86) International application number:
**PCT/US2005/045907**

(87) International publication number:
**WO 2006/068992 (29.06.2006 Gazette 2006/26)**

(54) **CYCLOALKYL LACTAM DERIVATIVES AS INHIBITORS OF 11-BETA-HYDROXYSTEROID DEHYDROGENASE 1**

CYCLOALKYL-LACTAM-DERIVATE ALS INHIBITOREN VON 11-BETA-HYDROXYSTEROIDDEHYDROGENASE 1

DERIVES DE CYCLOALKYL-LACTAME UTILISES EN TANT QU'INHIBITEURS DE LA 11-BETA-HYDROXYSTEROIDE DESHYDROGENASE 1

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.12.2004 US 637488 P**

(43) Date of publication of application:
**12.09.2007 Bulletin 2007/37**

(73) Proprietor: **ELI LILLY AND COMPANY**
**Indianapolis, Indiana 46285 (US)**

(72) Inventors:
- **AICHER, Thomas, Daniel**
  **Superior, Colorado 80027 (US)**
- **CHICARELLI, Mark, Joseph**
  **Longmont, Colorado 80501 (US)**
- **GAUTHIER, Cassandra, A.**
  **Longmont, Colorado 80501 (US)**
- **HINKLIN, Ronald, Jay**
  **Longmont, Colorado 80503 (US)**
- **TIAN, Hongqi**
  **Longmont, Colorado 80501 (US)**
- **WALLACE, Owen, Brendan**
  **Zionsville, Indiana 46077 (US)**
- **KRASUTSKY, Alexei, Pavlovych**
  **Zionsville, Indiana 46077 (US)**
- **ALLEN, John, Gordon**
  **Newbury Park, California 91320 (US)**

(74) Representative: **Smith, Andrew George et al**
**Eli Lilly and Company Limited**
**European Patent Operations**
**Lilly Research Centre**
**Erl Wood Manor**
**Windlesham, Surrey GU20 6PH (GB)**

(56) References cited:
**WO-A-02/26706**
**WO-A-20/04033427**
**WO-A-20/04041776**
**WO-A-20/05108361**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 830 841 B1

## Description

This application claims the benefit of U.S. Provisional Patent Application No. 60/637,488, filed December 20, 2004.

Diabetes is caused by multiple factors and is most simply characterized by elevated levels of plasma glucose (hyperglycemia) in the fasting state. There are two generally recognized forms of diabetes: Type 1 diabetes, or insulin-dependent diabetes mellitus (IDDM), in which patients produce little or no insulin, the hormone which regulates glucose utilization, and Type 2 diabetes, or noninsulin-dependent diabetes mellitus (NIDDM), wherein patients produce insulin and even exhibit hyperinsulinemia (plasma insulin levels that are the same or even elevated in comparison with non-diabetic subjects), while at the same time demonstrating hyperglycemia. Type 1 diabetes is typically treated with exogenous insulin administered via injection. However, Type 2 diabetics often develop "insulin resistance", such that the effect of insulin in stimulating glucose and lipid metabolism in the main insulin-sensitive tissues, namely, muscle, liver, and adipose tissues, is diminished. Patients who are insulin resistant but not diabetic have elevated insulin levels that compensate for their insulin resistance, so that serum glucose levels are not elevated. In patients with NIDDM, the plasma insulin levels, even when they are elevated, are insufficient to overcome the pronounced insulin resistance, resulting in hyperglycemia.

Insulin resistance is primarily due to a receptor signaling defect that is not yet completely understood. Resistance to insulin results in insufficient activation of glucose uptake, diminished oxidation of glucose and storage of glycogen in muscle, inadequate insulin repression of lipolysis in adipose tissue, and inadequate glucose production and secretion by the liver.

Persistent or uncontrolled hyperglycemia that occurs in diabetics is associated with increased morbidity and premature mortality. Abnormal glucose homeostasis is also associated both directly and indirectly with obesity, hypertension, and alterations in lipid, lipoprotein, and apolipoprotein metabolism. Type 2 diabetics are at increased risk of developing cardiovascular complications, e.g., atherosclerosis, coronary heart disease, stroke, peripheral vascular disease, hypertension, nephropathy, neuropathy, and retinopathy. Therefore, therapeutic control of glucose homeostasis, lipid metabolism, obesity, and hypertension are critically important in the clinical management and treatment of diabetes mellitus.

Many patients who have insulin resistance but have not developed Type 2 diabetes are also at risk of developing "Syndrome X" or "metabolic syndrome". Syndrome X or metabolic syndrome is a condition characterized by insulin resistance, along with abdominal obesity, hyper insulinemia, high blood pressure, low HDL and High VLDL. These patients, whether or not they develop overt diabetes mellitus, are at increased risk of developing the cardiovascular complications listed above.

Evidence in rodents and humans links 11-beta hydroxysteroid dehydrogenase 1 ("11-β-HSD1") to metabolic syndrome. Evidence suggests that a drug which specifically inhibits 11-β-HSD1 in type 2 obese diabetic patients will lower blood glucose by reducing hepatic gluconeogenesis, reduce central obesity, improve atherogenic lipoprotein phenotypes, lower blood pressure, and reduce insulin resistance. Insulin effects in muscle will be enhanced, and insulin secretion from the beta cells of the islet may also be increased.

There is a continuing need for new methods of treating diabetes and related conditions, such as metabolic syndrome. It is an object of this invention to meet this and other needs.

## SUMMARY OF THE INVENTION

The present invention provides a compound structurally represented by formula I:

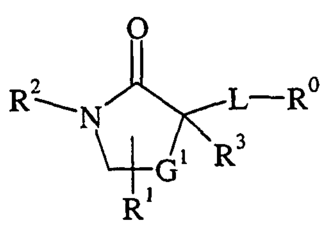

(I)

or a pharmaceutically acceptable salt thereof wherein

$R^0$ is

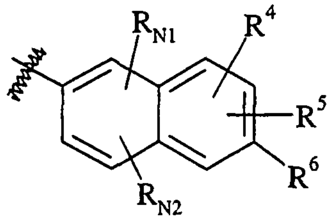 or 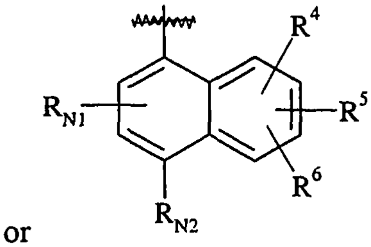 ;

wherein the zigzag line represents the point of attachment to the $R^0$ position in formula I;

$G^1$ is methylene or ethylene;

L is $-(C_1-C_4)$alkylene-, -S-, -CH(OH)-, or -O-;

$R^1$ is hydrogen, hydroxy, $-(C_1-C_4)$alkyl(optionally substituted with one to three halogens), $-(C_1-C_4)$ alkoxy(optionally substituted with one to three halogens), or $-CH_2OR^7$, wherein $R^7$ is hydrogen or $-(C_1-C_4)$alkyl;

$R^2$ is

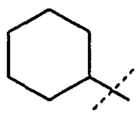 , 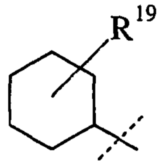 , 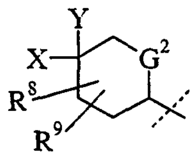 , 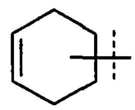 , 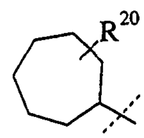 ,

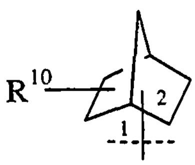 , 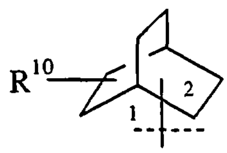 , 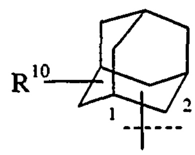 , 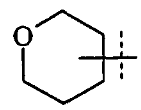 , 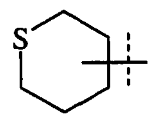 ,

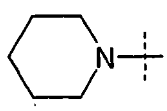 , 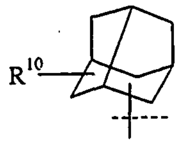 , 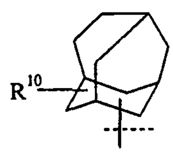 , or 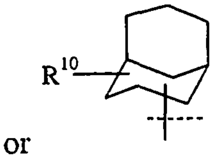 ;

wherein the dashed line represents the point of attachment to the $R^2$ position in formula I; wherein X is hydrogen, hydroxy, $-OCH_3$ or $-CH_2OH$; wherein Y is hydrogen or methyl, provided that at least one of X and Y is not hydrogen; and wherein optionally X and Y together with the carbon to which they are attached form carbonyl; and wherein $R^8$ is independently hydrogen, hydroxy, $-(C_1-C_4)$alkyl(optionally substituted with one to three halogens); $R^9$ is independently hydrogen, hydroxy, or $-(C_1-C_4)$alkyl(optionally substituted with one to three halogens); $R^{10}$ is independently at each occurrence hydrogen, hydroxy, or $-(C_1-C_4)$alkyl (optionally substituted with one to three halogens); and $G^2$ is methylene, ethylene, or 1-propylene;

$R^3$ is hydrogen, hydroxy, (provided that when L is -S- or -CH(OH)- then $R^3$ is not hydroxy), or $-(C_1-C_4)$alkyl(optionally substituted with one to three halogens);

$R_{N1}$ is hydrogen, hydroxy, halo, $-(C_1-C_4)$alkyl(optionally substituted with one to three halogens), or $-(C_1-C_4)$alkoxy(optionally substituted with one to three halogens);

$R_{N2}$ is hydrogen, hydroxy, halo, $-(C_1-C_4)$alkyl(optionally substituted with one to three halogens), $-(C_1-C_4)$alkoxy(optionally substituted with one to three halogens), $Ar^1$, or $Ar^2$;

$R^4$ is hydrogen, halo, hydroxy, $-(C_1-C_4)$alkyl(optionally substituted with one to three halogens), $-(C_1-C_4)$alkoxy(optionally substituted with one to three halogens), cyano;

$R^5$ is hydrogen, hydroxy, -($C_1$-$C_4$)alkyl(optionally substituted with one to three halogens), -($C_1$-$C_4$)alkoxy(optionally substituted with one to three halogens), halo, cyano, -$SCF_3$, -($C_1$-$C_4$)alkyl-C(O)OH, -($C_1$-$C_4$)alkyl-C(O)O($C_1$-$C_4$)alkyl, -($C_1$-$C_4$)alkyl-OH, -O-($C_1$-$C_4$)alkyl-C(O)O($C_1$-$C_4$)alkyl, -C(O)O($C_1$-$C_4$)alkyl, -$OSO_2CF_3$, -($C_2$-$C_4$)alkenyl, $Ar^1$, $Ar^2$, or -($C_1$-$C_4$)alkyl-C(O)N($R^{11}$)($R^{12}$); wherein $R^{11}$ and $R^{12}$ are each independently hydrogen or -($C_1$-$C_4$)alkyl, or $R^{11}$ and $R^{12}$ taken together with the nitrogen to which they are attached form piperidinyl or pyrrolidinyl;

$R^6$ is hydrogen, hydroxy, halo, -($C_1$-$C_4$)alkyl(optionally substituted with one to three halogens), -($C_1$-$C_4$)alkoxy(optionally substituted with one to three halogens), -C(O)O($C_1$-$C_4$)alkyl, $Ar^1$, $Het^1$, $Ar^2$, $Het^2$, -$Ar^1$-($C_1$-$C_4$)alkyl, -$Het^1$-($C_1$-$C_4$)alkyl, -O-($C_1$-$C_4$ alkyl)-$Ar^2$, -$Ar^2$-($C_1$-$C_4$)alkyl, -$Het^2$-($C_1$-$C_4$)alkyl, -C(O)-$Ar^2$, -C(O)-$Het^2$, or -O($C_1$-$C_4$)alkyl-N($R^{13}$)($R^{14}$); wherein $R^{13}$ and $R^{14}$ are each independently hydrogen or -($C_1$-$C_4$)alkyl, or $R^{13}$ and $R^{14}$ taken together with the nitrogen to which they are attached form piperidinyl or pyrrolidinyl; or

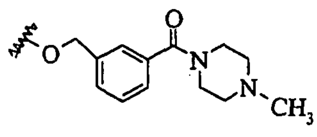 ;

wherein the zigzag line represents the point of attachment to $Ar^1$;

$Ar^1$ is phenyl or naphthyl;

$Ar^2$ is $Ar^1$ optionally substituted with from one to three moieties selected from halo, hydroxy, cyano, -($C_1$-$C_4$)alkyl(optionally substituted with one to three halogens), -($C_1$-$C_4$ alkyl)C(O)OH, -O($C_1$-$C_4$)alkyl-C(O)OH, -C(O)O-($C_1$-$C_4$)alkyl, -$NH_2$, -C(O)OH, -($C_1$-$C_4$)alkyl-N($R^{15}$)($R^{16}$), -O($C_1$-$C_4$)alkyl-N($R^{15}$)($R^{16}$), imidazolyl, pyridinyl, or -($C_1$-$C_4$)alkyl-imidazolyl; wherein $R^{15}$ and $R^{16}$ are each independently hydrogen or -($C_1$-$C_4$)alkyl or $R^{15}$ and $R^{16}$ taken together with the nitrogen to which they are attached form piperidinyl or pyrrolidinyl;

$Het^1$ is a heterocyclic radical selected from pyridinyl, piperidinyl, pyrimidinyl, pyrazinyl, piperazinyl, pyridazinyl, indolyl, isoindolyl, indolinyl, furanyl, benzofuranyl, thiazolyl, oxazolyl, isoxazolyl, isothiazolyl, benzothiophenyl, thiophenyl, quinolinyl, isoquinolinyl, quinoxalinyl, quinazolinyl, or phthalazinyl;

$Het^2$ is $Het^1$ optionally substituted with from one to three moieties selected from halo, hydroxy, cyano, -$CF_3$, -($C_1$-$C_4$)alkyl, -($C_1$-$C_4$)alkoxy, -($C_1$-$C_4$)alkyl-C(O)OH, -O($C_1$-$C_4$)alkyl-C(O)OH, -C(O)O($C_1$-$C_4$)alkyl, -($C_1$-$C_4$)alkyl-N($R^{17}$)($R^{18}$), -O($C_1$-$C_4$)alkyl-N($R^{17}$)($R^{18}$), imidazolyl, pyridinyl, or -($C_1$-$C_4$)alkyl-imidazolyl; wherein $R^{17}$ and $R^{18}$ are each independently hydrogen or -($C_1$-$C_4$)alkyl or $R^{17}$ and $R^{18}$ taken together with the nitrogen to which they are attached form piperidinyl or pyrrolidinyl; and

$R^{19}$ and $R^{20}$ are each independently hydroxy, -($C_1$-$C_4$)alkyl(optionally substituted with one to three halogens), or -$CH_2OH$.

The present invention provides compounds of formula I that are useful as potent and selective inhibitors of 11-beta hydroxysteroid dehydrogenase 1. The present invention further provides a pharmaceutical composition which comprises a compound of Formula I, or a pharmaceutical salt thereof, and a pharmaceutically acceptable carrier, diluent, or excipient. In addition, the present invention provides the use of a present compound for the manufacture of a medicament for the treatment of metabolic syndrome, and related disorders, which comprise administering to a patient in need thereof an effective amount of a compound of formula I or a pharmaceutically acceptable salt thereof.

Due to their inhibition of 11-beta hydroxysteroid dehydrogenase 1, the present compounds are useful in the treatment of a wide range of conditions and disorders in which inhibition of 11-beta hydroxysteroid dehydrogenase 1 is beneficial. These disorders and conditions are defined herein as "diabetic disorders" and "metabolic syndrome disorders". One of skill in the art is able to identify "diabetic disorders" and "metabolic syndrome disorders" by the involvement of 11-beta hydroxysteroid dehydrogenase 1 activity either in the pathophysiology of the disorder, or in the homeostatic response to the disorder. Thus, the compounds may find use for example to prevent, treat, or alleviate, diseases or conditions or associated symptoms or sequelae, of "Diabetic disorders" and "metabolic syndrome disorders". "Diabetic disorders" and "metabolic syndrome disorders"include, but are not limited to, diabetes, type 1 diabetes, type 2 diabetes, hyperglycemia, hyper insulinemia, beta-cell rest, improved beta-cell function by restoring first phase response, prandial hyperglycemia, preventing apoptosis, impaired fasting glucose (IFG), metabolic syndrome, hypoglycemia, hyper-/hypokalemia, normalizing glucagon levels, improved LDL/HDL ratio, reducing snacking, eating disorders, weight loss, polycystic ovarian syndrome (PCOS), obesity as a consequence of diabetes, latent autoimmune diabetes in adults (LADA), insulitis, islet transplantation, pediatric diabetes, gestational diabetes, diabetic late complications, micro-/macroalbuminuria, nephropathy, retinopathy, neuropathy, diabetic foot ulcers, reduced intestinal motility due to glucagon administration, short bowel syndrome, antidiarrheic, increasing gastric secretion, decreased blood flow, erectile dysfunction, glaucoma, post surgical stress, ameliorating organ tissue injury caused by reperfusion of blood flow after ischemia, ischemic heart damage, heart insufficiency, congestive heart failure, stroke, myocardial infarction, arrhythmia, premature death, wound healing, impaired glucose tolerance (IGT), insulin resistance syndromes, syndrome X, hyperlipidemia, dyslipidemia, hypertriglyceridemia, hyperlipoproteinemia, hypercholesterolemia, arteriosclerosis including atherosclerosis, glucagonomas, acute pancreatitis, cardiovascular diseases, hypertension, cardiac hypertrophy, gastrointestinal disorders, obesity, diabetes as a consequence of obesity, diabetic dyslipidemia, etc. Thus, the present invention also provides the use of a present compound for the manufacture of a medicament useful for treatment of "Diabetic disorders" and "metabolic syndrome disorders" while reducing and or eliminating one or more of the unwanted side effects associated with the current treatments.

## DETAILED DESCRIPTION OF THE INVENTION

General terms used in the description of compounds herein described bear their usual meanings.

As used herein, the term "$(C_1-C_4)$alkyl" refers to straight-chain or branched-chain saturated aliphatic groups of 1 to 4 carbon atoms including methyl, ethyl, n-propyl, isopropyl, n-butyl, isobutyl, sec-butyl, t-butyl, and the like.

Similarly, the term "$(C_1-C_4)$alkoxy" represents a $C_1-C_4$ alkyl group attached through an oxygen atom and examples include methoxy, ethoxy, n-propoxy, isopropoxy, and the like.

The term "$-(C_1-C_4)$alkylene-" refers to straight-chain or branched-chain saturated divalent aliphatic groups such as methylene, ethylene, n-propylene, gemdimethyl methylene, and the like.

The term "$(C_2-C_4)$ alkenyl" means hydrocarbon chains of the indicated number of carbon atoms of either a straight or branched configuration having at least one carbon-carbon double bond which may occur at any point along the chain, such as ethenyl, propenyl, butenyl, 2-butenyl and the like.

The term "halogen" refers to fluoro, chloro, bromo, and iodo.

"$HET^1$" and "$HET^2$" may be attached at any point which affords a stable structure.

The term "optionally substituted," or "optional substituents," as used herein, means that the groups in question are either unsubstituted or substituted with one or more of the substituents specified. When the groups in question are substituted with more than one substituent, the substituents may be the same or different. The terms "independently," "independently are," and "independently selected from" mean that the groups in question may be the same or different. Certain of the herein defined terms may occur more than once in the structural formulae, and upon such occurrence each term shall be defined independently of the other.

As used herein, the term "patient" refers to a warm-blooded animal or mammal that has or is at risk of developing a disease selected from (1) through (20) described below. It is understood that guinea pigs, dogs, cats, rats, mice, hamsters, and primates, including humans, are examples of patients within the scope of the meaning of the term "patient". The term "patient" includes and livestock animals. Livestock animals are animals raised for food production. Ruminants or "cud-chewing" animals such as cows, bulls, heifers, steers, sheep, buffalo, bison, goats and antelopes are examples of livestock. Other examples of livestock include pigs and avians (poultry) such as chickens, ducks, turkeys and geese. Yet other examples of livestock include fish, shellfish and crustaceans raised in aquaculture. Also included are exotic animals used in food production such as alligators, water buffalo and ratites (e.g., emu, rheas or ostriches). The patient to be treated is preferably a mammal, in particular a human being.

The terms "treatment", "treating" and "treat", as used herein, include their generally accepted meanings, i.e., the management and care of a patient for the purpose of preventing, reducing the risk in incurring or developing a given condition or disease, prohibiting, restraining, alleviating, ameliorating, slowing, stopping, delaying, or reversing the progression or severity, and holding in check and/or treating existing characteristics, of a disease, disorder, or pathological condition, described herein, including the alleviation or relief of symptoms or complications, or the cure or elimination of the disease, disorder, or condition. The present method includes both medical therapeutic and/or prophylactic treatment, as appropriate.

As used herein, the term "therapeutically effective amount" means an amount of compound of the present invention that is capable of alleviating the symptoms of the various pathological conditions herein described. The specific dose of a compound administered according to this invention will, of course, be determined by the particular circumstances surrounding the case including, for example, the compound administered, the route of administration, the state of being of the patient, and the pathological condition being treated.

"Composition" means a pharmaceutical composition and is intended to encompass a pharmaceutical product comprising the active ingredient(s) including compound(s) of Formula I, and the inert ingredient(s) that make up the

carrier. Accordingly, the pharmaceutical compositions of the present invention encompass any composition made by admixing a compound of the present invention and a pharmaceutically acceptable carrier.

The term "suitable solvent" refers to any solvent, or mixture of solvents, inert to the ongoing reaction that sufficiently solubilizes the reactants to afford a medium within which to effect the desired reaction.

The term "unit dosage form" means physically discrete units suitable as unitary dosages for human subjects and other non-human animals, each unit containing a predetermined quantity of active material calculated to produce the desired therapeutic effect, in association with a suitable pharmaceutical carrier.

As used herein, the term "stereoisomer" refers to a compound made up of the same atoms bonded by the same bonds but having different three-dimensional structures which are not interchangeable. The three-dimensional structures are called configurations. As used herein, the term "enantiomer" refers to two stereoisomers whose molecules are nonsuperimposable mirror images of one another. The term "chiral center" refers to a carbon atom to which four different groups are attached. As used herein, the term "diastereomers" refers to stereoisomers which are not enantiomers. In addition, two diastereomers which have a different configuration at only one chiral center are referred to herein as "epimers". The terms "racemate", "racemic mixture" or "racemic modification" refer to a mixture of equal parts of enantiomers.

The term "enantiomeric enrichment" as used herein refers to the increase in the amount of one enantiomer as compared to the other. A convenient method of expressing the enantiomeric enrichment achieved is the concept of enantiomeric excess, or "ee", which is found using the following equation:

$$ee = \frac{E^1 - E^2}{E^1 + E^2} \text{ X } 100$$

wherein $E^1$ is the amount of the first enantiomer and $E^2$ is the amount of the second enantiomer. Thus, if the initial ratio of the two enantiomers is 50:50, such as is present in a racemic mixture, and an enantiomeric enrichment sufficient to produce a final ratio of 70:30 is achieved, the ee with respect to the first enantiomer is 40%. However, if the final ratio is 90: 10, the ee with respect to the first enantiomer is 80%. An ee of greater than 90% is preferred, an ee of greater than 95% is most preferred and an ee of greater than 99% is most especially preferred. Enantiomeric enrichment is readily determined by one of ordinary skill in the art using standard techniques and procedures, such as gas or high performance liquid chromatography with a chiral column. Choice of the appropriate chiral column, eluent and conditions necessary to effect separation of the enantiomeric pair is well within the knowledge of one of ordinary skill in the art. In addition, the specific stereoisomers and enantiomers of compounds of formula I can be prepared by one of ordinary skill in the art utilizing well known techniques and processes, such as those disclosed by J. Jacques, et al., "Enantiomers, Racemates, and Resolutions", John Wiley and Sons, Inc., 1981, and E.L. Eliel and S.H. Wilen," Stereochemistry of Organic Compounds", (Wiley-Interscience 1994), and European Patent Application No. EP-A-838448, published April 29, 1998. Examples of resolutions include recrystallization techniques or chiral chromatography.

Some of the compounds of the present invention have one or more chiral centers and may exist in a variety of stereoisomeric configurations. As a consequence of these chiral centers, the compounds of the present invention occur as racemates, mixtures of enantiomers and as individual enantiomers, as well as diastereomers and mixtures of diastereomers. All such racemates, enantiomers, and diastereomers are within the scope of the present invention.

The terms "R" and "S" are used herein as commonly used in organic chemistry to denote specific configuration of a chiral center. The term "R" (rectus) refers to that configuration of a chiral center with a clockwise relationship of group priorities (highest to second lowest) when viewed along the bond toward the lowest priority group. The term "S" (sinister) refers to that configuration of a chiral center with a counterclockwise relationship of group priorities (highest to second lowest) when viewed along the bond toward the lowest priority group. The priority of groups is based upon their atomic number (in order of decreasing atomic number). A partial list of priorities and a discussion of stereochemistry is contained in "Nomenclature of Organic Compounds: Principles and Practice", (J.H. Fletcher, et al., eds., 1974) at pages 103-120.

The designation "  " refers to a bond that protrudes forward out of the plane of the page.

The designation "  " refers to a bond that protrudes backward out of the plane of the page.

The designation " 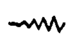 " refers to a bond wherein the stereochemistry is not defined.

In one embodiment, the present invention provides a compound of Formula I, or a pharmaceutically acceptable salt thereof, as described in detail above. While all of the compounds of the present invention are useful, certain of the compounds are particularly interesting and are preferred. The following listings set out several groups of preferred embodiments.

In another embodiment the invention provides a compound of formula I, or a pharmaceutically acceptable salt thereof, wherein

$R^0$ is

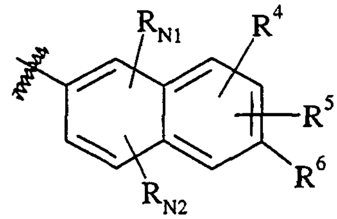 or 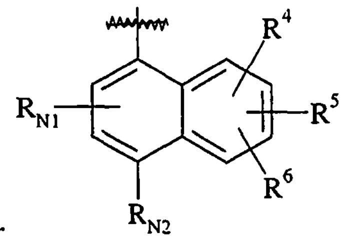 ;

wherein the zigzag line represents the point of attachment to the $R^0$ position in formula I;

$G^1$ is methylene or ethylene;

L is $-CH_2-$;

$R^1$ is hydrogen;

$R^2$ is

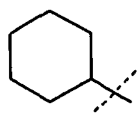 , 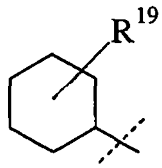 , 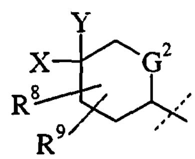 , 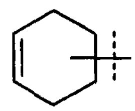 , 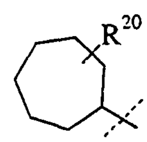 ,

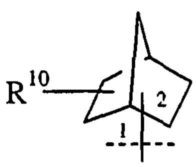 , 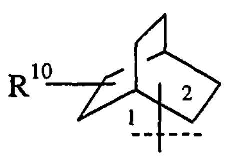 , 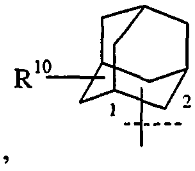 , 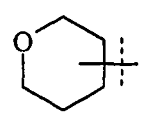 , 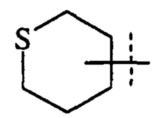 ,

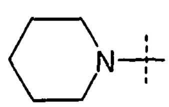 , 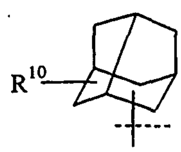 , 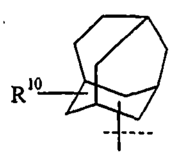 , or 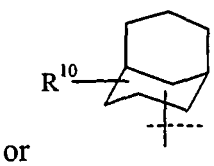 ;

wherein the dashed line represents the point of attachment to the $R^2$ position in formula I; wherein X is hydrogen, hydroxy, $-OCH_3$ or $-CH_2OH$; wherein Y is hydrogen or methyl, provided that at least one of X and Y is not hydrogen; and wherein optionally X and Y together with the carbon to which they are attached form carbonyl; and wherein $R^8$ is independently hydrogen, hydroxy, $-(C_1-C_4)$alkyl(optionally substituted with one to three halogens); $R^9$ is independently hydrogen, hydroxy, or $-(C_1-C_4)$alkyl(optionally substituted with one to three halogens); $R^{10}$ is independently at each occurrence hydrogen, hydroxy, or $-(C_1-C_4)$alkyl (optionally substituted with one to three halogens); and $G^2$ is methylene, ethylene, or 1-propylene;

$R^3$ is hydrogen or $-CH_3$;

$R_{N1}$ is hydrogen, hydroxy, halo, $-(C_1-C_4)$alkyl(optionally substituted with one to three halogens), or $-(C_1-C_4)$alkoxy(optionally substituted with one to three halogens);

$R_{N2}$ is hydrogen, hydroxy, halo, $-(C_1-C_4)$alkyl(optionally substituted with one to three halogens), $-(C_1-C_4)$alkoxy(optionally substituted with one to three halogens), $Ar^1$, or $Ar^2$;

$R^4$ is hydrogen, halo, hydroxy, $-(C_1-C_4)$alkyl(optionally substituted with one to three halogens), $-(C_1-C_4)$alkoxy(optionally substituted with one to three halogens), cyano;

$R^5$ is hydrogen, hydroxy, $-(C_1-C_4)$alkyl(optionally substituted with one to three halogens), $-(C_1-C_4)$alkoxy(optionally substituted with one to three halogens), halo, cyano, $-SCF_3$, $-(C_1-C_4)$alkyl-

C(O)OH, $-(C_1-C_4)$alkyl-C(O)O$(C_1-C_4)$alkyl, $-(C_1-C_4)$alkyl-OH, -O-$(C_1-C_4)$alkyl-C(O)O$(C_1-C_4)$alkyl, -C(O)O$(C_1-C_4)$alkyl, $-OSO_2CF_3$, $-(C_2-C_4)$alkenyl, $Ar^1$, $Ar^2$, or $-(C_1-C_4)$alkyl-C(O)N$(R^{11})(R^{12})$; wherein $R^{11}$ and $R^{12}$ are each independently hydrogen or $-(C_1-C_4)$alkyl, or $R^{11}$ and $R^{12}$ taken together with the nitrogen to which they are attached form piperidinyl or pyrrolidinyl;

$R^6$ is hydrogen, hydroxy, halo, $-(C_1-C_4)$alkyl(optionally substituted with one to three halogens), $-(C_1-C_4)$alkoxy(optionally substituted with one to three halogens), -C(O)O$(C_1-C_4)$alkyl, $Ar^1$, $Het^1$, $Ar^2$, $Het^2$, $-Ar^1-(C_1-C_4)$alkyl, $-Het^1-(C_1-C_4)$alkyl, -O-$(C_1-C_4$ alkyl$)-Ar^2$, $-Ar^2-(C_1-C_4)$alkyl, $-Het^2-(C_1-C_4)$alkyl, -C(O)-$Ar^2$, -C(O)-$Het^2$, or -O$(C_1-C_4)$alkyl-N$(R^{13})(R^{14})$; wherein $R^{13}$ and $R^{14}$ are each independently hydrogen or $-(C_1-C_4)$alkyl, or $R^{13}$ and $R^{14}$ taken together with the nitrogen to which they are attached form piperidinyl or pyrrolidinyl; or

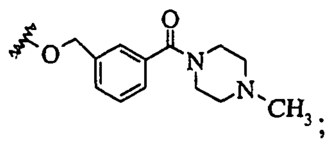

wherein the zigzag line represents the point of attachment to $Ar^1$;

$Ar^1$ is phenyl;

$Ar^2$ is $Ar^1$ optionally substituted once or twice with moieties independently selected from halo, hydroxy, cyano, $-(C_1-C_4)$alkyl(optionally substituted with one to three halogens), $-(C_1-C_4$ alkyl)C(O)OH, -O$(C_1-C_4)$alkyl-C(O)OH, -C(O)O-$(C_1-C_4)$alkyl, $-NH_2$, -C(O)OH, $-(C_1-C_4)$alkyl-N$(R^{15})(R^{16})$, -O$(C_1-C_4)$alkyl-N$(R^{15})(R^{16})$, imidazolyl, pyridinyl, or $-(C_1-C_4)$alkyl-imidazolyl; wherein $R^{15}$ and $R^{16}$ are each independently hydrogen or $-(C_1-C_4)$alkyl or $R^{15}$ and $R^{16}$ taken together with the nitrogen to which they are attached form piperidinyl or pyrrolidinyl;

$Het^1$ is a heterocyclic radical selected from pyridinyl, piperidinyl, pyrimidinyl, pyrazinyl, piperazinyl, pyridazinyl, indolyl, isoindolyl, indolinyl, furanyl, benzofuranyl, thiazolyl, oxazolyl, isoxazolyl, isothiazolyl, benzothiophenyl, thiophenyl, quinolinyl, isoquinolinyl, quinoxalinyl, quinazolinyl, or phthalazinyl;

$Het^2$ is $Het^1$ optionally substituted once or twice with moieties independently selected from halo, hydroxy, cyano, $-CF_3$, $-(C_1-C_4)$alkyl, $-(C_1-C_4)$alkoxy, $-(C_1-C_4)$alkyl-C(O)OH, -O$(C_1-C_4)$alkyl-C(O)OH, -C(O)O$(C_1-C_4)$alkyl, $-(C_1-C_4)$alkyl-N$(R^{17})(R^{18})$, -O$(C_1-C_4)$alkyl-N$(R^{17})(R^{18})$, imidazolyl, pyridinyl, or $-(C_1-C_4)$alkyl-imidazolyl; wherein $R^{17}$ and $R^{18}$ are each independently hydrogen or $-(C_1-C_4)$alkyl or $R^{17}$ and $R^{18}$ taken together with the nitrogen to which they are attached form piperidinyl or pyrrolidinyl; and

$R^{19}$ and $R^{20}$ are each independently hydroxy, $-(C_1-C_4)$alkyl(optionally substituted with one to three halogens), or $-CH_2OH$.

In another embodiment the invention provides a compound of formula I, or a pharmaceutically acceptable salt thereof, wherein

$R^0$ is

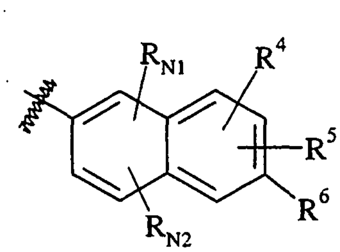

or

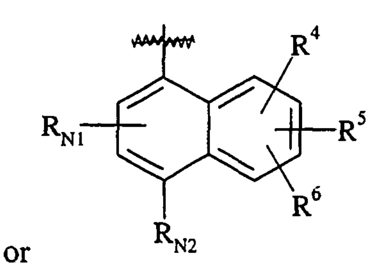

;

wherein the zigzag line represents the point of attachment to the $R^0$ position in formula I;

$G^1$ is methylene or ethylene;

L is $-CH_2-$;

$R^1$ is hydrogen;

$R^2$ is

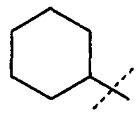 or 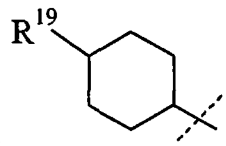 ;

wherein the dashed line represents the point of attachment to the $R^2$ position in formula I;

$R^3$ is hydrogen;

$R_{N1}$ is hydrogen, hydroxy, halo, $-(C_1-C_4)$alkyl(optionally substituted with one to three halogens), or $-(C_1-C_4)$alkoxy(optionally substituted with one to three halogens);

$R_{N2}$ is hydrogen, hydroxy, halo, $-(C_1-C_4)$alkyl(optionally substituted with one to three halogens), $-(C_1-C_4)$alkoxy(optionally substituted with one to three halogens), $Ar^1$, or $Ar^2$;

$R^4$ is hydrogen or halo;

$R^5$ is hydrogen, hydroxy, $-(C_1-C_4)$alkyl(optionally substituted with one to three halogens), $-(C_1-C_4)$alkoxy(optionally substituted with one to three halogens), halo, cyano, $-SCF_3$, $-(C_1-C_4)$alkyl-C(O)OH, $-(C_1-C_4)$alkyl-C(O)O$(C_1-C_4)$alkyl, $-(C_1-C_4)$alkyl-OH, -O-$(C_1-C_4)$alkyl-C(O)O$(C_1-C_4)$alkyl, -C(O)O$(C_1-C_4)$alkyl, $-OSO_2CF_3$, $-(C_2-C_4)$alkenyl, $Ar^1$, $Ar^2$, or $-(C_1-C_4)$alkyl-C(O)N$(R^{11})(R^{12})$; wherein $R^{11}$ and $R^{12}$ are each independently hydrogen or $-(C_1-C_4)$alkyl, or $R^{11}$ and $R^{12}$ taken together with the nitrogen to which they are attached form piperidinyl or pyrrolidinyl;

$R^6$ is hydrogen, hydroxy, halo, $-(C_1-C_4)$alkyl(optionally substituted with one to three halogens), $-(C_1-C_4)$alkoxy(optionally substituted with one to three halogens), -C(O)O$(C_1-C_4)$alkyl, $Ar^1$, $Het^1$, $Ar^2$, $Het^2$, $-Ar^1-(C_1-C_4)$alkyl, $-Het^1-(C_1-C_4)$alkyl, -O-$(C_1-C_4$ alkyl)-$Ar^2$, $-Ar^2-(C_1-C_4)$alkyl, $-Het^2-(C_1-C_4)$alkyl, -C(O)-$Ar^2$, -C (O)-$Het^2$, or -O$(C_1-C_4)$alkyl-N$(R^{13})(R^{14})$; wherein $R^{13}$ and $R^{14}$ are each independently hydrogen or $-(C_1-C_4)$alkyl, or $R^{13}$ and $R^{14}$ taken together with the nitrogen to which they are attached form piperidinyl or pyrrolidinyl; or

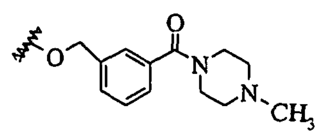 ;

wherein the zigzag line represents the point of attachment to $Ar^1$;

$Ar^1$ is phenyl;

$Ar^2$ is is $Ar^1$ optionally substituted once or twice with moieties independently selected from halo, hydroxy, cyano, $-(C_1-C_4)$alkyl(optionally substituted with one to three halogens), $-(C_1-C_4$ alkyl)C(O)OH, -O$(C_1-C_4)$alkyl-C(O)OH, -C(O)O-$(C_1-C_4)$ alkyl, $-NH_2$, -C(O)OH, $-(C_1-C_4)$alkyl-N$(R^{15})(R^{16})$, -O$(C_1-C_4)$alkyl-N$(R^{15})(R^{16})$; wherein $R^{15}$ and $R^{16}$ are each independently hydrogen or $-(C_1-C_4)$alkyl or $R^{15}$ and $R^{16}$ taken together with the nitrogen to which they are attached form piperidinyl or pyrrolidinyl;

$Het^1$ is a heterocyclic radical selected from pyridinyl, piperidinyl, pyrimidinyl, pyrazinyl, piperazinyl, pyridazinyl, furanyl, thiazolyl, oxazolyl, isoxazolyl, isothiazolyl, thiophenyl;

$Het^2$ is $Het^1$ optionally substituted once or twice with moieties independently selected from

halo, hydroxy, cyano, $-CF_3$, $-(C_1-C_4)$alkyl, $-(C_1-C_4)$alkoxy, $-(C_1-C_4)$alkyl-C(O) OH, -O$(C_1-C_4)$alkyl-C(O)OH, -C(O)O$(C_1-C_4)$alkyl, $-(C_1-C_4)$alkyl-N$(R^{17})(R^{18})$, -O$(C_1-C_4)$alkyl-N$(R^{17})(R^{18})$; wherein $R^{17}$ and $R^{18}$ are each independently hydrogen or $-(C_1-C_4)$alkyl or $R^{17}$ and $R^{18}$ taken together with the nitrogen to

which they are attached form piperidinyl or pyrrolidinyl; and

$R^{19}$ is hydroxy, $-(C_1-C_4)$alkyl(optionally substituted with one to three halogens), or $-CH_2OH$.

In another embodiment the invention provides a compound of formula I, or a pharmaceutically acceptable salt thereof, wherein

$R^0$ is

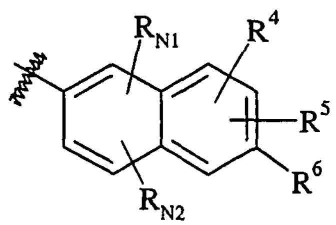

or

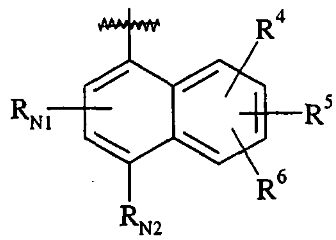

;

wherein the zigzag line represents the point of attachment to the $R^0$ position in formula I;

$G^1$ is methylene;

L is $-CH_2-$;

$R^1$ is hydrogen;

$R^2$ is

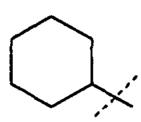

or

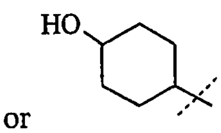

;

wherein the dashed line represents the point of attachment to the $R^2$ position in formula I;

$R^3$ is hydrogen;

$R_{N1}$ is hydrogen, halo, $-CH_3$, or $-O-CH_3$;

$R_{N2}$ is hydrogen, hydroxy, halo, $-(C_1-C_4)$alkyl(optionally substituted with one to three halogens), $-(C_1-C_4)$alkoxy(optionally substituted with one to three halogens), $Ar^1$, or $Ar^2$;

$R^4$ is hydrogen or halo;

$R^5$ is hydrogen, hydroxy, $-(C_1-C_4)$alkyl(optionally substituted with one to three halogens), $-(C_1-C_4)$alkoxy(optionally substituted with one to three halogens), halo, cyano, $-SCF_3$, $-(C_1-C_4)$alkyl-C(O)OH, $-(C_1-C_4)$alkyl-C(O)O$(C_1-C_4)$alkyl, $-(C_1-C_4)$alkyl-OH, -O-$(C_1-C_4)$alkyl-C(O)O$(C_1-C_4)$alkyl, -C(O)O$(C_1-C_4)$alkyl, $-OSO_2CF_3$, $-(C_2-C_4)$alkenyl, or $-(C_1-C_4)$alkyl-C(O)N$(R^{11})(R^{12})$; wherein $R^{11}$ and $R^{12}$ are each independently hydrogen or $-(C_1-C_4)$alkyl, or $R^{11}$ and $R^{12}$ taken together with the nitrogen to which they are attached form piperidinyl or pyrrolidinyl;

$R^6$ is hydroxy, halo, $-(C_1-C_4)$alkyl(optionally substituted with one to three halogens),$-(C_1-C_4)$alkoxy(optionally substituted with one to three halogens), -C(O)O$(C_1-C_4)$alkyl, $Ar^1$, $Het^1$, $Ar^2$, $Het^2$, $-Ar^1-(C_1-C_4)$alkyl, $-Het^1-(C_1-C_4)$alkyl, -O-$(C_1-C_4$ alkyl)-$Ar^2$, $-Ar^2-(C_1-C_4)$alkyl, $-Het^2-(C_1-C_4)$alkyl, -C(O)-$Ar^2$, -C(O)-$Het^2$, or -O$(C_1-C_4)$alkyl-N$(R^{13})(R^{14})$; wherein $R^{13}$ and $R^{14}$ are each independently hydrogen or $-(C_1-C_4)$alkyl, or $R^{13}$ and $R^{14}$ taken together with the nitrogen to which they are attached form piperidinyl or pyrrolidinyl; or

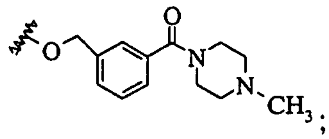

| | |
|---|---|
| | wherein the zigzag line represents the point of attachment to $Ar^1$; |
| $Ar^1$ is | phenyl; |
| $Ar^2$ is | $Ar^1$ optionally substituted once or twice with moieties independently selected from halo, hydroxy, cyano, $-(C_1-C_4)$alkyl(optionally substituted with one to three halogens), $-(C_1-C_4$ alkyl)C(O)OH, $-O(C_1-C_4)$akyl-C(O)OH, $-C(O)O-(C_1-C_4)$alkyl, $-NH_2$, -C(O)OH, $-(C_1-C_4)$alkyl-N($R^{15}$)($R^{16}$), $-O(C_1-C_4)$alkyl-N($R^{15}$)($R^{16}$); wherein $R^{15}$ and $R^{16}$ are each independently hydrogen or $-(C_1-C_4)$alkyl or $R^{15}$ and $R^{16}$ taken together with the nitrogen to which they are attached form piperidinyl or pyrrolidinyl; |
| $Het^1$ is | a heterocyclic radical selected from pyridinyl, piperidinyl, pyrimidinyl, pyrazinyl, piperazinyl, pyridazinyl, furanyl, thiazolyl, oxazolyl, isoxazolyl, isothiazolyl, thiophenyl; and |
| $Het^2$ is | $Het^1$ optionally substituted once or twice with moieties independently selected from halo, hydroxy, cyano, $-CF_3$, $-(C_1-C_4)$alkyl, $-(C_1-C_4)$alkoxy, $-(C_1-C_4)$alkyl-C(O)OH, $-O(C_1-C_4)$alkyl-C(O)OH, $-C(O)O(C_1-C_4)$alkyl, $-(C_1-C_4)$alkyl-N($R^{17}$)($R^{18}$), $-O(C_1-C_4)$alkyl-N($R^{17}$)($R^{18}$); wherein $R^{17}$ and $R^{18}$ are each independently hydrogen or $-(C_1-C_4)$alkyl or $R^{17}$ and $R^{18}$ taken together with the nitrogen to which they are attached form piperidinyl or pyrrolidinyl. |

Other embodiments of the invention are provided wherein each of the embodiments described herein above is further narrowed as described in the following preferences. Specifically, each of the preferences below is independently combined with each of the embodiments above, and the particular combination provides another embodiment in which the variable indicated in the preference is narrowed according to the preference.

Preferably $R^0$ is

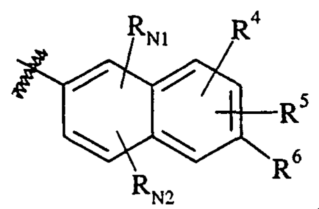

Preferably $R^0$ is

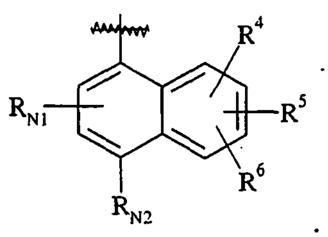

Preferably $G^1$ is methylene. Preferably $G^1$ is ethylene. Preferably L is $-CH_2-$.

Preferably $R^1$ is hydrogen. Preferably $R^1$ is $-CH_3$.

Preferably $R^2$ is

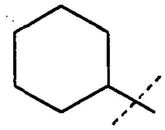 , 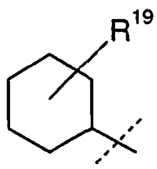 , 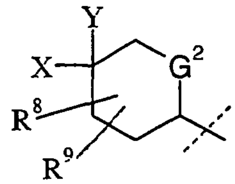 , or 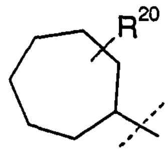 ,

wherein the dashed line indicates the point of attachment to the $R^2$ position in formula I; $G^2$ is methylene, ethylene, or 1-propylene; X is hydrogen, hydroxy, or - $CH_2OH$; Y is hydrogen or methyl, provided that at least one of X and Y is not hydrogen; or X and Y together with the carbon to which they are attached form a carbonyl; $R^8$ and $R^9$ are each independently hydrogen, hydroxy, or -($C_1$-$C_4$)alkyl(optionally substituted with one to three halogens).

Preferably $R^2$ is

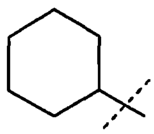 , 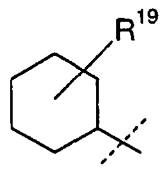 , 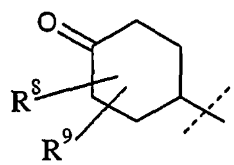 ,

or,

wherein the dashed line indicates the point of attachment to the $R^2$ position in formula I; $R^8$ and $R^9$ are each independently hydrogen, hydroxy, or -($C_1$-$C_4$)alkyl(optionally substituted with one to three halogens).

Preferably $R^2$ is

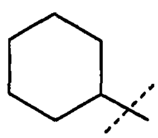 , or 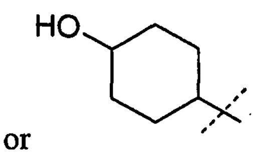 .

Preferably $R^2$ is

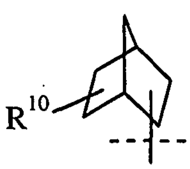 , 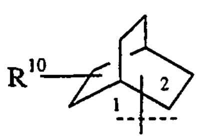 , 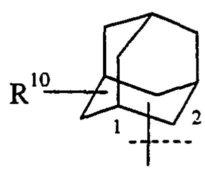 , 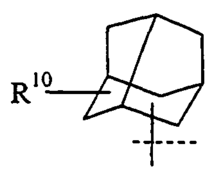 , 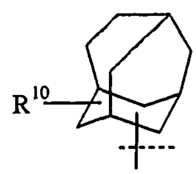 ,

or 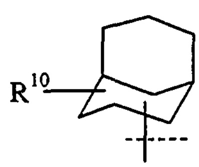 ;

wherein the dashed line indicates the point of attachment to the $R^2$ position in formula I; $R^{10}$ is hydrogen, hydroxy, or -($C_1$-$C_4$)alkyl(optionally substituted with one to three halogens).

Preferably $R^2$ is

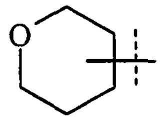, 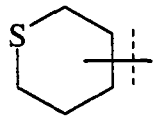, or 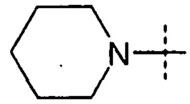.

Preferably $R^3$ is hydrogen. Preferably $R^3$ is $-CH_3$.

Preferably $R_{N1}$ is hydrogen. Preferably $R_{N1}$ is hydroxy, halo, $-(C_1-C_4)$alkyl(optionally substituted with one to three halogens), or $-(C_1-C_4)$alkoxy(optionally substituted with one to three halogens). Preferably $R_{N1}$ is hydrogen, halo, methyl, or methoxy.

Preferably $R_{N2}$ is hydrogen. Preferably $R_{N2}$ is hydroxy, halo, $-(C_1-C_4)$alkyl(optionally substituted with one to three halogens), $-(C_1-C_4)$alkoxy(optionally substituted with one to three halogens), $Ar^1$, or $Ar^2$. Preferably $R_{N2}$ is halo, $-(C_1-C_4)$alkyl(optionally substituted with one to three halogens), or $-(C_1-C_4)$alkoxy(optionally substituted with one to three halogens). Preferably $R_{N2}$ is $Ar^2$.

Preferably $R^4$ is hydrogen. Preferably $R^4$ is halo, hydroxy, $-(C_1-C_4)$alkyl(optionally substituted with one to three halogens), $-(C_1-C_4)$alkoxy(optionally substituted with one to three halogens). Preferably $R^4$ is halo.

Preferably $R^5$ is hydrogen. Preferably $R^5$ is hydroxy, $-(C_1-C_4)$alkyl(optionally substituted with one to three halogens), $-(C_1-C_4)$alkoxy(optionally substituted with one to three halogens), halo, cyano, $-SCF_3$, $-(C_1-C_4)$alkyl-C(O)OH, $-(C_1-C_4)$alkyl-C(O)O$(C_1-C_4)$alkyl, $-(C_1-C_4)$alkcyl-OH, -O-$(C_1-C_4)$alkyl-C(O)O$(C_1-C_4)$alkyl, -C(O)O$(C_1-C_4)$alkyl, $-OSO_2CF_3$, $-(C_2-C_4)$alkenyl, $Ar^1$, $Ar^2$, or $-(C_1-C_4)$alkyl-C(O)N$(R^{11})(R^{12})$; wherein $R^{11}$ and $R^{12}$ are each independently hydrogen or $-(C_1-C_4)$alkyl, or $R^{11}$ and $R^{12}$ taken together with the nitrogen to which they are attached form piperidinyl or pyrrolidinyl. Preferably $R^5$ is hydrogen, hydroxy, $-(C_1-C_4)$alkyl(optionally substituted with one to three halogens), $-(C_1-C_4)$alkoxy(optionally substituted with one to three halogens), halo, cyano, $-SCF_3$, - $(C_1-C_4)$allcyl-C(O)OH, $-(C_1-C_4)$alkyl-C(O)O$(C_1-C_4)$alkyl, $-(C_1-C_4)$alkyl-OH, -O-$(C_1-C_4)$alkyl-C(O)O$(C_1-C_4)$alkyl, -C(O)O$(C_1-C_4)$alkyl, $-OSO_2CF_3$, $-(C_2-C_4)$alkenyl, or $-(C_1-C_4)$alkyl-C(O)N$(R^{11})(R^{12})$; wherein $R^{11}$ and $R^{12}$ are each independently hydrogen or $-(C_1-C_4)$alkyl, or $R^{11}$ and $R^{12}$ taken together with the nitrogen to which they are attached form piperidinyl or pyrrolidinyl. Preferably $R^5$ is hydrogen, hydroxy, $-(C_1-C_4)$alkyl(optionally substituted with one to three halogens), $-(C_1-C_4)$alkoxy(optionally substituted with one to three halogens), halo, $-(C_1-C_4)$alkyl-C(O)OH, $-(C_1-C_4)$alkyl-C(O)O$(C_1-C_4)$alkyl, $-(C_1-C_4)$alkyl-OH, -O-$(C_1-C_4)$alkyl-C(O)O$(C_1-C_4)$alkyl, -C(O)O$(C_1-C_4)$alkyl, $-(C_2-C_4)$alkenyl. Preferably $R^5$ is hydrogen, or halo.

Preferably $R^6$ is hydrogen. Preferably $R^6$ is hydroxy, halo, $-(C_1-C_4)$alkyl(optionally substituted with one to three halogens), $-(C_1-C_4)$alkoxy(optionally substituted with one to three halogens), -C(O)O$(C_1-C_4)$alkyl, $Ar^1$, $Het^1$, $Ar^2$, $Het^2$, $-Ar^1-(C_1-C_4)$alkyl, $-Het^1-(C_1-C_4)$alkyl, -O-$(C_1-C_4$ alkyl)-$Ar^2$, $-Ar^2-(C_1-C_4)$alkyl, $-Het^2-(C_1-C_4)$alkyl, -C(O)-$Ar^2$, -C(O)-$Het^2$, or -O$(C_1-C_4)$alkyl-N$(R^{13})(R^{14})$; wherein $R^{13}$ and $R^{14}$ are each independently hydrogen or $-(C_1-C_4)$alkyl, or $R^{13}$ and $R^{14}$ taken together with the nitrogen to which they are attached form piperidinyl or pyrrolidinyl; or

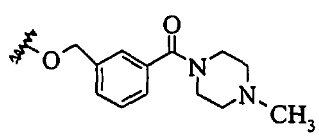;

wherein the zigzag line represents the point of attachment to $Ar^1$. Preferably $R^6$ is hydrogen, hydroxy, halo, $-(C_1-C_4)$alkyl(optionally substituted with one to three halogens), $-(C_1-C_4)$alkoxy(optionally substituted with one to three halogens), -C(O)O$(C_1-C_4)$alkyl, or - O$(C_1-C_4)$alkyl-N$(R^{13})(R^{14})$; wherein $R^{13}$ and $R^{14}$ are each independently hydrogen or- $(C_1-C_4)$alkyl, or $R^{13}$ and $R^{14}$ taken together with the nitrogen to which they are attached form piperidinyl or pyrrolidinyl; or

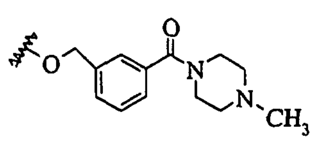;

wherein the zigzag line represents the point of attachment to $Ar^1$.

Preferably $R^6$ is $Ar^1$, $Het^1$, $Ar^2$, $Het^2$, $-Ar^1-(C_1-C_4)$alkyl, $-Het^1-(C_1-C_4)$alkyl, -O-$(C_1-C_4$ alkyl)-$Ar^2$, $-Ar^2-(C_1-C_4)$alkyl, $-Het^2-(C_1-C_4)$alkyl, -C(O)-$Ar^2$, or -C(O)-$Het^2$. Preferably $R^6$ is $-Ar^1-(C_1-C_4)$alkyl, $-Het^1-(C_1-C_4)$alkyl, -O-$(C_1-C_4$

allcyl)-$Ar^2$, -$Ar^2$-($C_1$-$C_4$)alkyl, -$Het^2$-($C_1$-$C_4$)alkyl, -C(O)-$Ar^2$, or -C(O)-$Het^2$.

Preferably $Ar^1$ is phenyl. Preferably $Ar^2$ is $Ar^1$ optionally substituted with from one or two moieties independently selected from halo, hydroxy, cyano, -($C_1$-$C_4$)alkyl(optionally substituted with one to three halogens), -C(O)OH, -C(O)$OCH_3$, -($C_1$-$C_4$)alkyl-C(O)OH, -O-($C_1$-$C_4$)alkyl-C(O)OH, -($C_1$-$C_4$)alkyl-N($R^{15}$)($R^{16}$), -O-($C_1$-$C_4$)alkyl-N($R^{15}$)($R^{16}$); wherein $R^{15}$ and $R^{16}$ are each independently hydrogen or -($C_1$-$C_4$)alkyl, or $R^{15}$ and $R^{16}$ taken together with the nitrogen to which they are attached form piperidinyl or pyrrolidinyl.

Preferably $Ar^2$ is $Ar^1$ substituted once with a moiety independently selected from halo, hydroxy, cyano, -($C_1$-$C_4$)alkyl(optionally substituted with one to three halogens), - C(O)OH, -C(O)$OCH_3$, -($C_1$-$C_4$)alkyl-C(O)OH, -O-($C_1$-$C_4$)alkyl-C(O)OH, -($C_1$-$C_4$)alkyl-, N($R^{15}$)($R^{16}$), -O-($C_1$-$C_4$)alkyl-N($R^{15}$)($R^{16}$); wherein $R^{15}$ and $R^{16}$ are each independently hydrogen or -($C_1$-$C_4$)alkyl, or $R^{15}$ and $R^{16}$ taken together with the nitrogen to which they are attached form piperidinyl or pyrrolidinyl.

Preferably $Het^1$ is a heterocyclic radical selected from pyridinyl, piperidinyl, pyrimidinyl, pyrazinyl, piperazinyl, pyridazinyl, indolyl, isoindolyl, indolinyl, furanyl, thiazolyl, oxazolyl, isoxazolyl, isothiazolyl, thiophenyl. Preferably $Het^1$ is a heterocyclic radical selected from pyridinyl, piperidinyl, pyrimidinyl, pyrazinyl, piperazinyl, pyridazinyl, furanyl, thiazolyl, oxazolyl, isoxazolyl, isothiazolyl, thiophenyl. Preferably $Het^1$ is pyridinyl.

Preferably $Het^2$ is $Het^1$ optionally substituted with from one or two moieties independently selected from halo, hydroxy, cyano, -($C_1$-$C_4$)alkyl(optionally substituted with one to three halogens), -C(O)OH, -C(O)$OCH_3$, -($C_1$-$C_4$)alkyl-C(O)OH, -O-($C_1$-$C_4$)alkyl)C(O)OH, -($C_1$-$C_4$)alkyl-N($R^{17}$)($R^{18}$), -O-($C_1$-$C_4$)alkyl-N($R^{17}$)($R^{18}$), wherein $R^{17}$ and $R^{18}$ are each independently hydrogen or -($C_1$-$C_4$)alkyl, or $R^{17}$ and $R^{18}$ taken together with the nitrogen to which they are attached form piperidinyl or pyrrolidinyl.

Preferably $Het^2$ is $Het^1$ substituted once by a moiety selected from halo, hydroxy, cyano, -($C_1$-$C_4$)alkyl(optionally substituted with one to three halogens), -C(O)OH, - C(O)$OCH_3$, -($C_1$-$C_4$)alkyl-C(O)OH, -O-($C_1$-$C_4$)alkyl)C(O)OH, -($C_1$-$C_4$)alkyl-N($R^{17}$)($R^{18}$), -O-($C_1$-$C_4$)alkyl-N($R^{17}$)($R^{18}$), wherein $R^{17}$ and $R^{18}$ are each independently hydrogen or -($C_1$-$C_4$)alkyl, or $R^{17}$ and $R^{18}$ taken together with the nitrogen to which they are attached form piperidinyl or pyrrolidinyl.

Preferably $R^{19}$ is hydroxy, or -$CH_3$(optionally substituted with one to three halogens), or -$CH_2OH$. Preferably $R^{19}$ is hydroxy. Preferably $R^{19}$ is -$CH_3$(optionally substituted with one to three halogens). Preferably $R^{19}$ is -$CH_2OH$.

Preferably $R^{20}$ is hydrogen, hydroxy, -($C_1$-$C_4$)alkyl(optionally substituted with one to three halogens), or -$CH_2OH$. Preferably $R^{20}$ is hydrogen or hydroxy.

The present invention relates to a compound or a pharmaceutically acceptable salt thereof represented by formula (I):

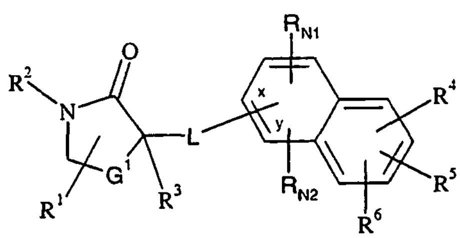

(I)

wherein

- $G^1$ is methylene or ethylene;
- L is a divalent linking group selected from $C_1$-$C_4$ alkylene, -S-, -CH(OH)-, -O-, or -NH-;
- $R^1$ is hydrogen, hydroxy, $C_1$-$C_4$ alkyl, $C_1$-$C_4$ alkoxy, or -$CH_2OR^7$ wherein $R^7$ is hydrogen or $C_1$-$C_4$ alkyl;
- $R^2$ is a monovalent radical having one of the following formulae

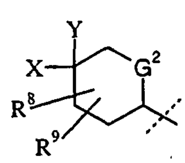

,

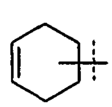

,

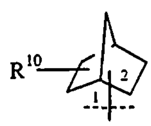, 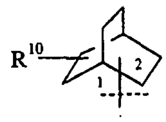, 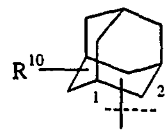,

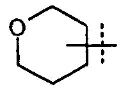, 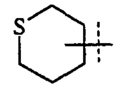, 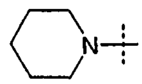,

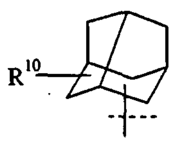, 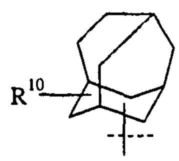, or 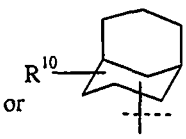;

wherein X is hydrogen, hydroxy or $-CH_2OH$ and Y is hydrogen or methyl or X and Y together form (=O) and wherein $R^8$ and $R^9$ are each independently hydrogen, hydroxy, $C_1$-$C_4$ alkyl or phenyl, and $R^{10}$ is hydrogen, hydroxy, or $C_1$-$C_4$ alkyl and $G^2$ is methylene, ethylene, or 1-propylene;

$R^3$ is hydrogen, hydroxy, or $C_1$-$C_4$ alkyl;

$R_{N1}$ and $R_{N2}$ are each independently hydrogen, $C_1$-$C_4$ alkyl, or halo;

$R^4$ and $R^5$ are each independently hydrogen, hydroxy, $C_1$-$C_4$ alkyl, $C_1$-$C_4$ alkoxy, halo, cyano, trifluoromethyl, trifluoromethylsulfanyl, trifluoromethoxy, $Ar^1$, $Het^1$, $Ar^1$-($C_1$-$C_4$ alkyl), $Het^1$-($C_1$-$C_4$ alkyl), -($C_1$-$C_4$ alkyl)COOH, -($C_1$-$C_4$ alkyl)COO($C_1$-$C_4$ alkyl), -($C_1$-$C_4$ alkyl)OH, or -($C_1$-$C_4$ alkyl)CON($R^{11}$)($R^{12}$); wherein $R^{11}$ and $R^{12}$ are each independently hydrogen or $C_1$-$C_4$ alkyl or $R^{11}$ and $R^{12}$ taken together with the nitrogen to which they are attached form piperidinyl or pyrrolidinyl;

$R^6$ is hydrogen, hydroxy, $C_1$-$C_4$ alkyl, $C_1$-$C_4$ alkoxy, halo, cyano, trifluoromethyl, $Ar^2$, $Het^2$, $Ar^2$-($C_1$-$C_4$ alkyl), $Het^2$-($C_1$-$C_4$ alkyl), -CO($C_1$-$C_4$ alkyl), -CO-$Ar^2$, -CO-$Het^2$, or -O($C_1$-$C_4$ alkyl)N($R^{13}$)($R^{14}$); wherein $R^{13}$ and $R^{14}$ are each independently hydrogen or $C_1$-$C_4$ alkyl or $R^{13}$ and $R^{14}$ taken together with the nitrogen to which they are attached form piperidinyl or pyrrolidinyl;

$Ar^1$ is phenyl or naphthyl;

$Ar^2$ is $Ar^1$ optionally substituted with from one to three moieties selected from halo, hydroxy, cyano, trifluoromethyl, $C_1$-$C_4$ alkyl, -($C_1$-$C_4$ alkyl)COOH, -O($C_1$-$C_4$ alkyl)COOH, -($C_1$-$C_4$ alkyl)N($R^{15}$)($R^{16}$), -O($C_1$-$C_4$ alkyl)N($R^{15}$)($R^{16}$), imidazolyl, pyridyl, or -($C_1$-$C_4$ alkyl)-imidazolyl; wherein $R^{15}$ and $R^{16}$ are each independently hydrogen or $C_1$-$C_4$ alkyl or $R^{15}$ and $R^{16}$ taken together with the nitrogen to which they are attached form piperidinyl or pyrrolidinyl;

$Het^1$ is a heterocyclic radical selected from pyridinyl, piperidinyl, pyrimidinyl, pyrazinyl, piperazinyl, pyridazinyl, indolyl, isoindolyl, indolinyl, furanyl, benzofuranyl, thiazolyl, oxazolyl, isoxazolyl, isothiazolyl, benzothiophenyl, thiophenyl, quinolinyl, isoquinolinyl, quinoxalinyl, quinazolinyl, or phthalazinyl; and

$Het^2$ is $Het^1$ optionally substituted with from one to three moieties selected from halo, hydroxy, cyano, trifluoromethyl, $C_1$-$C_4$ alkyl, -($C_1$-$C_4$ alkyl)COOH, -O($C_1$-$C_4$ alkyl)COOH, -($C_1$-$C_4$ alkyl)N($R^{17}$)($R^{18}$), -O($C_1$-$C_4$ alkyl)N($R^{17}$)($R^{18}$), imidazolyl, pyridyl, or -($C_1$-$C_4$ alkyl)-imidazolyl; wherein $R^{17}$ and $R^{18}$ are each independently hydrogen or $C_1$-$C_4$ alkyl or $R^{17}$ and $R^{18}$ taken together with the nitrogen to which they are attached form piperidinyl or pyrrolidinyl.

In another aspect, the present invention provides a pharmaceutical formulation comprising a therapeutically effective amount of a compound of formula (I) or a pharmaceutically acceptable salt thereof, together with one or more pharmaceutical carrier or diluent.

Another aspect of the invention is to use a compound of formula (I) or a pharmaceutically acceptable salt thereof to treat disease states responsive to 11-beta-hydroxysteroid dehydrogenase ligands.

Another aspect of the invention is the treatment of a condition selected from the group consisting of: (1) hyperglycemia, (2) low glucose tolerance, (3) insulin resistance, (4) obesity, (5) lipid disorders, (6) dyslipidemia, (7) hyperlipidemia, (8) hypertriglyceridemia, (9) hypercholesterolemia, (10) low HDL levels, (11) high LDL levels, (12) atherosclerosis and its sequelae, (13) vascular restenosis, (14) pancreatitis, (15) abdominal obesity, (16) neurodegenerative disease, (17) retinopathy, (18) nephropathy, (19) neuropathy, (20) Syndrome X, and other conditions and disorders where insulin resistance is a component, in a patient in need of such treatment, comprising administering to said patient

a therapeutically effective amount of a compound of formula (I) or a pharmaceutically acceptable salt thereof.

Preferred compounds of the invention include compounds or pharmaceutically acceptable salts of formula (I) wherein:

(1) $G^1$ is methylene;
(2) L is methylene;
(3) $R^1$ is hydrogen or methyl;
(4) $R^2$ is cyclohexyl, 4-hydroxycyclohexyl, or 1-adamantyl;
(5) $R^3$ is hydrogen;
(6) $R_{N1}$ and $R_{N2}$ are each independently hydrogen, methyl, or halo;
(7) $R^4$ and $R^5$ are each independently hydrogen, hydroxy, $C_1$-$C_4$ alkyl, $C_1$-$C_4$ alkoxy, halo, cyano, or trifluoromethyl;
(8) $R^6$ is hydrogen, hydroxy, $C_1$-$C_4$ alkyl, $C_1$-$C_4$ alkoxy, halo, cyano, or trifluoromethyl.

Further, any combination of the above groups, e.g., (1) and (2); (3) and (5); (3), (4), (5), (6), (7), and (8); and (1), (2), (3), (4), (5), (6), (7), and (8), are specifically contemplated.

Preferred compounds of the invention also include compounds or pharmaceutically acceptable salts of formula (II):

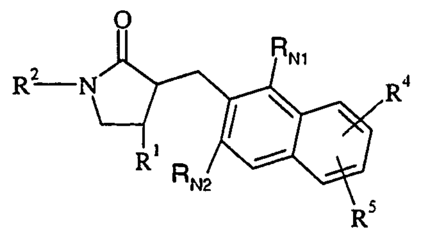

(II)

wherein

$R^1$ is hydrogen or methyl;
$R^2$ is a monovalent radical having one of the following formulae

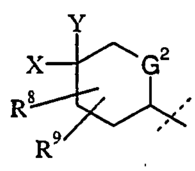
,
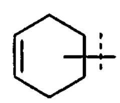
,

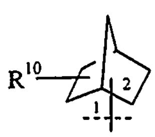
,
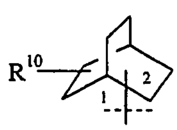
,
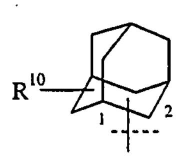
,

wherein X is hydrogen, hydroxy or $-CH_2OH$ and Y is hydrogen or methyl or X and Y together form (=O) and wherein $R^8$ and $R^9$ are each independently hydrogen, hydroxy, $C_1$-$C_4$ alkyl or phenyl, and $R^{10}$ is hydrogen, hydroxy, or $C_1$-$C_4$ alkyl and $G^2$ is methylene, ethylene, or 1-propylene;
$R_{N1}$ and $R_{N2}$ are each independently hydrogen, $C_1$-$C_4$ alkyl, or halo; and
$R^4$ and $R^5$ are each independently hydrogen, hydroxy, $C_1$-$C_4$ alkyl, $C_1$-$C_4$ alkoxy, halo, cyano, or trifluoromethyl.

Other preferred compounds of the invention include compounds or pharmaceutically acceptable salts of formula

(II) wherein

(1) $R^1$ is hydrogen;
(2) $R^2$ is cyclohexyl;
(3) $R_{N1}$ and $R_{N2}$ are:

(a) both hydrogen; or
(b) both methyl; or
(c) one of $R_{N1}$ and $R_{N2}$ is methyl and the other is hydrogen; or
(d) one of $R_{N1}$ and $R_{N2}$ is halo and the other is hydrogen; and

(4) $R^4$ and $R^5$ are each independently hydrogen, hydroxy, $C_1$-$C_4$ alkyl, $C_1$-$C_4$ alkoxy, halo, cyano, or trifluoromethyl.

Further, any combination of the above groups, e.g., (1) and (2); (3) and (4); (1), (2), (3), and (4); (1), (2), (3a), and (4); (1) and (3a); (2) and (3b), and the like, are specifically contemplated.

Other preferred compounds of the invention also include compounds or pharmaceutically acceptable salts of formula (III):

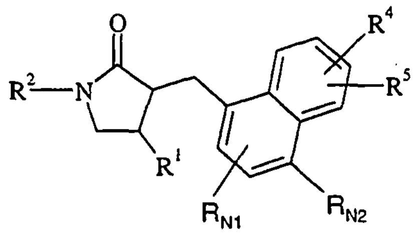

(III)

wherein

$R^1$ is hydrogen or methyl;
$R^2$ is a monovalent radical having one of the following formulae

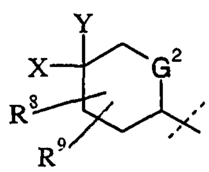
,
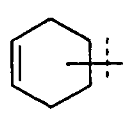
,

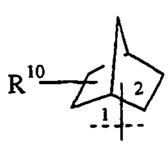
,
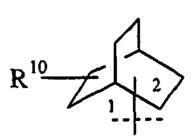
,
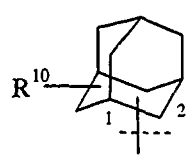
,

wherein X is hydrogen, hydroxy or $-CH_2OH$ and Y is hydrogen or methyl or X and Y together form (=O) and wherein $R^8$ and $R^9$ are each independently hydrogen, hydroxy, $C_1$-$C_4$ alkyl or phenyl, and $R^{10}$ is hydrogen, hydroxy, or $C_1$-$C_4$ alkyl and $G^2$ is methylene, ethylene, or 1-propylene;
$R_{N1}$ and $R_{N2}$ are each independently hydrogen, $C_1$-$C_4$ alkyl, or halo; and
$R^4$ and $R^5$ are each independently hydrogen, hydroxy, $C_1$-$C_4$ alkyl, $C_1$-$C_4$ alkoxy, halo, cyano, or trifluoromethyl.

Other preferred compounds of the invention include compounds or pharmaceutically acceptable salts of formula (III) wherein

(1) $R^1$ is hydrogen;
(2) $R^2$ is cyclohexyl;
(3) $R_{N1}$ and $R_{N2}$ are:

a. both hydrogen; or
b. both methyl; or
c. one of $R_{N1}$ and $R_{N2}$ is methyl and the other is hydrogen; or
d. one of $R_{N1}$ and $R_{N2}$ is halo and the other is hydrogen; and

(4) $R^4$ and $R^5$ are each independently hydrogen, hydroxy, $C_1$-$C_4$ alkyl, $C_1$-$C_4$ alkoxy, halo, cyano, or trifluoromethyl.

Further, any combination of the above groups, e.g., (1) and (2); (3) and (4); (1), (2), (3), and (4); (1), (2), (3a), and (4); (1) and (3a); (2) and (3b), and the like, are specifically contemplated.

Additional preferred compounds of the invention also include compounds or pharmaceutically acceptable salts of formula (IV):

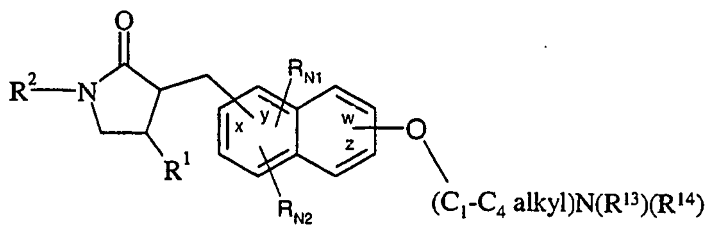

(IV)

wherein

$R^1$ is hydrogen or methyl;
$R^2$ is a monovalent radical having one of the following formulae

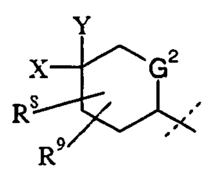 , 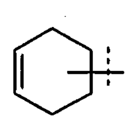 ,

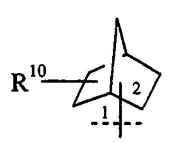 , 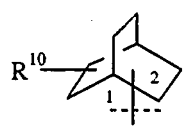 , 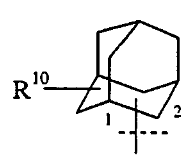 ,

wherein X is hydrogen, hydroxy or $-CH_2OH$ and Y is hydrogen or methyl or X and Y together form (=O) and wherein $R^8$ and $R^9$ are each independently hydrogen, hydroxy, $C_1$-$C_4$ alkyl or phenyl, and $R^{10}$ is hydrogen, hydroxy, or $C_1$-$C_4$ alkyl and $G^2$ is methylene, ethylene, or 1-propylene;
R13 and R14 are each independently hydrogen or $C_1$-$C_4$ alkyl or $R^{13}$ and $R^{14}$ taken together with the nitrogen to which they are attached form piperidinyl or pyrrolidinyl;
provided that the lactam methylene group attaches at either the x or y carbon on the naphthyl group and the oxy group attaches at either the w or z carbon on the naphthyl group.

Other preferred compounds of the invention include compounds or pharmaceutically acceptable salts of formula (III) wherein

(1) $R^1$ is hydrogen;
(2) $R^2$ is cyclohexyl;
(3) $R^{13}$ and $R^{14}$ are:

a. both hydrogen; or
b. both methyl; or
c. together with the nitrogen to which they are attached form piperidinyl; or
d. together with the nitrogen to which they are attached form pyrrolidinyl; Further, any combination of the above groups, e.g., (1) and (2); (2) and (3); (1), (2), and (3); (1), (2), and (3a); (1) and (3c); (2) and (3c); (1), (2), and (3d), and the like, are specifically contemplated.

Particularly preferred compounds of the invention, including pharmaceutically acceptable salts thererof, are represented by the following compounds: 1-Cyclohexyl-3-naphthalen-2-ylmethyl-pyrrolidin-2-one; 3-(1-Bromo-naphthalen-2-ylmethyl)-1-cyclohexyl-pyrrolidin-2-one; 1-Cyclohexyl-3-naphthalen-1-ylmethyl-pyrrolidin-2-one; 1-Cyclohexyl-3-(6-methoxy-naphthalen-2-ylmethyl)-pyrrolidin-2-one; 1-Cyclohexyl-3-(6-hydroxy-naphthalen-2-ylmethyl)-pyrrolidin-2-one; 1-Cyclohexyl-3-(1,4-dimethyl-naphthalen-2-ylmethyl)-pyrrolidin-2-one; 1-(*cis*)-(4-Hydroxy-cyclohexyl)-3-naphthalen-2-ylmethyl-pyrrolidin-2-one; 3-(1-Chloro-naphthalen-2-ylmethyl)-1-cyclohexyl-pyrrolidin-2-one; 1-Cyclohexyl-3-[6-(2-dimethylamino-ethoxy)-naphthalen-2-ylmethyl]-pyrrolidin-2-one; 1-Cyclohexyl-3-[6-(2-piperidin-1-yl-ethoxy)-naphthalen-2-ylmethyl]-pyrrolidin-2-one; 1-Cyclohexyl-3-[6-(3-dimethylamino-propoxy)-naphthalen-2-ylmethyl]-pyrrolidin-2-one; 4-[6-(1-Cyclohexyl-2-oxo-pyrrolidin-3-ylmethyl)-naphthalen-2-yloxy]-butyric acid; 1-(*cis*-4-Hydroxy-cyclohexyl)-3-(6-methoxy-naphthalen-2-ylmethyl)-pyrrolidin-2-one; 3-[6-(1-Cyclohexyl-2-oxo-pyrrolidin-3-ylmethyl)-naphthalen-2-yloxymethyl]-benzoic acid methyl ester; 3-[6-(1-Cyclohexyl-2-oxo-pyrrolidin-3-ylmethyl)-naphthalen-2-yloxymethyl]-benzoic acid; 1-Cyclohexyl-3-{6-[3-(4-methyl-piperazine-1-carbonyl)-benzyloxy]-naphthalen-2-ylmethyl}-pyrrolidin-2-one; 3-(1-Chloro-naphthalen-2-ylmethyl)-1-(*cis*-4-hydroxy-cyclohexyl)-pyrrolidin-2-one; 1-Cyclohexyl-3-(4-methoxy-naphthalen-1-ylmethyl)-pyrrolidin-2-one; 1-Cyclohexyl-3-(4-hydroxy-naphthalen-1-ylmethyl)-pyrrolidin-2-one; 1-Cyclohexyl-3-(2-methoxy-naphthalen-1-ylmethyl)-pyrrolidin-2-one; 1-Cyclohexyl-3-(2-hydroxy-naphthalen-1-ylmethyl)-pyrrolidin-2-one; 3-(5-bromo-6-methoxy-naphthalen-2-ylmethyl)-1-cyclohexyl-pyrrolidin-2 one; 3-(5-Bromo-6-hydroxy-naphthalen-2-ylmethyl)-1-cyclohexyl-pyrrolidin-2-one; 1-Cyclohexyl-3-(7-methoxy-naphthalen-2-ylmethyl)-pyrrolidin-2-one; 1-Cyclohexyl-3-(7-hydroxy-naphthalen-2-ylmethyl)-pyrrolidin-2-one; 1-Cyclohexyl-3-(6-isopropoxy-naphthalen-2-ylmethyl)-pynolidin-2-one; 3-(6-Bromo-naphthalen-2-ylmethyl)-1-cyclohexyl-pyrrolidin-2-one; 1-Cyclohexyl-3-naphthalen-2-ylmethyl-piperidin-2-one; and 1-Cyclohexyl-3-methyl-3-naphthalen-1-ylmethyl-pyrrolidin-2-one.

Embodiments of the invention include compounds of the following formulae, $X_1$-$X_{65}$ below, including all racemates, stereoisomers, enatiomers, and diasteriomers, and pharmaceutically acceptable salts thereof.

| | |
|---|---|
| $X_1$ | 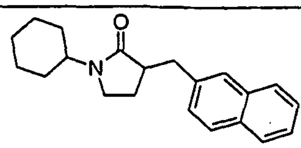 |
| $X_2$ | 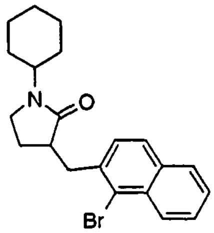 |
| $X_3$ | 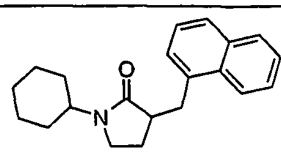 |
| $X_4$ | 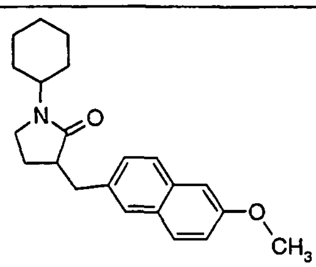 |
| $X_5$ | 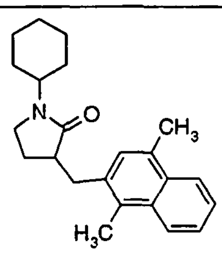 |
| $X_6$ | 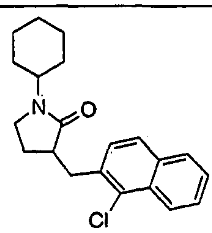 |
| $X_7$ | 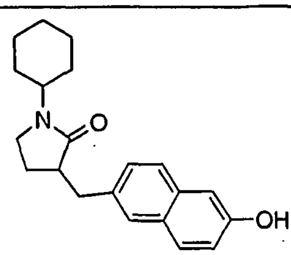 |

| | |
|---|---|
| $X_8$ | 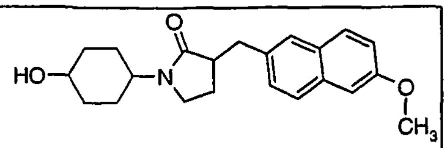 |
| $X_9$ | 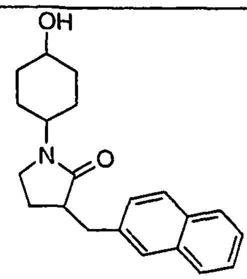 |
| $X_{10}$ | 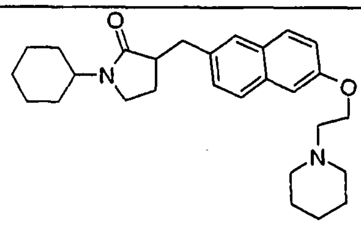 |
| $X_{11}$ | 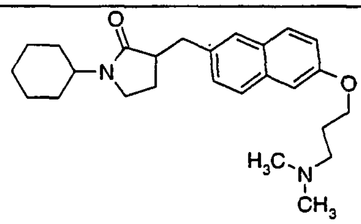 |
| $X_{12}$ | 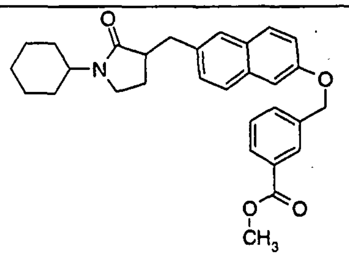 |
| $X_{13}$ | 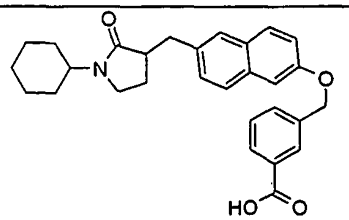 |

| | |
|---|---|
| $X_{14}$ | 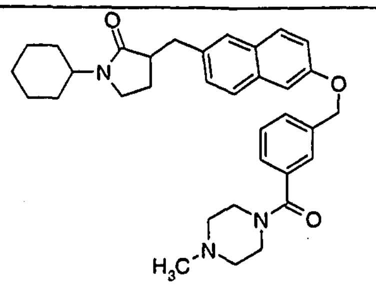 |
| $X_{15}$ | 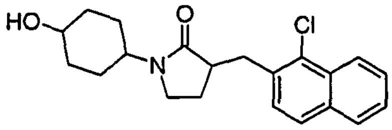 |
| $X_{16}$ | 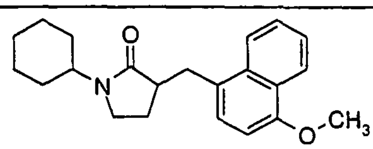 |
| $X_{17}$ | 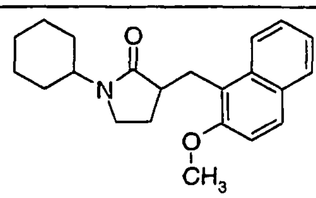 |
| $X_{18}$ | 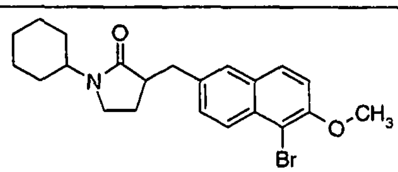 |
| $X_{19}$ | 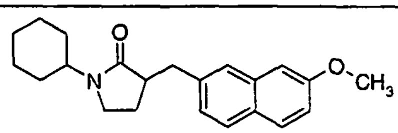 |
| $X_{20}$ | 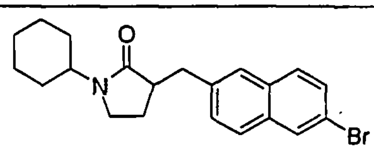 |
| $X_{21}$ | 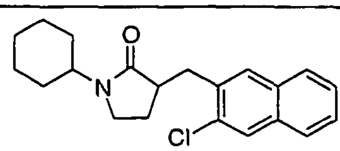 |
| $X_{22}$ | 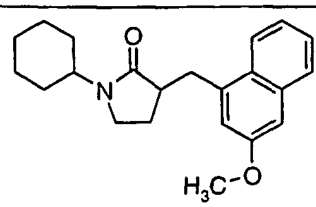 |

| | |
|---|---|
| $X_{23}$ | 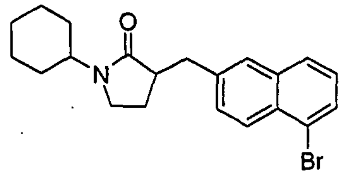 |
| $X_{24}$ | 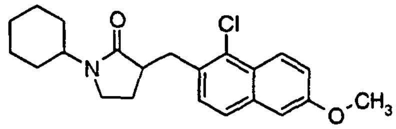 |
| $X_{25}$ | 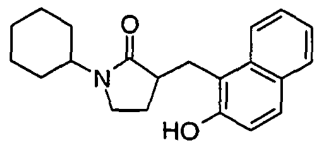 |
| $X_{26}$ | 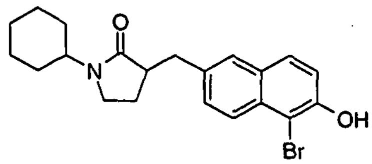 |
| $X_{27}$ | 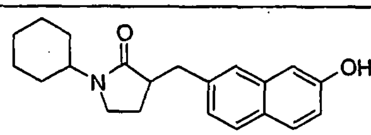 |
| $X_{28}$ | 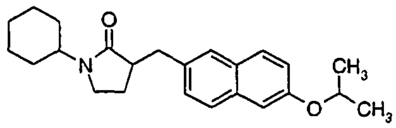 |
| $X_{29}$ | 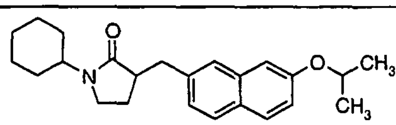 |
| $X_{30}$ | 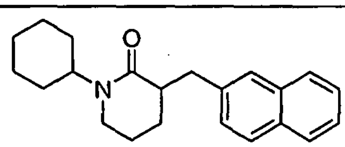 |
| $X_{31}$ | 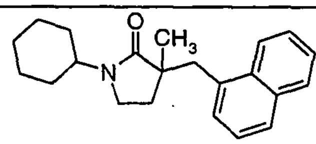 |
| $X_{32}$ | 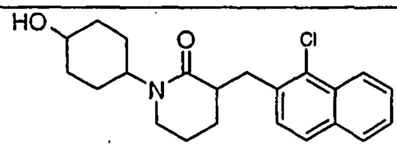 |
| $X_{33}$ | 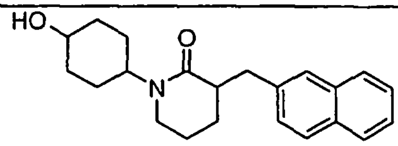 |

| | |
|---|---|
| $X_{34}$ | 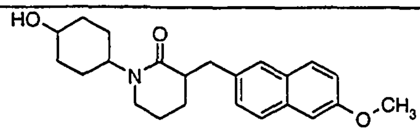 |
| $X_{35}$ | 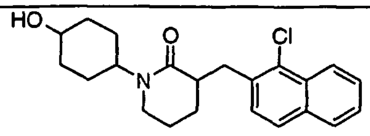 |
| $X_{36}$ | 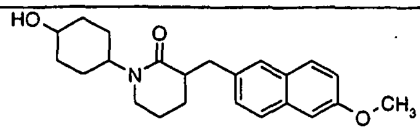 |
| $X_{37}$ | 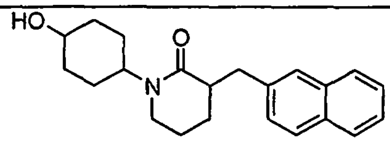 |
| $X_{38}$ | 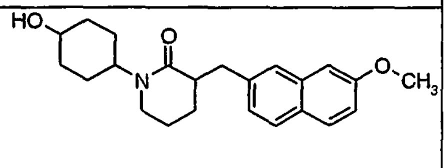 |
| $X_{39}$ | 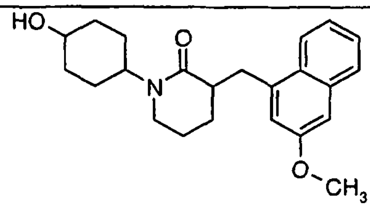 |
| $X_{40}$ | 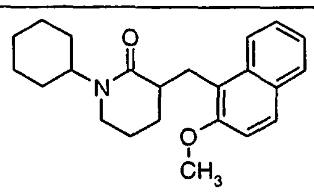 |
| $X_{41}$ | 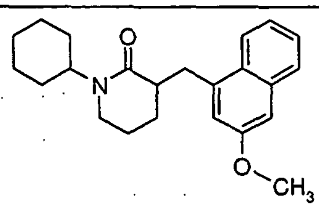 |
| $X_{42}$ | 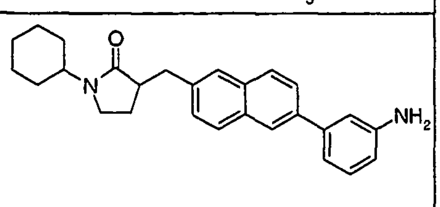 |
| $X_{43}$ | 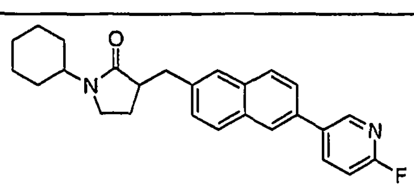 |

| | |
|---|---|
| $X_{44}$ | 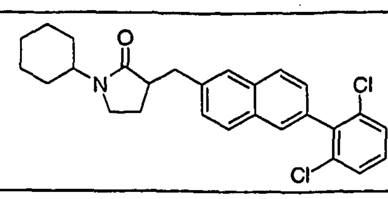 |
| $X_{45}$ | 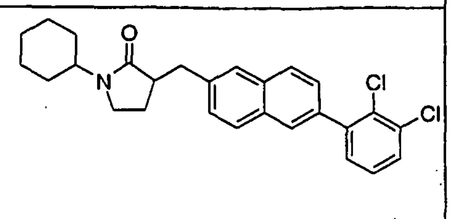 |
| $X_{46}$ | 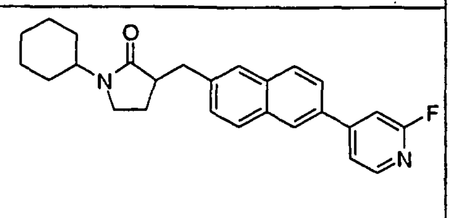 |
| $X_{47}$ | 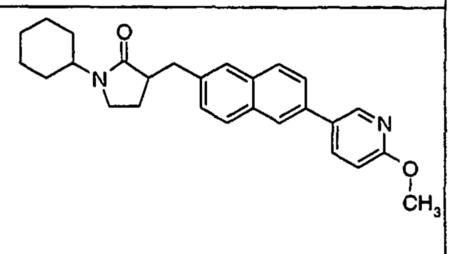 |
| $X_{48}$ | 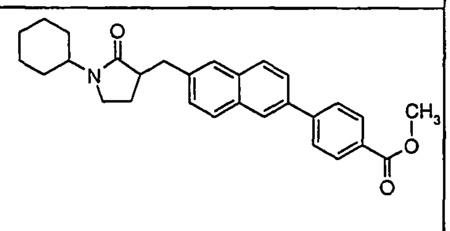 |
| $X_{49}$ | 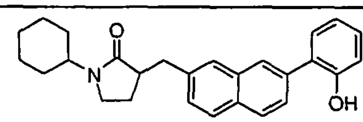 |
| $X_{50}$ | 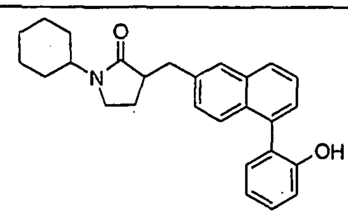 |
| $X_{51}$ | 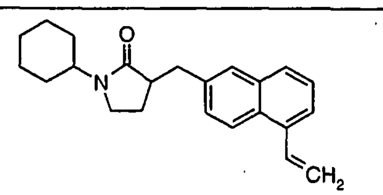 |

| | |
|---|---|
| $X_{52}$ | 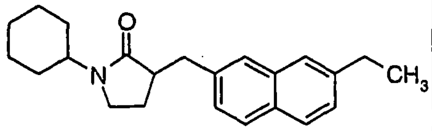 |
| $X_{53}$ | 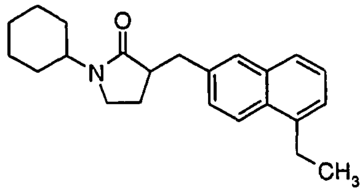 |
| $X_{54}$ | 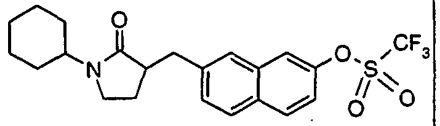 |
| $X_{55}$ | 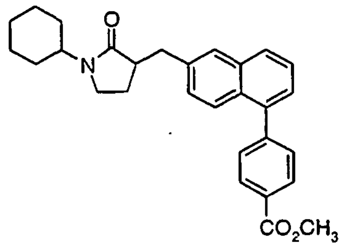 |
| $X_{56}$ | 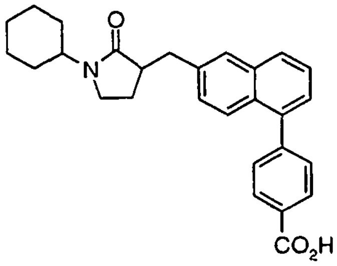 |
| $X_{57}$ | 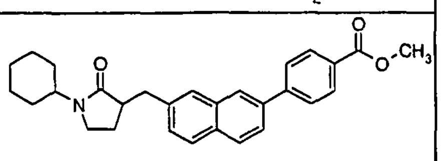 |
| $X_{58}$ | 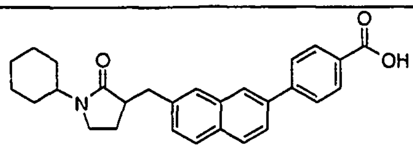 |
| $X_{59}$ | 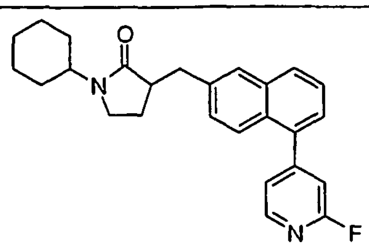 |
| $X_{60}$ | 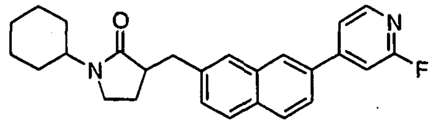 |
| $X_{61}$ | 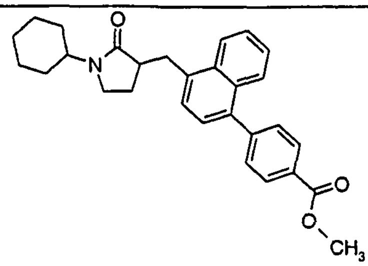 |
| $X_{62}$ | 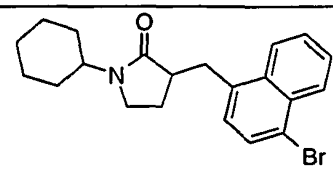 |
| $X_{63}$ | 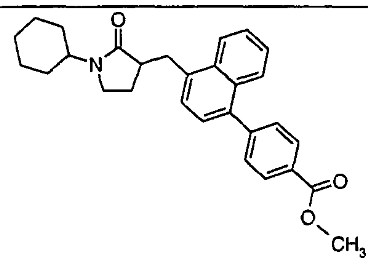 |
| $X_{64}$ | 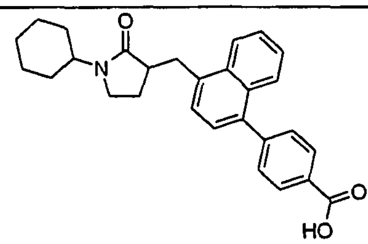 |
| $X_{65}$ | 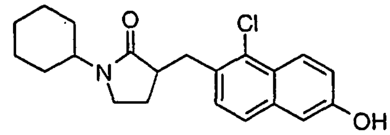 |

The compounds of Formula I, can be prepared by one of ordinary skill in the art following a variety of procedures,

some of which are illustrated in the procedures and schemes set forth below. The particular order of steps required to produce the compounds of Formula I is dependent upon the particular compound to being synthesized, the starting compound, and the relative liability of the substituted moieties. The reagents or starting materials are readily available to one of skill in the art, and to the extent not commercially available, are readily synthesized by one of ordinary skill in the art following standard procedures commonly employed in the art, along with the various procedures and schemes set forth below.

The following Schemes, Preparations, Examples and Procedures are provided to better elucidate the practice of the present invention and should not be interpreted in any way as to limit the scope of the same. Those skilled in the art will recognize that various modifications may be made while not departing from the spirit and scope of the invention. All publications mentioned in the specification are indicative of the level of those skilled in the art to which this invention pertains.

The optimal time for performing the reactions of the Schemes, Preparations, Examples and Procedures can be determined by monitoring the progress of the reaction via conventional chromatographic techniques. Furthermore, it is preferred to conduct the reactions of the invention under an inert atmosphere, such as, for example, argon, or, particularly, nitrogen. Choice of solvent is generally not critical so long as the solvent employed is inert to the ongoing reaction and sufficiently solubilizes the reactants to effect the desired reaction. The compounds are preferably isolated and purified before their use in subsequent reactions. Some compounds may crystallize out of the reaction solution during their formation and then collected by filtration, or the reaction solvent may be removed by extraction, evaporation, or decantation. The intermediates and final products of Formula I may be further purified, if desired by common techniques such as recrystallization or chromatography over solid supports such as silica gel or alumina.

The skilled artisan will appreciate that not all substituents are compatible with all reaction conditions. These compounds may be protected or modified at a convenient point in the synthesis by methods well known in the art.

The terms and abbreviations used in the instant Schemes, Preparations, Examples and Procedures have their normal meanings unless otherwise designated. For example, as used herein, the following terms have the meanings indicated: "eq" refers to equivalents; "N" refers to normal or normality, "M" refers to molar or molarity, "g" refers to gram or grams, "mg" refers to milligrams; "L" refers to liters; "mL" refers to milliliters; "μL" refers to microliters; "mol" refers to moles; "mmol" refers to millimoles; "psi" refers to pounds per square inch; "min" refers to minutes; "h" or "hr" refers to hours; "°C" refers to degrees Celsius.

"TLC" refers to thin layer chromatography; "HPLC" refers to high performance liquid chromatography; "$R_f$" refers to retention factor; "$R_t$" refers to retention time; "δ" refers to part per million down-field from tetramethylsilane; "MS" refers to mass spectrometry, Observed Mass indicates [M+H] unless indicated otherwise. "MS(FD)" refers to field desorption mass spectrometry, "MS(IS)" refers to ion spray mass spectrometry, "Mass spectrum (ion spray)" refers to ion-spray ionization mode. "MS(FIA)" refers to flow injection analysis mass spectrometry, "MS(FAB)" refers to fast atom bombardment mass spectrometry, "MS(EI)" refers to electron impact mass spectrometry, "MS(ES)" refers to electron spray mass spectrometry, "MS (EI)" refers to electron impact mass spectrometry-electrospray ionization, "MS (ES+)" refers to mass spectrometry-electrospray ionization, "MS(APCi) refers to atmospheric pressure chemical ionization mass spectrometry, "UV" refers to ultraviolet spectrometry, "$^1$H NMR" refers to proton nuclear magnetic resonance spectrometry. "LC-MS" refers to liquid chromatography-mass spectrometry, "GC/MS" refers to gas chromatography/mass spectrometry. "IR" refers to infra red spectrometry, and the absorption maxima listed for the IR spectra are only those of interest and not all of the maxima observed. "RT" refers to room temperature.

"THF" refers to tetrahydrofuran, "LAH" refers to lithium aluminum hydride, "LDA" refers to lithium diisopropylamide, "DMSO" refers to dimethylsulfoxide, "DMF" refers to dimethylforamide, "HCl" refers to hydrochloric acid, "EtOAc" refers to ethyl acetate, "Pd-C" refers to palladium on carbon, "DCM" refers to dichloromethane, "DMAP" refers to dimethylaminopyridine, "LiHMDS" refers to Lithium Hexamethyldisilisane, "TFA" refers to trifluoroacetic acid, "EDAC" refers to *N*-Ethyl-*N*'-(3-dimethylaminopropyl)carbodiimide hydrochloride, "HOBT" refers to 1-Hydroxy benzotriazole, "Bn-9-BBN" refers to Benzyl -9-borabicyclo[3.3.1]nonane, "Pd(dppf)$Cl_2$" refers to [1,1'-Bis(diphenylphosphino)-ferrocene)dichloropalladium(II), "EDCI" refers to *N*-Ethyl-*N*'-(3-dimethylaminopropyl)carbodiimide hydrochloride, "DBU" refers to 1,8-Diazabicyclo[5.4.0]undecene-7, "TBSCl" refers to tert-butyl-dimethyl-silanyloxymethyl chloride, "NBS" refers to N-Bromosuccinimide, "TsOH" refers to p-toluenesulfonic acid, "DCE" refers to dichloroethane, "DAST" refers to (Diethylamino) sulfur trifluoride, "EA/H" refers to ethyl acetate/hexanes mixture, "$Pd_2(dba)_3$" refers to Bis(dibenzylideneacetone)palladium, "BINAP" refers to 2,2'-Bis(diphenylphospino-1,1'-binaphthalene, "NMP" refers to N-Methylpynollidine, "TMSCN" refers to Trimethylsilyl cyanide, "TBAF" refers to Tetrabutylammonium fluoride, "$Tf_2O$" refers to trifluoromethanesulfonic anhydride, "TBSO" refers to tert-butyl-dimethyl-silanyloxy, "OTf" refers to trifluoromethanesulfonate, MeTi(Oi-Pr)$_3$ refers to methyltitanium triisopropoxide. "DPPA" refers to diphenylphosphorylazide. "DME" refers to dimethoxyethane. "DIAD" refers to diisopropyl azodicarboxylate. In a structure, "Ar" refers to aryl, "Ph" refers to phenyl, "Me" refers to methyl, "Et" refers to ethyl, "Bn" refers to benzyl, and "MeOH" refers to methanol.

General Procedures

Compounds of the present invention are formed as specifically described in the examples. Alternative synthesis methods may also be effective and known to the skilled artisan. Unless otherwise indicated, all variables, such as L, $G^1$, $R^1$ to $R^{20}$, etc., are as defined for analogous variables in the summary of the invention, and otherwise as defined herein.

Scheme A

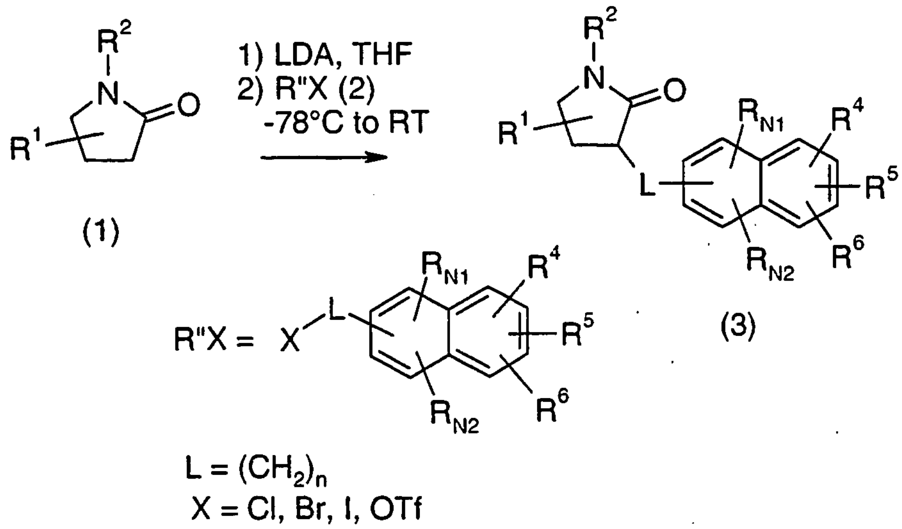


In Scheme A the lactam (1) is conjugated with an alkylating agent R"X (2) to give (3). The reaction is carried out using lithium diisopropylamide (LDA) to form the lithium anion of the lactam but other bases could be used (lithium hexamethyl disilazide, sodium hydride, phosphazenes, potassium *tert*-butoxide) (Conditions used are a modification of the conditions to alkylate 1-methyl-pyrrolidinone, see: Hullet, P. et al. Can. J. Chem. (1976) 54, 1098-1104; For use of phosphazenes in alkylation of lactams, see: Goumri-Magnet et al. J. Org. Chem. (1999) 64, 3741-3744). The reaction is carried out in THF but other solvents could be used (i.e.; dichloromethane, ether, toluene, etc. to facilitate solubility of the components). The reaction can be run with either an excess of the lactam and LDA or with an excess of the alkylating agent. The ease of purification of the product from the starting materials and the relative expense of the components and the preference of the chemist led to different choices of which ratios of starting materials to use. In general the reaction affords good to moderate yields of product; especially for benzylic alkylating reagents. The reaction is initiated at temperatures of -78˚C and warmed to room temperature. Depending on the reactivity of the alkylating reagent, the time varies. Alkyl alkylating agents take longer (1-3 hours or more, while the subset of benzyl alkylating agents proceed rapidly at -78˚C (<15 minutes). The alkylating agents to date have been halides; generally the iodides or bromides but occasionally the chlorides; however one skilled in the art would recognize that tosylates, triflates, nosylates, and other alkylating agents would work. When $R^1$ is not hydrogen, the major product of the alkylation is the *trans*-isomer and this is the preferred method for the preparation of these compounds.

Scheme B

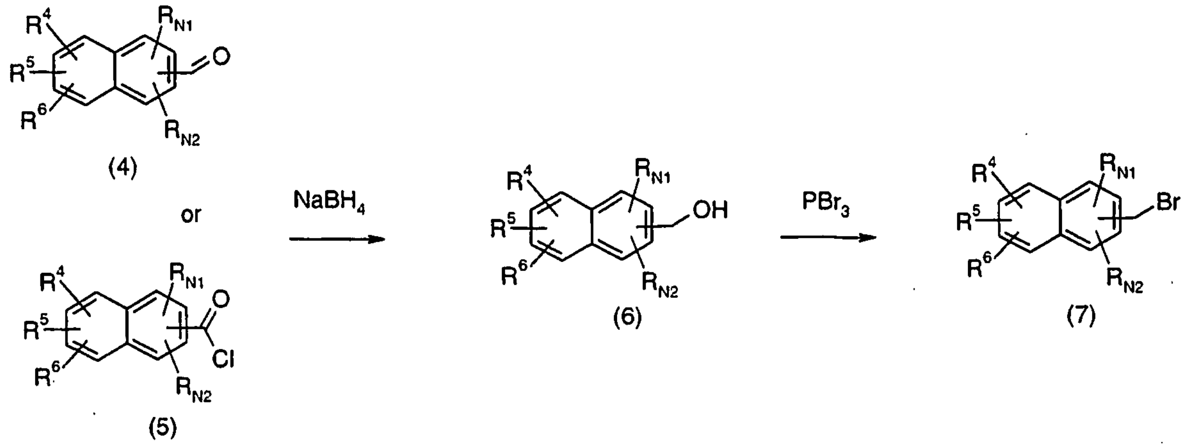


(4) or (5) → (6) → (7)

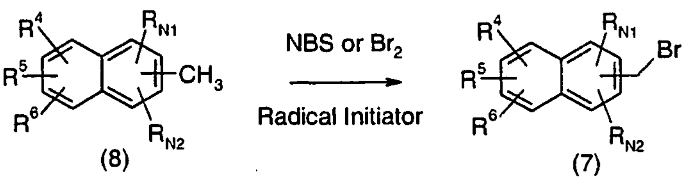


(8) → (7)

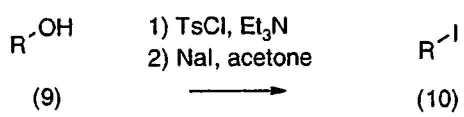


(9) → (10)

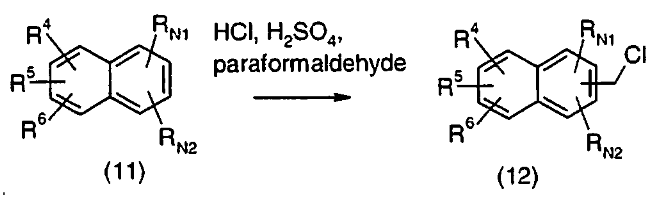


(11) → (12)

In Scheme B the naphthylating agents (7) can be prepared by modifications of a variety of literature conditions a few of which are illustrated here. Substituted naphthylaldehydes (4) or substituted naphthoyl chlorides (5), which are readily available from the corresponding naphthenoic acids with thionyl chloride or oxalyl chloride, are reduced readily by dropwise addition into a mixture of sodium borohydride in ethanol/THF to form the substituted naphthylic alcohols (6). Conversion of the substituted naphthylic alcohols (6) to the bromides (7) can generally be achieved by adding a moderate excess of phosphorous tribromide to a solution of the alcohol in a solvent (either ether or dichloromethane have been used; but others compatible with phosphorous tribromide would work). Other literature procedures can effect the conversion of (6) to (7); i.e.; treatment with HBr in AcOH with some substrates; conversion of the alcohol to a mesylate followed by Br- displacement, or treatment with $CBr_4$ and triphenylphosphine to name but three of many possibilities. The naphthylic iodides and chlorides can be made by trivial modifications of the above procedures.

In cases where there is but one alkyl moiety attached to the aryl moiety as in (8), conversion of the methyl moiety to the naphthyl halide (7) can be effected by treatment with a radical precursor (AIBN, naphthyl peroxide, a peroxide, etc.) in a suitable solvent with a bromide radical precursor (NBS, bromine, etc.) to afford the naphthyl bromide (7). Replacement of the bromide radical precursor with a chloride or iodide radical precursor can afford the corresponding naphthylic halides.

In cases where R is not naphthylic, the alkyl iodides are generally the best alkylating agent for the reaction in General Scheme A. A versatile method of preparing these alkylating partners is to first make the tosylate (triflate and mesylate with alternative bases than triethyl amine can also be effectively used) from an alcohol (9) and then displace

the tosylate with iodide ion in acetone.

Naphthylic chlorides in certain cases can be easily made from paraformaldehyde or freshly cracked formaldehyde or another formaldehyde synthetic equivalent via acid catalyzed aromatic substitution. This procedure is most efficient with electron rich naphthyl rings (11) to form the newly formed $-CH_2Cl$ bond at the most electron rich position of the naphthyl ring (J. Med Chem. (1988) 31, 72-83).

## Scheme C

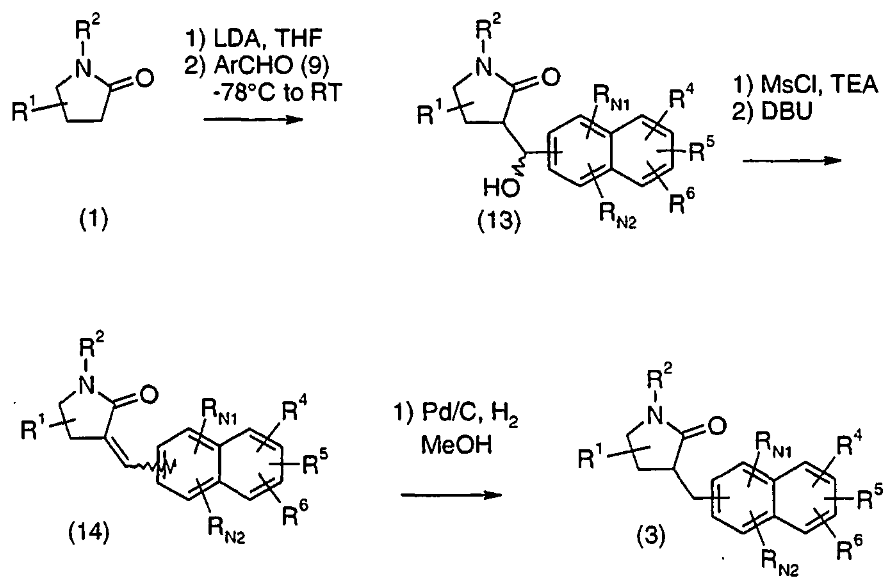


(1) (13) (14) (3)

In Scheme C, an alternative to using an alkylating agent to prepare (3) is described. Substituted lactams (1) can be converted to the alcohols (13) (J. Med. Chem. (1991) 34, 887-900) by treatment of the lactam with LDA followed by treatment with an aldehyde. Alternatively, these alcohols could be access from carboxylic esters via a Claisen reaction to form an intermediate ketone, followed by a hydride reduction (Liebigs, Ann. Chemie. (1983) 165-180). Elimination of the alcohol to the $\alpha,\beta$-unsaturated lactam (14) can be effected by formation of the mesylate with methanesulfonyl chloride and triethyl amine as base; followed by treatment with DBU (Chem. Pharm. Bull. (1990) 38 393-399). Other conditions to affect this transformation (i.e.; different bases to substitute for triethyl amine or DBU or different activation agents to replace DBU) could be used and should be obvious to those trained in the art. Reduction of the double bond moiety of (14) by catalytic hydrogenation affords (3). Obviously, catalytic hydrogenation could potentially be replaced with 1,4-conjugate addition of hydride or alkyl metal species to form (3) or alkylated variants thereof.

When $R^1$ does not equal hydrogen, the major compound of these reduction is the *cis*-isomer and this is the preferred method for the preparation of these compounds.

## Scheme D

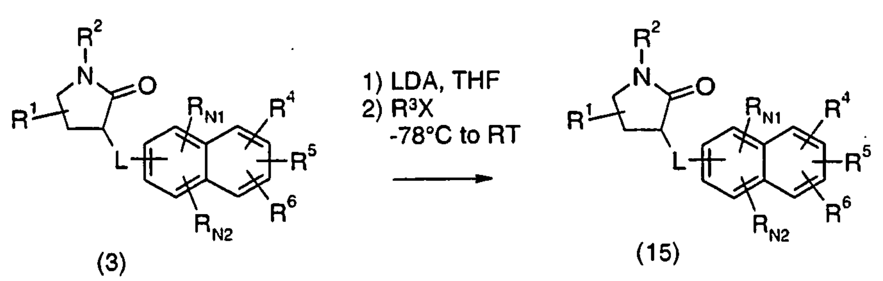


(3) (15)

L = $(CH_2)_n$ , O, S

In Scheme D the lactam (3) is conjugated with an alkylating agent (2) to give (15). As in the case of Scheme

A, other bases and solvents can be used. When $R^1$ does not equal H, the major product has a *trans*-relationship between the 3-substitutent on the lactam and $R^1$. It is obvious to those trained in the art that both isomers of (15) when L = $(CH_2)_n$ can be preferentially made as the major product by judicious choice of which alkylating agent, $R^3X$ or ArLX, to introduce first.

Scheme E

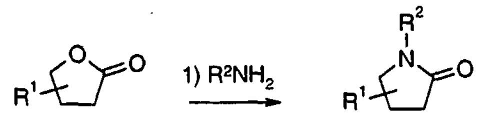


(16) (1)

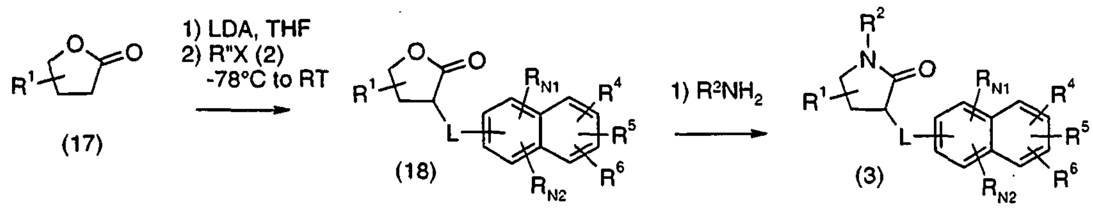


(17) (18) (3)

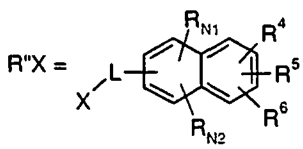


L = $(CH_2)_n$
X = Cl, Br, I, OTf

In Scheme E the butyrolactone (16) is reacted with a primary amine to form the lactam starting material (J. Am. Chem. Soc. (1947) 69, 715-716). A large number of primary amines can be utilized in this procedure. Primary amines; i.e., especially preferred are: substituted cycloalkyl amines (substituted with alkyl, amine, alcohols, etc), and fused bi- and tri-cyclic amines (i.e., adamantyl, norborenyl, camphoryl, etc) may be used. The reaction proceeds in two steps and involves a thermal elimination of water at high temperature. No solvent is necessary; but a high boiling solvent could be added if perceived to be desirable. It should be noted that if $R^3$ is at the 3-position of the pyrrolidinone, then the product is the same as (3) and an alkylation is not necessary. This procedure is done as shown in the second synthetic depiction in Scheme E. Alkylation of the lactone (17) with LDA and an alkylating agent using the conditions of Scheme A affords (18) and condensation with the amine under thermal conditions without solvent forms (3) directly.

Scheme F

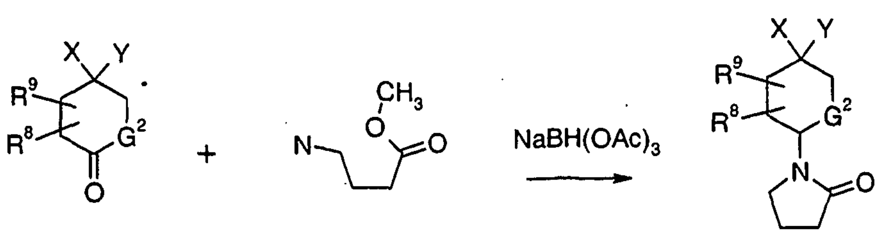


(19) (20) (21)

In Scheme F, cyclic ketones (19) are condensed with methyl 4-aminobutyrate hydrochloride (20) in a reductive amination with sodium triacetoxyborohydride to afford the lactams (21) (Syn Lett. (1994) 81-83). The reaction is done using a modification of the conditions described by Marynoff et al. The solvent is 1,2-dichloroethane and the reaction takes 1-4 days to complete depending upon the ketone. In some cases, the crude product is heated to reflux in toluene to force the ring closure and drive the reaction to completion. This cyclization can be done with 5-, 6-, and 7-member

ring ketones (19); substituted and not, and with ketals (Y and Z connect to form = $OCH_2CH_2O$) on the ring to aid in the further preparation of advanced intermediates.

Scheme G

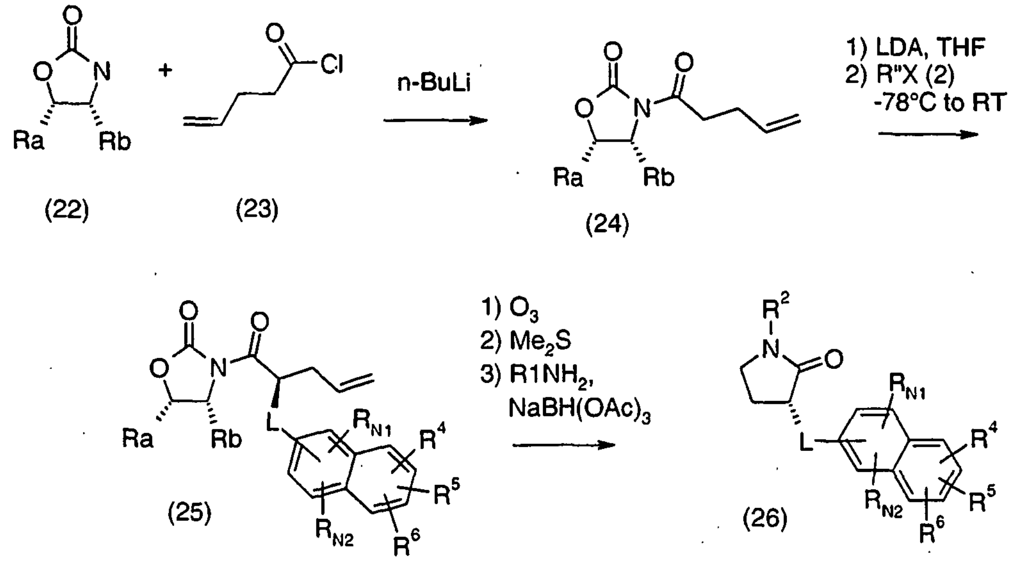


(22) (23) (24) (25) (26)

Ra = phenyl or H
Rb = phenyl or benzyl

In Scheme G, a route to chiral 3-substituted lactams is shown. Acylation of the chiral auxiliary (22) with pent-4-enoyl chloride (acylation with longer unsaturated acyl chlorides would give 6-and higher member ring lactams via analogy) affords the imide (24). Alkylation of the imide (24) using the general alkylation conditions of general Scheme A affords in high diastereomeric excess the drawn diastereomer (25). It is probable that other chiral auxillaries similar to (22) could be utilized with similar or higher diastereomeric excess. Ozonolysis of the olefin affords an aldehyde intermediate that is immediately reductively cyclized with a primary amine in conditions similar to those of Scheme F to afford the lactam (26) *(*Bioorg. Med. Chem. Lett. (2003) 2035-2040). Of course, utilization of the other enantiomer of (22) gives the other enantiomer of (26) and both enantiomers are claimed. In Scheme G, when Rb is benzyl, then Ra is H and when Rb is phenyl, then Ra is phenyl.

Scheme H

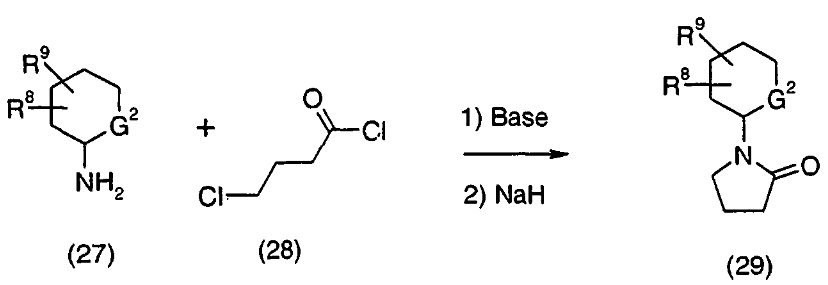


(27) (28) (29)

In Scheme H, substituted cyclohexyl amines are acylated with 4-chlorobutyryl chloride using triethylamine, pyridine, or another appropriate acid scavenger base. The second cyclization sometimes occurs in this acylation, but usually a stronger base such as NaH or KH is necessary to effect the second cyclization. Other strong bases such as *tert*-BuOK could potentially be used. This procedure is particularly effective to make lactams with a 1-alkyl substituent on the cyclic amine moiety.

## Scheme I

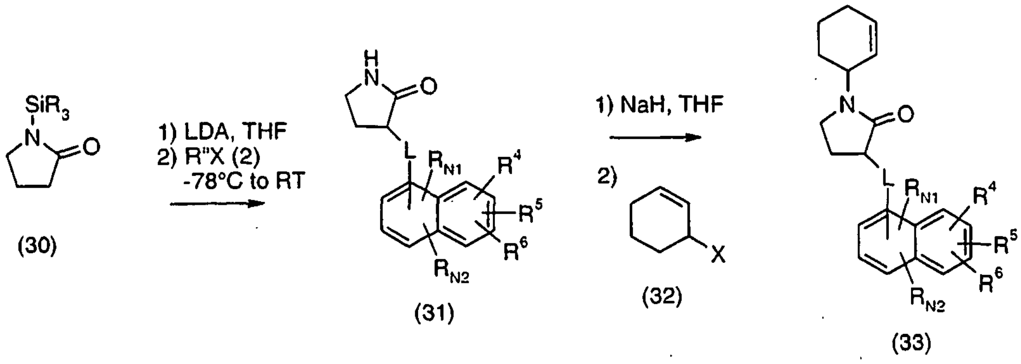


(30) (31) (32) (33)

In Scheme I, the silylated lactam (30) is alkylated via treatment with LDA, followed by treatment with an alkylated agent (2) in conditions similar to Scheme A. The silyl moiety is removed in the aqueous workup of the reaction. The substituted lactam product (31) can be *N*-alkylated by treatment with NaH in THF with a substituted or unsubstituted 3-halo-cyclohex-1-ene.

## Scheme J

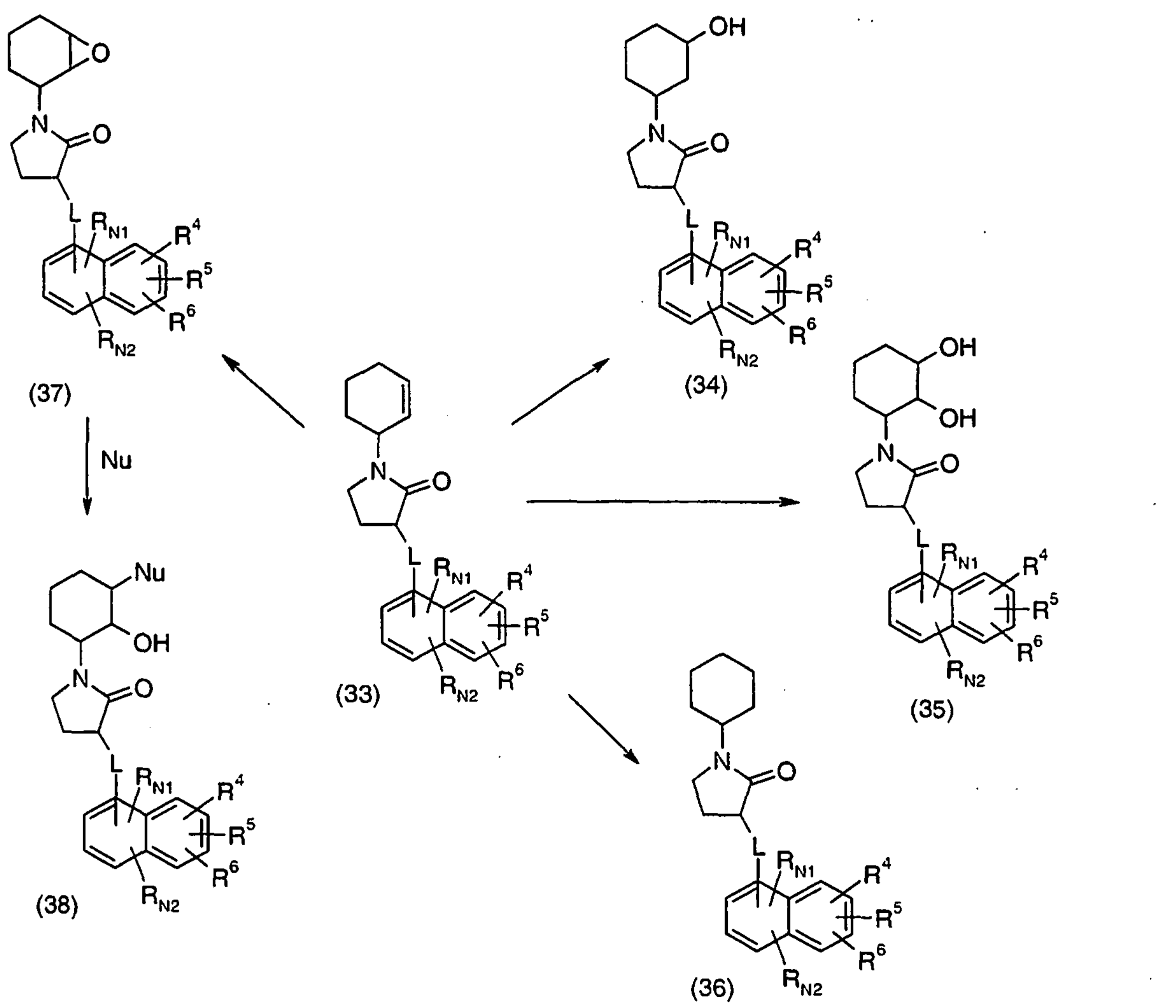


(37) (34) (35) (33) (38) (36)

In Scheme J, the cyclohexenyl product (33) can be optionally oxidized via literature procedures to cyclohexyl

alcohols (34), diols (35), reduced to the cyclohexyl moiety (36), or be oxidized to an epoxide intermediate (37). Epoxide intermediate (37) can be further functionalized with a nucleophile to form substituted alcohols (38).

Scheme K

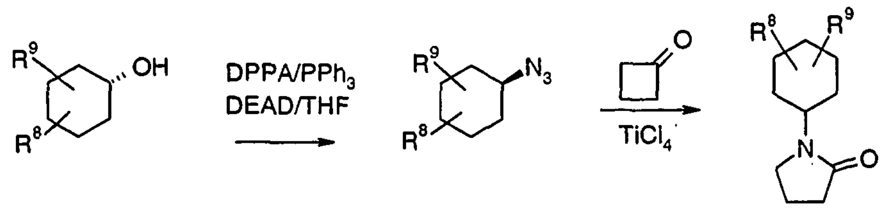


(39) (40) (41)

In Scheme K, substituted cyclohexyl alcohols (39), which are readily available either commercially or by known literature procedures can be converted to azides via treatment with diphenylphosphoryl azide (DPPA) and triphenyl azide and DEAD in THF to form the azide (40). During this reaction, the relative stereochemistry of the starting alcohol is inverted and is obvious to those trained in the art. Treatment of the azide (40) with butyrylactone forms the lactam (41). During the Schmidt reaction the relative stereochemistry of the N-moiety to the substituents $R^8$ and $R^9$ is conserved as is obvious to those trained in the art and is illustrated in the examples below.

Scheme L

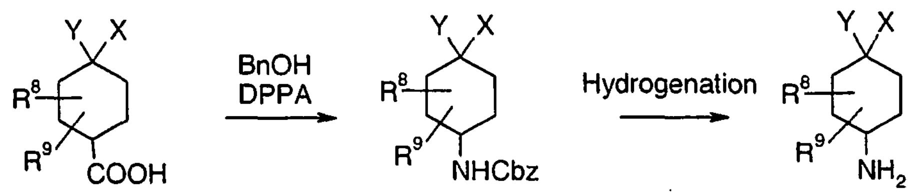


(42) (43) (44)

In Scheme L, a variety of substituted cyclohexyl amines can be easily acquired from the substituted carboxylic acids (42), which are easily prepared via known literature methods [i.e., alkylation of a parent carboxylic acid with RX (X = halide or triflate)]. In this procedure the carboxylic acid is first subjected to Curtius rearrangement in the presence of benzyl alcohol to form the CBZ carbamate (43). In this reaction the relative stereochemistry of the starting material (42) is conserved as is obvious to those trained in the art. Hydrogenation of the CBZ carbamate forms the amine (44). A variety of hydrogenation conditions can be used to effect this transformation as is obvious to those trained in the art (i.e.; see Green's protecting group book for numerous conditions) (Greene, Protective Groups in Organic Synthesis, John Wiley & Sons, New York, N.Y.). These amine starting materials (44) are useful starting materials for Schemes E, G, H, and M to prepare the claimed lactams.

Scheme M

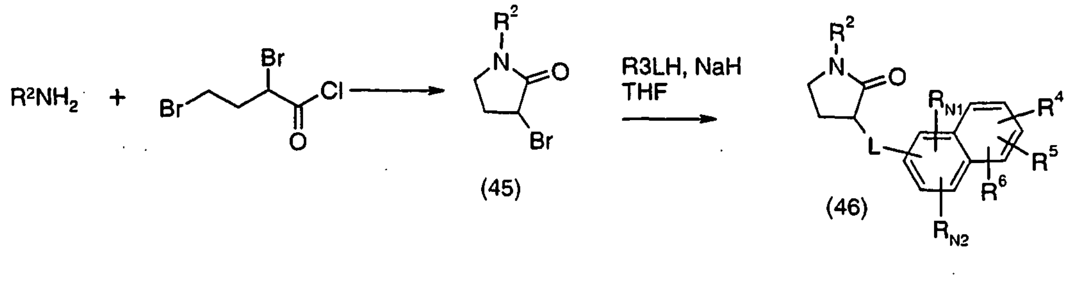


(45)

(46)

In Scheme M, amines are bis acylated and cyclized with 2,4-dibromobutyryl chloride to produce the N-alkylated-3-bromopyrrolidinones (45) in good yield (J. Med. Chem. (1987) 30, 1995-1998). The bromide can be displaced by naphthenols, thionaphthenols, naphthylamines, alcohols, thiols, and amines to form the lactams (46) [L = O, S, NH, NR].

Scheme N

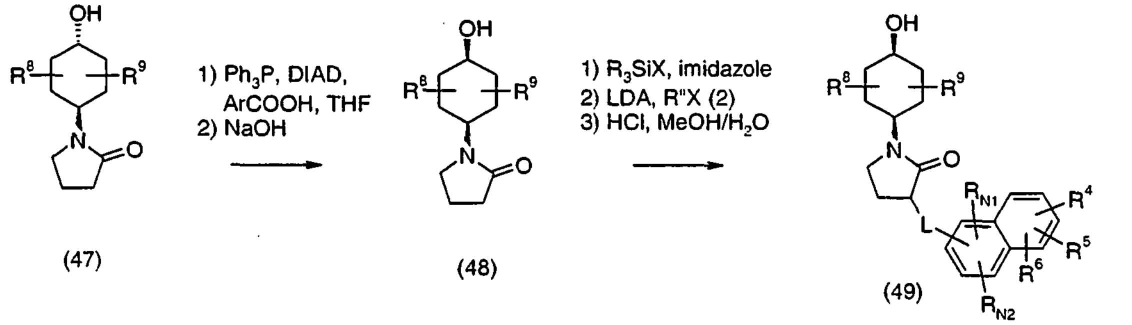


(47)

(48)

(49)

In Scheme N, a hydroxyl substituted lactam (47) stereochemistry is inverted with the Mitsunobu reaction to form its diastereomer (48) (*trans* to *cis* conversion illustrated here; but the reverse could easily be done). The alcohol substituted lactams are conveniently alkylated by first protecting the alcohol moiety with a silyl protecting group (TBS used but a variety of protecting groups from Green's Protecting Groups in Org Synthesis could be employed), and then alkylated employing the conditions of Scheme A. Deprotection of the alcohol with appropriate conditions (acid/HCl or fluoride deprotection of silyl moieties are convenient) yield the hydroxylated lactams (49).

## Scheme O

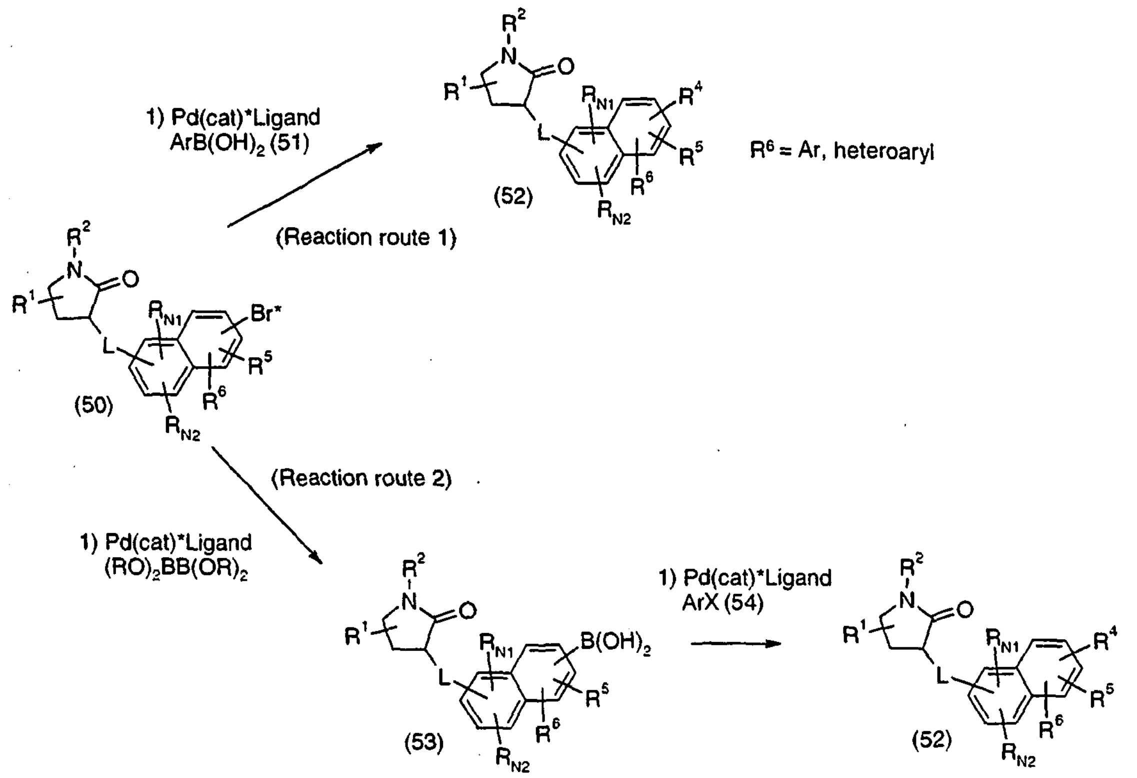


$R^6$ = Ar, heteroaryl

In Scheme O, aryl "Br" lactams (50) (bromine as indicated by Br* can also be I, Cl, or OTf and this same chemistry would produce the drawn compounds with the appropriate catalysts known in the literature by those trained in the art) are converted to the biaryl/aryl-heteroaryl compounds by coupling to the appropriate aryl/heteroaryl boronic acid (51) via reaction route 1 to produce the lactams (52) directly. The linker (L) can be any of the following [$CH_2$, CHR, O, S, NH, or $(CH_2)_n$]. It is also convenient to make the boronic acid convergent intermediate (53) and couple with the appropriate Pd(cat) ligand system with a variety of aryl/heteroaryl halides/triflates (54) to form the lactams in two steps as shown in reaction route 2. This route is convenient and more versatile if the boronic acids (51) are not easy to prepare or acquire from commercial sources or literature methods.

## Scheme P

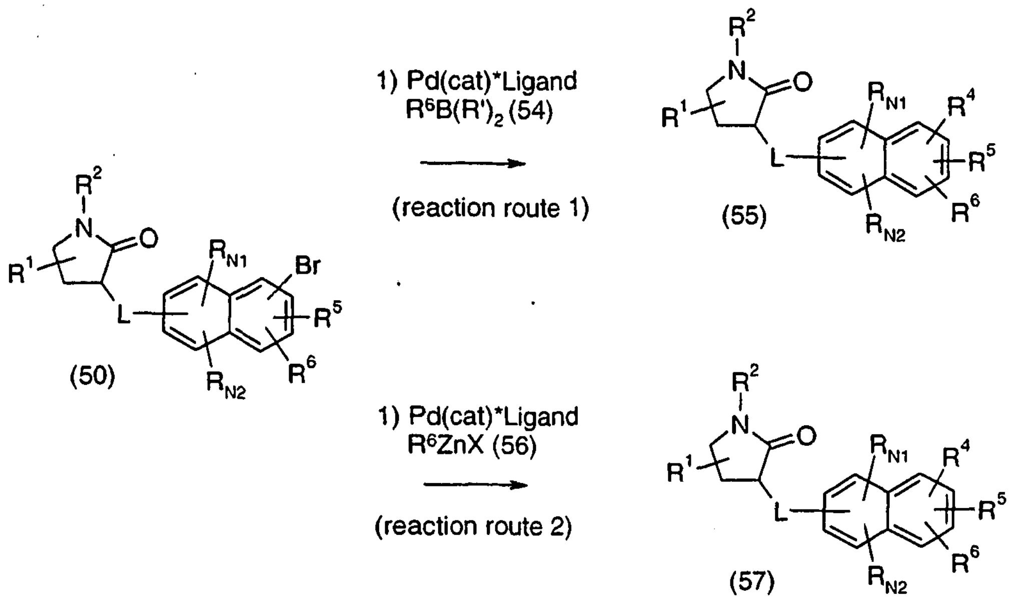


(50) (55) (57)

In Scheme P, the aryl bromides (Br could also be expected to be replaced with OTf, I) are conveniently converted into substituted arylalkyl lactams (eg., Br, I, Cl or Tf conversion to alkyl). This conversion (reaction route 1) is achieved via Pd-catalyzed insertion of $(R^6)_3B$ (54) or BBN-R6 [a subclass of (54) (made from either BBN-H regioselective addition to primary alkenes, or via organometalic addition of R6-Metal to BBN-OMe)]. In reaction route 2, organometallic conversion to introduce alkyl moieties containing nitrile, ester and other functionality is a Pd-catalyzed Negishi insertion of an $R^6$zinc halide (56) to the halide/triflate (50) to produce the lactams (57). This route is the preferred method for preparation of compounds of the structure where $R^6 = (CH_2)_nFG$ (FG = COOR, CN). A similar Negishi reaction has been used to produce (57) where $R^6$=CN when $R^6ZnX$ (56) is replaced with ZnCN in the reaction.

Scheme Q

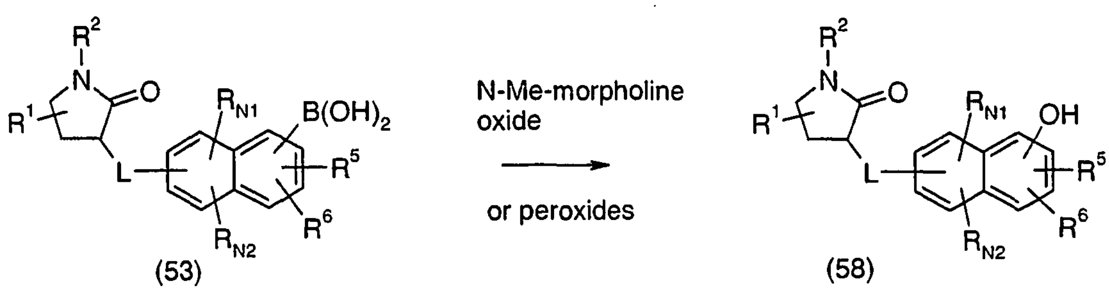


(53) (58)

In Scheme Q, the boronic acids (53) prepared in Scheme O, are conveniently converted into phenols (58) via oxidation with *N*-methyl morpholine oxide in a suitable solvent or via treatment of the boronic acid with another oxidizing agent such as peroxides. Other oxidants known in the literature could likely also be utilized. These phenolic products (58) are useful starting materials in alkylations as in Scheme Q.

## Scheme R

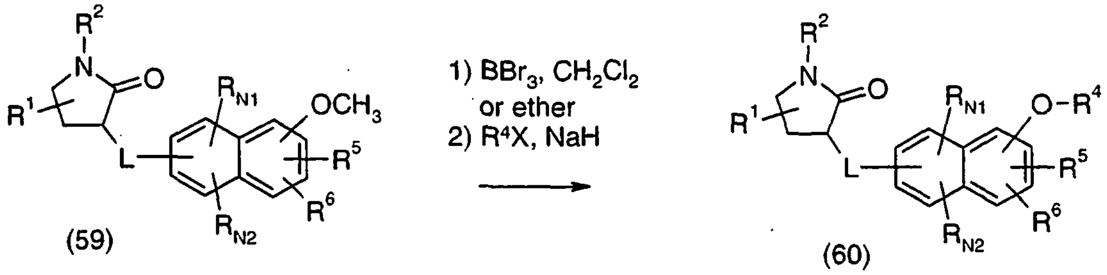


(59) (60)

In Scheme R, the methoxy functionalized lactams prepared via one of the above schemes, can be further elaborated via demethylation of the $OCH_3$ moiety of the lactam (59) to make a phenol (58) which can be alkylated with $(CH_2)_nFG$ (FG = functionalized group) to produce the lactam (60). This chemistry is used to introduce moieties where R = $(CH_2)_nFG$ where FG = ester, acid, primary, secondary, and tertiary amines. It is expected that Mitsunobu reactions of the phenols (59) with alcohols could also produce the lactams (60). This should be the preferred method for more functionalized and sensitive $R^4$-substituents.

## Scheme S

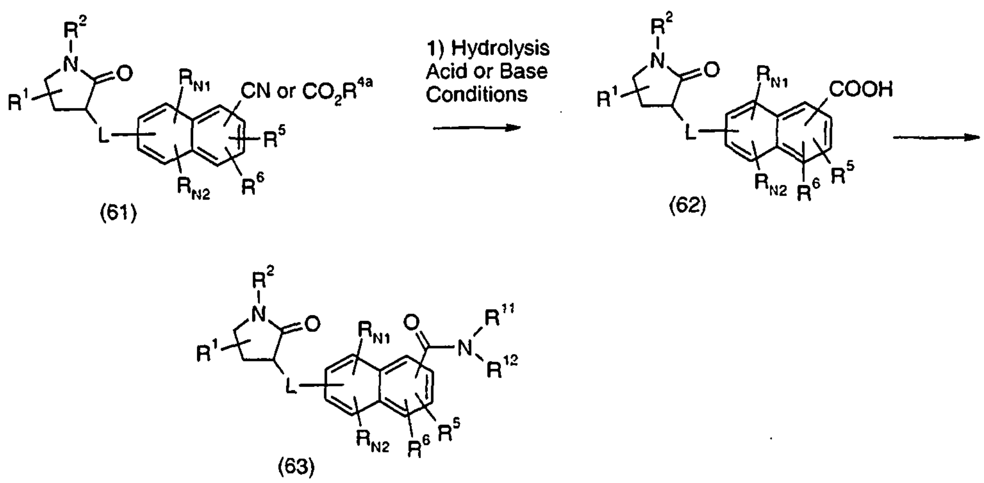


(61) (62)

(63)

In Scheme S, the nitriles (61) prepared via Scheme P) or the esters (61) (on biaryl substituted compounds to date prepared via Scheme O) are hydrolyzed under standard basic or acidic conditions (the optimal condition varies with the sensitivity of $R^5$ and $R^6$) to afford the carboxylic acid compound (62) which is further elaborated using standard dicarbodiimide coupling methods to prepare amides (63). Other amide coupling techniques (which are numerous and known to those trained in the art) would give amides (63).

General Experimental Details

A Varian INOVA 400 MHz spectrometer is used to obtain $^1H$ NMR Specta the in the solvent indicated. A Finnigan LCQ Duo instrument using a mobile phase of 50% acetonitrile, 25% methanol, and 25% 2mM aqueous ammonium acetate is used to obtain the Electrospray mass spectra. A Varian Prostar 210 instrument equipped with a PDA detector is used to run the analytical HPLC. A 5-cm YMC ODS-AQ column with a particle size of 3 microns is used as the stationary phase and 0.1% TFA in water is used as mobile phase A and 0.05% TFA in acetonitrile is used as mobile phase B. The standard method is a gradient of 5 to 95% B over 5 minutes, unless otherwise indicated. Starting materials are either purchased commercially, prepared as described, or prepared by the literature procedure indicated. ChemDraw version 7.0.1 (CambridgeSoft) is used to name the preparations and examples.

**Preparations and Examples**

Preparation 1

1-(*trans*-4-hydroxy-cyclohexyl)-pyrrolidin-2-one

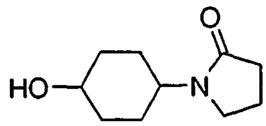

Add *trans*-4-aminocyclohexanol (230 g; 2.0 mol) to γ-butyrolactone (140 mL; 1.82 mol) in a 1L round-bottom flask equipped with large magnetic stirrer, thermometer and condenser/nitrogen bubbler. Heat at 190°C for 68 hours. Cool to ambient temperature and dissolve in water (1L). Extract into dichloromethane (10 x 1.5L). Dry the extracts over magnesium sulfate, filter and evaporate to a brown solid. Triturate with diethyl ether to afford 144.7 g (43%) of the title compound. LC-MS (M+1=184).

Preparation 2

*cis*-4-Nitro-benzoic acid 4-(2-oxo-pyrrolidin-1-yl)-cyclohexyl ester

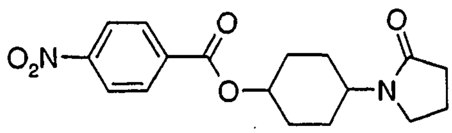

Dissolve 1-(*trans*-4-hydroxy-cyclohexyl)-pyrrolidin-2-one (Preparation 1) (144 g; 0.79 mol) in dry tetrahydrofuran (5L) and cool to -5°C under nitrogen. Add triphenylphosphine (310 g; 1.185 mol) and 4-nitrobenzoic acid (198 g; 1.185 mol). Add diisopropyl azodicarboxylate (230 mL; 1.185 mol) drop-wise and stir at room temperature overnight. Add saturated aqueous sodium hydrogencarbonate (1L) extract into dichloromethane (2 x 2.5L) in a 20L separating funnel. Dry the combined organic layers over magnesium sulfate, filter and concentrate. Purify over silica gel (iso-hexane/ethyl acetate 50-100% then 10% methanol in ethyl acetate) to afford 163 g (62%) of the title compound.

Preparation 3

1-(*cis*-4-hydroxy-cyclohexyl)-pyrrolidin-2-one

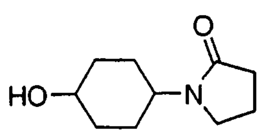

Dissolve *cis*-4-nitro-benzoic acid 4-(2-oxo-pyrrolidin-1-yl)-cyclohexyl ester (Preparation 2) (87.9 g; 264 mmol) in methanol (1.35 L) and water (150 mL) and add potassium carbonate (109.5 g; 800 mmol). Stir at room temperature overnight to give a white precipitate. Evaporate to dryness. Azeotrope with ethanol (x2). Stir in tetrahydrofuran (1L) for 1 hour then filter. Evaporate the filtrate to an oil and crystallize from diethyl ether (100 mL) to afford 40 g (83%) of the title compound.

Preparation 4

*cis*-1-[4-(*tert*-butyl-dimethyl-silanyloxy)-cyclohexyl]-pyrrolidin-2-one

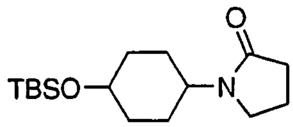

Dissolve *cis*-1-(4-hydroxy-cyclohexyl)-pyrrolidin-2-one (Preparation 3) (40 g; 220 mmol) in dry dichloromethane (1 L). Add imidazole (22.5 g; 330 mmol) followed by *tert*-butyldimethylsilyl chloride (50 g; 330 mmol). Stir under nitrogen at room temperature overnight. Wash with water (250 mL) and saturated aqueous sodium hydrogencarbonate (250 mL). Dry over magnesium sulfate, filter and evaporate to an oil. Pass through a silica gel pad with iso-hexane/ethyl acetate (0-50%) to afford 51 g (79%) the title compound as a clear, pale-yellow oil. LC-MS (M+1=298.5).

Preparation 5

1-Cyclohexyl-piperidin-2-one

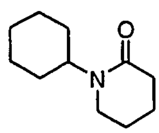

5-Chloro-pentanoic acid cyclohexylamide: Dissolve cyclohexylamine (8.86 mL, 77.5 mmol) in dichloromethane (700 mL), cool to 0°C and add 5-chloropentanoyl chloride (10 mL, 77.5 mmol). After 6 h, wash the mixture with 1N HCl and saturated aqueous sodium bicarbonate and brine, dry the organic layer over sodium sulfate and evaporate to a white solid. Take up the residue in THF (700 mL), add sodium hydride (31 g, 60 % dispersion on mineral oil, 775 mmol) and heat at 70°C 19 h. Cool the mixture, filter through a fritted funnel, wash the filtrate with brine, dry the organic layer over sodium sulfate and evaporate. Purify the residue by column chromatography on silica gel (eluting 50 % to 100 % ethyl acetate) to obtain the desired product as a beige solid (8.8 g, 63 %). MS(EI) *m*/*z*=181.

Preparation 6

1-Chloro-6-methoxynaphthalen-2-yl trifluoromethanesulfonate

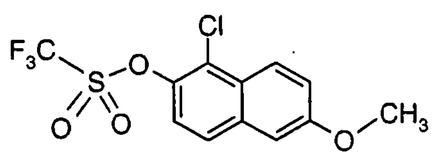

Add triethylamine (30 mL) into a solution of 1-chloro-6-methoxynaphthalen-2-ol *(*Tetrahedron 1982, 2347) (15 g, 72 mmol), N-phenyl-bis(trifluoromethanesulfonimide) (31 g, 86 mmol) and THF (200 mL). Stir for 1 hour. TLC indicated the reaction is completed. Evaporate solvent and purify the residue by column chromatography to afford the title compound as a white solid (24 g, 98%).

Preparation 7

1-Chloro-6-methoxy-2-vinylnaphthalene

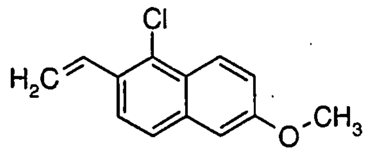

Using the procedure to synthesize Preparation 13 and using reagent 1-chloro-6-methoxynaphthalen-2-yl trifluoromethanesulfonate (12 g, 35 mmol) affords the title compound (5.0 g, 65%) as a white solid.

Preparation 8

2-(Bromomethyl)-1-chloro-6-methoxynaphthalene

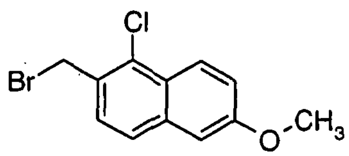

Add sodium periodate (13.5 g, 63 mmol) into a solution of 1-chloro-6-methoxy-2-vinylnaphthalene (4.61 g, 21 mmol), 2.5 wt% OsO4 (4 mL, 0.39 mmol) in THF (300 mL) and water (100 mL) and stir for 2 hours. Add 2.5 wt% $OsO_4$ (2 mL, 0.20 mmol). Extract the reaction mixture with ethyl acetate and wash the organic layer with sodium thiosulfate solution and brine. Dry over sodium sulfate, filter and concentrate. Purify the residue with silica gel column to afford 1-chloro-6-methoxy-2-naphthaldehyde as a white solid (3.3 g, 71 %).

Add $NaBH_4$ (1.0 g, 30 mmol) portionwise into a solution of 1-chloro-6-methoxy-2-naphthaldehyde (3.3 g, 15 mmol) in MeOH (20 mL). Stir for 5 minutes. Pour the reaction mixture into ice and water. Extract the reaction mixture with ethyl acetate, wash with brine, dry with sodium sulfate, filter and concentrated. Purify the residue by column chromatography to afford (1-Chloro-6-methoxynaphthalen-2-yl)methanol (2.4g, 72%) as a white solid.

Add $PBr_3$ (1.0 mL, 11 mmol) to a solution of (1-Chloro-6-methoxynaphthalen-2-yl)methanol (2.4g, 11 mmol) in $CH_2Cl_2$ (20 mL). Pour the reaction mixture into ice and water. Extract the mixture with $CH_2Cl_2$, wash with $NaHCO_3$, brine, dry over sodium sulfate, filtered and concentrated. Purify the residue by silica gel chromatography to afford the title compound as a white solid (2g, 65%).

Preparation 9

*Trans*-1-(4-[*tert*-butyl-dimethyl-silanyloxy)-cyclohexyl]-piperidin-2-one

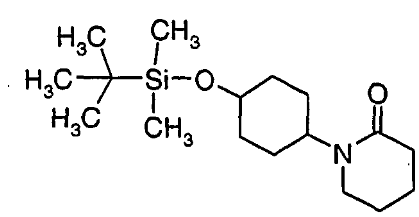

Trans-(4-hydroxy-cyclohexyl)-carbamic acid benzyl ester Combine *trans*-cyclohexylamine hydrochloride (14.0 g, 92.3 mmol), sodium carbonate (19.6 g, 0.185 mol), DCM (50 mL), water (50 mL) and stir for 5 minutes at room temperature. Add benzoyl chloroformate (15.6 mL, 111 mmol) dropwise to the reaction mixture and stir at room temperature for 2 hours. Separate the organic layer, wash with water (3 x 50 mL) and dry over anhydrous $Na_2SO_4$. Evaporate the solvent to obtain the desired intermediate as a white solid (22.7 g, 99 %).

*Trans*-[4-(*tert*-butyl-dimethyl-silanyloxy)-cyclohexy]-carbamic acid benzyl ester: Combine *trans*-(4-hydroxy-cyclohexyl)-carbamic acid benzyl ester (16.0 g, 0.064 mol), imidazole (13.9 g, 0.10 mol), and anhydrous THF (300 mL), add *tert*-butyldimethylsilyl chloride (14.5 g, 0.10 mol) and stir at room temperature for 18 hours. Wash the reaction mixture with water (250 mL), saturated aqueous $NaHCO_3$ (250 mL) and dry the organic layer over anhydrous $Na_2SO_4$. Remove the solvent and purify the residue by chromatography over silica gel (eluting with 0 to 30 % EtOAc in hexane) to obtain the desired intermediate as clear oil (23.0 g, 98 %).

*Trans*-4-(*tert*-butyl-dimethyl-silanyloxy)-cyclohexylamine: Combine *trans*-[4-(*tert*-butyl-dimethyl-silanyloxy)-cyclohexyl]-carbamic acid benzyl ester (23.0 g, 0.06 mol), palladium, 10% wt. on activated carbon (0.5 g), in EtOAc (100 mL) and charge the flask with hydrogen (50 psi). After 3 hours, filter the reaction mixture through a pad of Celite® and evaporate the solvent to obtain the desired intermediate as a dark oil (14.4 g, 99 %): MS(EI) *m/z* = 229 (M+).

*Trans*-5-chloro-pentanoic acid-(4-*tert*-butyl- dimethyl- silanyloxy)-cyclohexyl]-amide: Combine 5-chlorovaleric acid (19.7 g, 0.16 mol), thionyl chloride (20 mL) and reflux for 3 hours. Remove unreacted thionyl chloride by evaporation with toluene (3 x 10 mL) to obtain 5-chloro-pentanoyl chloride as a clear oil (24.1 g, 97 %). Combine *trans*-4-(*tert*-butyl-dimethyl-silanyloxy)-cyclohexylamine (17.7 g, 0.08 mol), and anhydrous pyridine (10.9 mL, 0.23 mol) in anhydrous DCM (100 mL) and cool to 0°C. Add 5-chloro-pentanoyl chloride (14.2 g, 0.09 mol) dropwise to the reaction mixture and stir at room temperature for 1 hour. Partition the reaction mixture between brine and EtOAc. Dry the organic layer over $Na_2SO_4$, evaporate the solvent and purify the residue by chromatography over silica gel (eluting with 0 to 30 % EtOAc in hexane) to obtain the desired intermediate as a colorless oil (22.7 g, 85 %): MS (ES+) *m/z* = 349 $(M+H)^+$.

*Trans*-1-(4-[*tert*-butyl- dimethyl- silanyloxy)-cyclohexyl]-piperidin- 2- one: Dissolve trans-5-chloro-pentanoic acid-[4-(*tert*-butyl-dimethyl-silanyloxy)-cyclohexyl]-amide (22.7 g, 65.1 mmol) in anhydrous THF (500 mL), add sodium hydride (60 % dispersion in mineral oil, 13.0 g, 0.32 mol) by portions and heat the reaction mixture at 70°C for 18 hours. Cool the reaction mixture to room temperature, quench with water (200 mL) and extract with DCM (3 x 100 mL). Dry the organic layer over anhydrous $Na_2SO_4$, remove the solvent and purify the residue by chromatography over silica gel (eluting with 0 to 50 % EtOAc in hexane) to obtain the title compound as a white solid (15.0 g, 74 %): MS (ES+) *m/z* = 312 $(M+H)^+$.

Preparation 10

*Cis*-1-[4-(*tert*-butyl-dimethyl-silaniloxy)-cyclohexyl]-piperidin-2-one

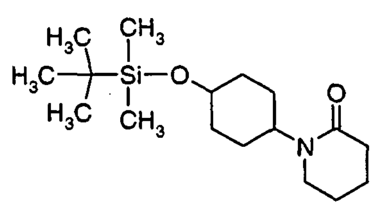

*Trans*-1-(4-hydroxy-cyclohexyl)-piperidin-2-one: Dissolve *trans*-1-(4-[*tert*-butyl-dimethyl-silanyloxy)-cyclohexyl]-piperidin-2-one (10 g, 21.1 mmol) in ethanol containing concentrated hydrochloric acid (5% v/v, 30 mL) and stir at room temperature for 18 hours. Evaporate the solvent, take the residue up in DCM (300 mL) and wash with saturated aqueous $NaHCO_3$ (100 mL). Dry the organic layer over anhydrous $Na_2SO_4$ and remove the solvent to obtain the desired intermediate as a clear oil (4.0 g, 95 %): MS (ES+) *m/z* = 198 $(M+H)^+$.

*Cis*-4-nitro-benzoic acid-4-(2-oxo-piperidin-1-ly)-cyclohexyl-ester: Dissolve *trans*-1-(4-hydroxy-cyclohexyl)-piperidine-2-one (4.0 g, 20.0 mol) in THF (250 mL), cool to -5°C and add triphenyl phosphine (12.0 g, 0.05 mol) and benzoic acid (8.4 g, 0.05 mol). Add diisopropylazodicarboxylate (10.1 g, 0.05 mol) dropwise to the reaction mixture, warm to room temperature and stir for 18 hours. Quench the reaction mixture with saturated aqueous $NaHCO_3$ and extract with DCM (3 x 100 mL). Dry the organic layer over anhydrous $Na_2SO_4$, remove the solvent and purify the residue by chromatography over silica gel eluting with EtOAc to obtain the desired intermediate (5.0 g, 72 %): MS (ES+) *m/z* = 347 $(M+H)^+$.

*Cis*-1-[4-(*tert*-butyl-dimethyl-silaniloxy)-cyclohexyl]-piperidin-2-one: Dissolve *cis*-4-nitro-benzoic acid-4-(2-oxo-piperidin-1-yl)-cyclohexyl-ester (5.0 g, 14.4 mmol) in methanol (150 mL), add water (20 mL), $K_2CO_3$ (8.7 g, 0.06 mol) and stir the reaction mixture at room temperature for 18 hours. Extract the reaction mixture with DCM (2 x 100 mL), dry the organic layer over $Na_2SO_4$ and remove the solvent to obtain *cis*-1-(4-hydroxy-cyclohexyl)-piperidin-2-one as clear oil (6.0 g). Combine *cis*-1-(4-hydroxycyclohexyl)-piperidin-2-one (6.0 g, 0.03 mol), imidazole (3.1 g, 0.05 mol), *tert*-butyl chloro dimethyl silane (6.9 g, 0.05 mol) and stir at room temperature for 18 hours. Wash the reaction mixture with water (150 mL) and dry the organic layer over $Na_2SO_4$. Remove the solvent and purify the residue by chromatography over silica gel (eluting with 0 to 50 % EtOAc in hexane) to obtain the title compound as a clear oil (3.4 g, 76 %): MS (ES+) *m/z* = 312 $(M+H)^+$.

Preparation 11

4-Benzyl-3-[2-(4-bromo-naphthalen-1-ylmethyl)-pent-4-enoyl]-oxazolidin-2-one

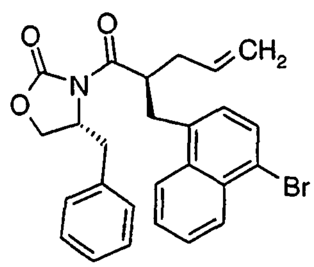

Using the procedure as described in Example 65 and using the reagents (R)-4-benzyl-3-pent-4-enoyl-oxazolidin-2-one (1.088 g, 4.2 mmol) and 1-bromo-4-bromomethyl-naphthalene (1.89 g, 6.3 mmol) yields 1.02 g of 4-benzyl-3-[2-(4-bromo-naphthalen-1-ylmethyl)-pent-4-enoyl]-oxazolidin-2-one (4.2 mmol) in 51% yield (1.02g). Mass spectrum (apci) m/z = 287 (M+H).

Preparation 12

4-(4-Benzyl-2-oxo-oxazolidin-3-yl)-3-(4-bromo-naphthalen-1-ylmethyl)-4-oxo-butyraldehyde

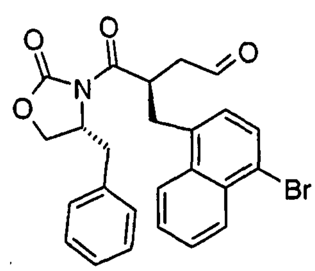

Mix 4-benzyl-3-[2-(4-bromo-naphthalen-1-ylmethyl)-pent-4-enoyl]-oxazolidin-2-one (1.02 g, 2.13 mmol), $OsO_4$ (2.17 g, 0.21mmol), $NaIO_4$ (1.37 g, 6.40 mmol) and 15 mL of water in 50 mL of THF. Stir vigorously for 12 hours at room temperature. Quench with water and extract with ethyl acetate. Wash the extract with $Na_2S_2O_3$, $NaHCO_3$, and brine. Dry over magnesium sulfate, filter, and concentrate. After flash column chromatography receive 0.30 g (30%) of the title compound: Mass spectrum (apci) m/z=481 (M+H).

Preparation 13

1-Cyclohexyl-3-((7-vinylnaphthalen-2-yl)methyl)pyrrolidin-2-one

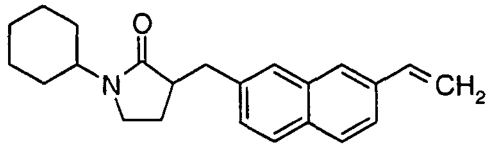

Heat a solution of 7-((1-cyclohexyl-2-oxopyrrolidin-3-yl)methyl)naphthalen-2-yl trifluoromethanesulfonate (500 mg, 1.1 mmol), potassium vinyltrifluoroborate (500 mg, 3.8 mmol), Pd(dppf)$Cl_2$.$CH_2Cl_2$ (20 mg, 0.02 mmol), triethylamine (1.0 mL, 7 mmol) and n-propanol (5 mL) to reflux for 3 hours. Pour the reaction mixture in $H_2O$ and extract with ethyl acetate. Wash the organic layer with brine and separate the organic layer. Dry over sodium sulfate, filter and concentrate. Purify the residue by column chromatography to afford the title compound as a white solid (350 mg, 79%): MS (APCI-pos mode) m/z (rel intensity): 334.2 (M+H, 100%).

Preparation 14

1-Cyclohexyl-3-[6-(2-dimethylamino-ethoxy)-naphthalen-2-ylmethyl]-pynolidin-2-one hydrochloride salt.

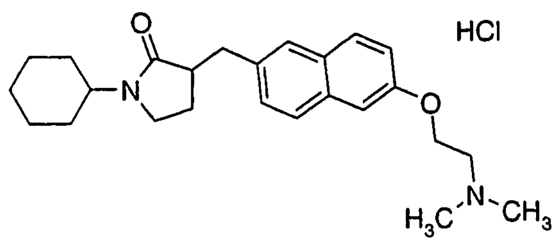

Charge a vial with 1-cyclohexyl-3-(6-hydroxy-naphthalen-2-ylmethyl)-pyrrolidin-2-one (Example 5) (150 mg, 0.46 mmol) and dissolve in acetone (0.3 M). Add $K_2CO_3$ (250 mg, 1.8 mmol) and $Cs_2CO_3$ (59 mg, 0.18 mmol) and stir at room temperature for 5 minutes. Add (2-chloro-ethyl)-dimethyl-amine hydrochloride (150 mg, 1.1 mmol) and heat to 50˚C overnight. Cool to room temperature, filter, concentrate and purify over silica gel. Dissolve the residue in methylene chloride (0.1 M) and add 2 M HCl in ether (0.18 mL, 0.36 mmol) and concentrate in vacuo to afford 144 mg (84%) of the title compound: Mass spectrum (apci) m/z=395.3 (M+H-HCl).

Preparation 15

4-[6-(1-Cyclohexyl-2-oxo-pyrrolidin-3-ylmethyl)-naphthalen-2-yloxy]-butyric acid

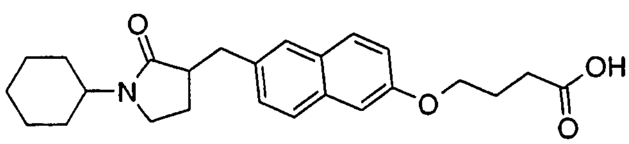

Dissolve 4-[6-(1-cyclohexyl-2-oxo-pyrrolidin-3-ylmethyl)-naphthalen-2-yloxy]-butyric acid methyl ester (196 mg, 0.46 mmol) in ethanol (10 mL) and add potassium hydroxide (260 mg, 4.6 mmol) and stir at 50 ˚C overnight. Filter the resultant precipitate and wash with ethanol. Add solid to water and acidify to pH 1. Extract with methylene chloride,

dry over sodium sulfate and concentrate in vacuo to yield the title compound as a white solid (163 mg). Mass spectrum (apci) m/z=410.3 (M+H).

Preparation 16

1-Cyclohexyl-3-(4-hydroxy-naphthalen-1-ylmethyl)-pyrrolidin-2-one

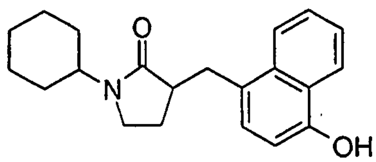

Add $BBr_3$ (0.6 mL, 7.1 mmol) into a solution of 1-cyclohexyl-3-(4-methoxy-naphthalen-1-ylmethyl)-pyrrolidin-2-one (1.2 g, 3.6 mmol) in $CH_2Cl_2$ (10 mL) at 0˚C. Stir at room temperature for 1 hour. Pour the reaction mixture into ice and extract with $CH_2Cl_2$. Separate the organic layer and dry with $Na_2SO_4$. Filter and concentrate the organic layer. Purify the residue by column chromatography to afford the title compound as a white solid (800 mg, 70%): MS (APCI-pos mode) m/z (rel intensity): 324.2 (M+H, 100%).

Example 1

1-Cyclohexyl-3-naphthalen-2-ylmethyl-pyrrolidin-2-one

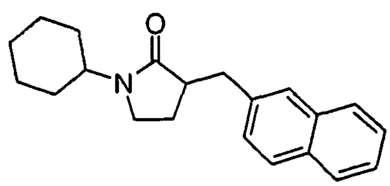

Place 1-cyclohexyl-pyrrolidin-2-one (0.50 g, 3.0 mmol) in THF (30 mL) and cool to -78˚C. Slowly add LDA (2.0 M, 2.4 mL, 4.8 mmol) and stir for 15 minutes. Add 2-bromomethyl-naphthalene (2 g) and stir for 3 hours. Quench with ammonium chloride and extract with dichloromethane. Dry over sodium sulfate, filter, and concentrate. Purify by silica gel (20-50% ethyl acetate in hexanes) to afford 839 mg of the title compound: MS (APCI-pos mode) *m*/*z* (rel intensity) 308 (100).

Table 1

| The following examples are prepared essentially as described in Example 1 except using the reagents in the "Reagents used" column. | | | |
|---|---|---|---|
| Example | Structure and Name | Reagents used | Mass Spec |
| 2 | 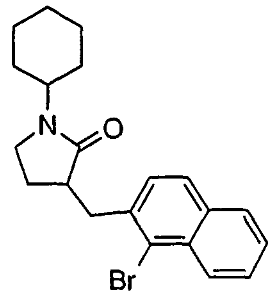 3-(1-Bromo-naphthalen-2-ylmethyl)-1-cyclohexyl-pyrrolidin-2-one | 1-cyclohexyl pyrrolidinone (140 mg, 0.84 mmol) and 1-bromo-2-bromomethyl-naphthalene (300 mg, 1.0 mmol) | (APCI-pos mode) m/z (rel intensity) 386 (100), 388 (100) |
| 3 | 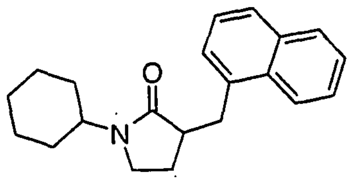 1-Cyclohexyl-3-naphthalen-1-ylmethyl-pyrrolidin-2-one | 1-cyclohexyl pyrrolidinone (850 mg, 5.08 mmol) and 1-bromomethyl-naphthalene (1.35 g, 6.10 mmol) | (APCI-pos mode) *m/z* (rel intensity) 308 (100) |
| 4 | 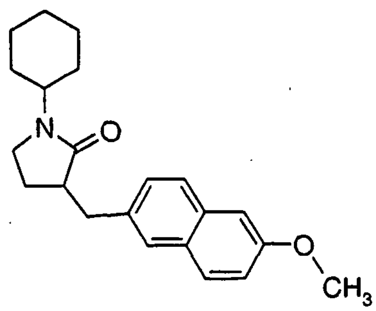 1-Cyclohexyl-3-(6-methoxy-naphthalen-2-ylmethyl)-pyrrolidin-2-one | 1-cyclohexyl pyrrolidinone (2.75 g, 16.4 mmol) and 2-bromomethyl-6-methoxy-naphthalene [J. Org. Chem. (2002) 6216-6219] (4.95 g, 19.7 mmol) | (APCI-pos mode) *m/z* (rel intensity) 338 (100) |
| 5 | 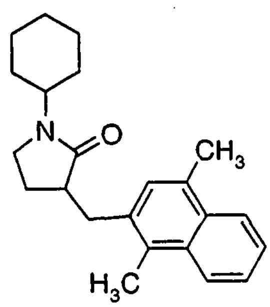 1-Cyclohexyl-3-(1,4-dimethyl-naphthalen-2-ylmethyl)-pyrrolidin-2-one | 1-cyclohexyl pyrrolidinone (0.10 g, 0.60 mmol) and 2-chloromethyl-1,4-dimethyl-naphthalene [Chem. Ber. (1958) 1354-1356] (0.15 g, 0.72 mmol) | (APCI-pos mode) *m/z* (rel intensity) 336 (100) |

(continued)

| The following examples are prepared essentially as described in Example 1 except using the reagents in the "Reagents used" column. | | | |
|---|---|---|---|
| Example | Structure and Name | Reagents used | Mass Spec |
| 6 | 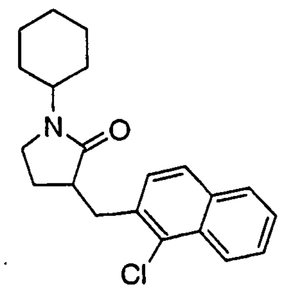 3-(1-Chloro-naphthalen-2-ylmethyl)-1-cyclohexyl-pyrrolidin-2-one | 1-cyclohexyl pyrrolidinone (1.87 g, 11.2 mmol) and 2-bromomethyl-1-chloro-naphthalene [J. Label. Compounds and Radiopharm. (1987) 851-858] (1.90 g, 7.44 mmol) | (APCI-pos mode) *m/z* (rel intensity) 342 (100), 344 (40) |

Example 7

1-Cyclohexyl-3-(6-hydroxy-naphthalen-2-ylmethyl)-pyrrolidin-2-one

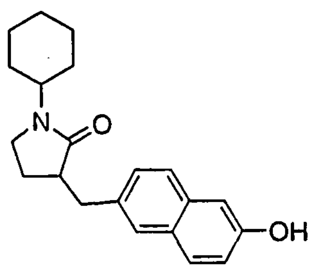

Add boron tribromide (2.20 mL, 23.3 mmol) to a solution of 1-cyclohexyl-3-(6-methoxy-naphthalen-2-ylmethyl)-pyrrolidin-2-one (2.62 g, 7.76 mmol) in dichloromethane (50 mL) at 0°C and stir 40 minutes. Quench with water (50 mL) and stir overnight at room temperature. Wash the organic layer with water, brine, dry, filter. Add hexanes (100 mL) to the filtrate and filter to yield the title compound as an off white powder (2.41 g, 96%): MS (APCI-pos mode) *m/z* (rel intensity) 324 (100).

Example 8

1-(*cis*-4-Hydroxy-cyclohexyl)-3-(6-methoxy-naphthalen-2-ylmethyl)-pyrrolidin-2-one

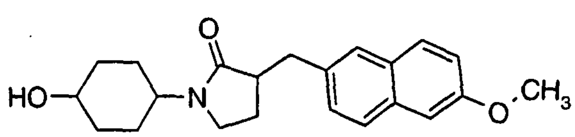

Using the procedure to synthesize Example 5 and using reagents *cis*-1-[4-(*tert*-butyl-dimethyl-silanyloxy)-cyclohexyl]-pyrrolidin-2-one (1.30 g, 4.37 mmol) and 2-bromomethyl-6-methoxy-naphthalene (1.30 g, 5.18 mmol) affords 1.05 g of the title compound: Mass spectrum (apci) m/z=354.1 (M+H).

Example 9

1-(*cis*)-(4-Hydroxy-cyclohexyl)-3-naphthalen-2-ylmethyl-pyrrolidin-2-one

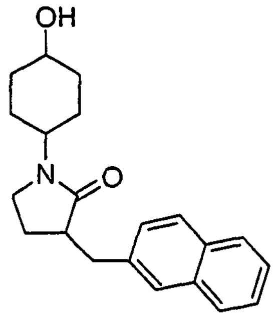

Charge a flask with *cis*-1-[4-(*tert*-butyl-dimethyl-silanyloxy)-cyclohexyl]-pyrrolidin-2-one (0.600 g, 2.02 mmol) (Preparation 4), dissolve with THF (0.2 M) and cool to -78˚C. Add LDA (1.1 to 1.5 eq) and stir at -78˚C for 5 minutes. Add 1-bromomethyl-naphthalene (535 mg, 2.42 mmol) and warm to room temperature overnight. Dilute with methanol (0.2 M) and add concentrated HCl (10 eq.) and stir at room temperature. Pour into water after reaction complete by HPLC and extract with methylene chloride, dry over sodium sulfate, filter and concentrate in vacuo. Purify the residue over silica gel (20% hexane in ethyl acetate) to afford 360 mg (55%) of the title compound: MS (APCI-pos mode) *m*/*z* (rel intensity) 324 (100).

Example 10

1-Cyclohexyl-3-[6-(2-piperidin-1-yl-ethoxy)-naphthalen-2-ylmethyl]-pyrrolidin-2-one hydrochloride salt.

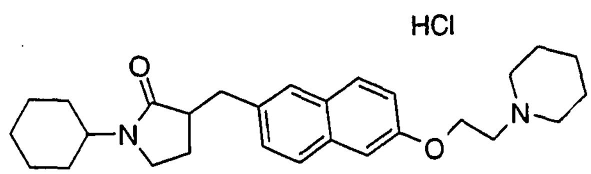

Using the procedure to synthesize Preparation 14 and using reagents 1-cyclohexyl-3-(6-hydroxy-naphthalen-2-ylmethyl)-pyrrolidin-2-one (Example 5) (150 mg, 0.46 mmol) and 1-(2-chloro-ethyl)-piperidine hydrochloride (200 mg, 1.1 mmol) affords 145 mg (66%) of the title compound after HCl salt formation: Mass spectrum (apci) m/z=435.4 (M+H-HCl).

Example 11

1-Cyclohexyl-3-[6-(3-dimethylamino-propoxy)-naphthalen-2-ylmethyl]-pyrrolidin-2-one hydrochloride salt.

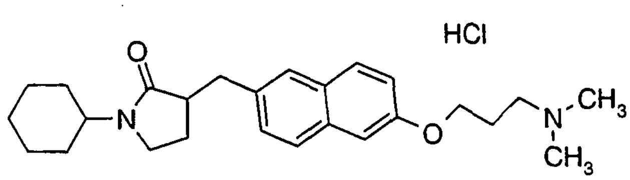

Using the procedure to synthesize Preparation 14 and using reagents 1-cyclohexyl-3-(6-hydroxy-naphthalen-2-ylmethyl)-pyrrolidin-2-one (150 mg, 0.46 mmol) and (2-chloro-ethyl)-dimethyl-amine hydrochloride (170 mg, 1.1 mmol) affords 154 mg (75%) of the title compound after HCl salt formation: Mass spectrum (apci) m/z=409.3 (M+H-HCl).

Example 12

3-[6-(1-Cyclohexyl-2-oxo-pyrrolidin-3-ylmethyl)-naphthalen-2-yloxymethyl]-benzoic acid methyl ester

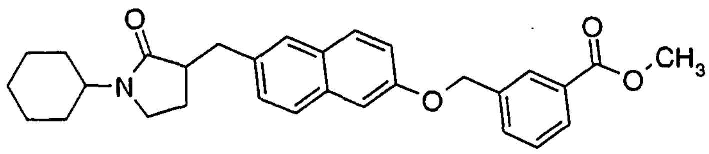

Dissolve 1-cyclohexyl-3-(6-hydroxy-naphthalen-2-ylmethyl)-pyrrolidin-2-one (Example 5) (500 mg, 1.55 mmol) in acetone (10 mL). Add potassium carbonate (1.3 g, 9.3 mmol) and cesium carbonate (250 mg, 0.75 mmol). Add 3-bromomethyl-benzoic acid methyl ester (810 mg, 3.6 mmol) and stir at 50°C for 2 days. Filter the solids and concentrate in vacuo. Purify the residue over silica gel (20 to 40% ethyl acetate in hexanes) to yield the title compound as a white solid (670 mg, 92%). Mass spectrum (apci) m/z=472.2 (M+H).

Example 13

3-[6-(1-Cyclohexyl-2-oxo-pyrrolidin-3-ylmethyl)-naphthalen-2-yloxymethyl]-benzoic acid

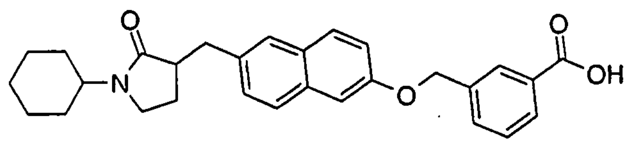

Dissolve 3-[6-(1-cyclohexyl-2-oxo-pyrrolidin-3-ylmethyl)-naphthalen-2-yloxymethyl]-benzoic acid methyl ester (600 mg, 1.27 mmol) in 1:1 ethanol:THF (40 mL). Add potassium hydroxide (710 mg, 13 mmol) and stir at room temperature overnight. Pour the reaction into 1N HCl and extract with methylene chloride. Dry the organic layer with sodium sulfate, filter and concentrate to yield the title compound as a white solid (600 mg, 100%): Mass spectrum (apci) m/z=458.2 (M+H).

Example 14

1-Cyclohexyl-3-{6-[3-(4-methyl-piperazine-1-carbonyl)-benzyloxy]-naphthalen-2-ylmethyl}-pyrrolidin-2-one trifluoroacetate salt

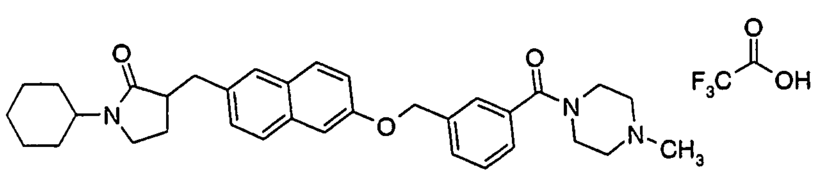

Charge a flask with 3-[6-(1-cyclohexyl-2-oxo-pymolidin-3-ylmethyl)-naphthalen-2-yloxymethyl]-benzoic acid

(300 mg, 0.66 mmol), EDCI (250 mg, 1.3 mmol), HOBt (89 mg, 0.66 mmol), triethylamine (0.36 mL, 2.6 mmol) and N-methylpiperazine (0.22 mL, 2.0 mmol) and dissolve with DMF (6 mL). Add more EDCI (250 mg, 1.3 mmol) after stirring overnight. Stir overnight again and pour into water and extract with ether. Purify with reverse phase HPLC to yield the title compound as a white solid (145 mg, 38%): Mass spectrum (apci) m/z=540.4 (M+H-TFA).

Example 15

3-(1-Chloro-naphthalen-2-ylmethyl)-1-(cis-4-hydroxy-cyclohexyl)-pyrrolidin-2-one

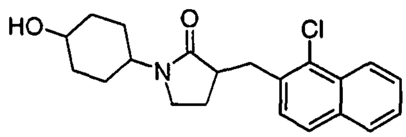

Dissolve *cis*-1-[4-(*tert*-butyl-dimethyl-silanyloxy)-cyclohexyl]-pyrrolidin-2-one (0.5 g, 1.68 mmol) in THF (30 mL) and cool to -78˚C under $N_2$. Stir the solution and add LDA (1.23 mL, 1.85 mmol, 1.5 M in THF). Continue to stir the solution for 0.5 hours at the same temperature and add 2-bromomethyl-1-chloro-naphthalene (0.52 g, 2.02 mmol). Stir the reaction for another two hours and add water (50 mL). Warm the reaction to room temperature and the extract the aquaous layer with $CH_2Cl_2$ (3 x 100 mL). Combine the organic layers and dry with $Na_2SO_4$, filter and concentrate to give the crude TBS ether product. Dissolve the crude product in MeOH (50 mL) and add 1 N HCl (10 mL). Stir the solution for overnight at room temperature and then remove MeOH under reduced pressure. Extract the aqueous with $CH_2Cl_2$ (3 x 50 mL) and dry with $Na_2SO_4$. Filter, concentrate and purify by flash column chromatography (silica gel, 50-80% EtOAc-Hexane) to give the title product as a white solid (432 mg, 72%): Mass spectrum (ion spray): m/z = 358.1, 360.1 (M+1).

Example 16

1-Cyclohexyl-3-(4-methoxy-naphthalen-1-ylmethyl)-pyrrolidin-2-one

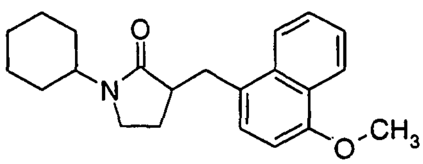

Generate LDA by adding 1.6 M *n*-BuLi (6.25 mL, 10 mmol) into a solution of diisopropylamine (1.4 mL, 10 mmol) in THF (10 mL) at -10˚C. Cool to -78˚C and add 1-cyclohexyl-pyrrolidin-2-one (1.7 g, 10 mmol). Stir for 15 minutes. Add 1-chloromethyl-4-methoxy-naphthalene (John L. Kice and Harvinder Lotey, J. Org. Chem., 1989, 54(15), 3596-602) (1.0 g, 5.0 mmol) in THF (10 mL) and warm up to room temperature. Quench with saturated ammonium chloride solution and extract with ethyl acetate. Dry over sodium sulfate, filter, and concentrate. Purify by silica gel (20-50% ethyl acetate in hexanes) to afford the title compound (1.4 g, 81%): MS (APCI-pos mode) m/z (rel intensity): 338.2 (M+H).

Table 2

The following examples are prepared essentially as described in Example 16 except using the reagents in the "Reagents used" column.

| Example | Structure and name | Reagents used | Mass Spec |
|---|---|---|---|
| 17 | 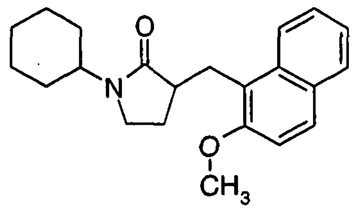 1-Cyclohexyl-3-(2-methoxy-naphthalen-1-ylmethyl)-pyrrolidin-2-one | 1-chloromethyl-2-methoxy-naphthalene (J. Med. Chem., 1996, 39 (16), 3089-3095) (1.05 g, 5 mmol) and 1-cyclohexyl pyrrolidinone (1.7 g, 10 mmol) | (APCI-pos mode) m/z (rel intensity): 338.2 (M+H, 100) |
| 18 | 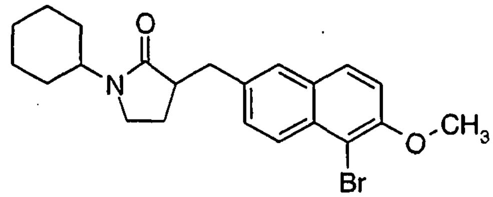 3-(5-bromo-6-methoxy-naphthalen-2-ylmethyl)-1-cyclohexyl-pyrrolidin-2-one | 1-bromo-7-bromomethyl-2-methoxy-naphthalene (3.0 g, 9.0 mmol) and 1-cyclohexyl pyrrolidinone (3.0 g, 18 mmol) | (APCI-pos mode) m/z (rel intensity): =416.2 (M+H, 100%), 418.2 (100%) |
| 19 | 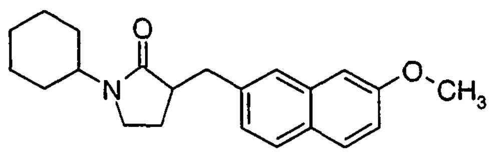 1-Cyclohexyl-3-(7-methoxy-naphthalen-2-ylmethyl)-pyrrolidin-2-one | 2-bromomethyl-7-methoxy-naphthalene (J. Am. Chem. Soc., 2000, 122(29), 6935-6949) (10.5 g, 42 mmol) and 1-cyclohexyl pyrrolidinone (14.0 g, 84 mmol) | (APCI-pos mode) m/z (rel intensity): 338.2 (M+H, 100%) |
| 20 | 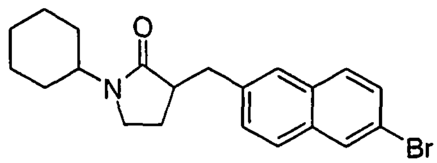 3-(6-Bromo-naphthalen-2-ylmethyl)-1 -cyclohexyl-pyrrolidin-2-one | 2-Bromo-6-bromomethyl-naphthalene (4.5 g, 15 mmol) and 1-cyclohexyl pyrrolidinone (4 g, 24 mmol) | (APCI-pos mode) m/z (rel intensity): 416.2 (M+H, 100%), 418.2 (100%) |
| 21 | 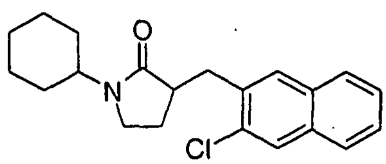 3-((3-Chloronaphthalen-2-yl)methyl)-1-cyclohexylpyrrolidin-2-one | 1-chloro-2-bromomethyl-naphthalene (J. Org. Chem. 1957, 1473) (1.3 g, 5 mmol) and 1-cyclohexyl pyrrolidinone | (APCI-pos mode) m/z (rel intensity): 342.2 (M+H, 100%) |

(continued)

The following examples are prepared essentially as described in Example 16 except using the reagents in the "Reagents used" column.

| Example | Structure and name | Reagents used | Mass Spec |
|---|---|---|---|
| 22 | 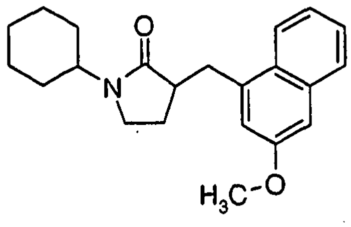 1-Cyclohexyl-3-((3-methoxynaphthalen-1-yl) methyl)pyrrolidin-2-one | 1-bromomethyl-3-methoxy-naphthalene (0.5 g, 1.0 mmol) and 1-cyclohexyl pyrrolidinone | (APCI-pos mode) m/z (rel intensity): 338.2 (M+H, 100%) |
| 23 | 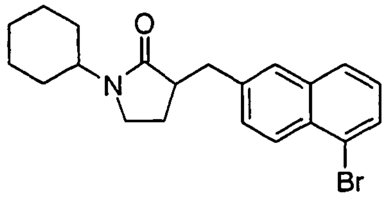 3-((5-Bromonaphthalen-2-yl)methyl)-1-cyclohexylpyrrolidin-2-one | 1-bromo-6-bromomethyl-naphthalene (7 g, 23 mmol) and 1-cyclohexyl pyrrolidinone | (APCI-pos mode) m/z (rel intensity): 386.2. (M+H, 100%) |
| 24 | 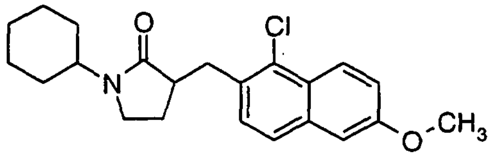 3-((1-Chloro-6-methoxynaphthalen-2-yl)methyl)-1-cyclohexylpyrrolidin-2-one | 2-(bromomethyl)-1-chloro-6-methoxynaphthalene (2 g, 7 mmol) and 1-cyclohexyl pyrrolidinone | (APCI-pos mode) m/z (rel intensity): 372.2 (M+H, 100%) |

Table 3

The following examples are prepared essentially as described in Preparation 16 except using the reagents in the "Reagents used" column.

| Example | Structure and name | Reagents used | Mass Spec |
|---|---|---|---|
| 25 | 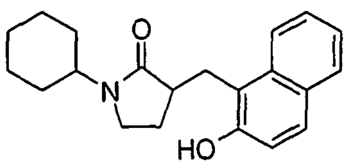 1-Cyclohexyl-3-(2-hydroxy-naphthalen-1-ylmethyl)-pyrrolidin-2-one | 1-cyclohexyl-3-(2-methoxy-naphthalen-1-ylmethyl)-pyrrolidin-2-one (1.3g, 3.9mmol) | (APCI-pos mode) m/z (rel intensity): 324.2 (M+H, 38%), 220.3 (M-103, 100%) |
| 26 | 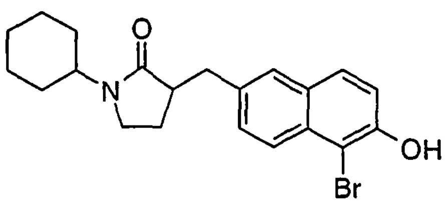 3-(5-Bromo-6-hydroxy-naphthalen-2-ylmethyl)-1-cyclohexyl-pyrrolidin-2-one | 3-(5-Bromo-6-methoxy-naphthalen-2-ylmethyl)-1-cyclohexyl-pyrrolidin-2-one (0.7 g, 1.7 mmol) | (APCI-pos mode) m/z (rel intensity): 402.1 (M+H, 30%) |

(continued)

| The following examples are prepared essentially as described in Preparation 16 except using the reagents in the "Reagents used" column. | | | |
|---|---|---|---|
| Example | Structure and name | Reagents used | Mass Spec |
| 27 | 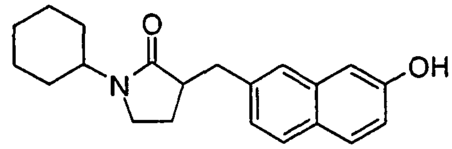 1-Cyclohexyl-3-(7-hydroxy-naphthalen-2-ylmethyl)-pyrrolidin-2-one | 1-cyclohexyl-3-(7-methoxy-naphthalen-2-ylmethyl)-pyrrolidin-2-one (5.8 g, 17 mmol) | (APCI-pos mode) m/z (rel intensity): 324.2 (M+H, 100%) |

Example 28

1-Cyclohexyl-3-(6-isopropoxy-naphthalen-2-ylmethyl)-pyrrolidin-2-one

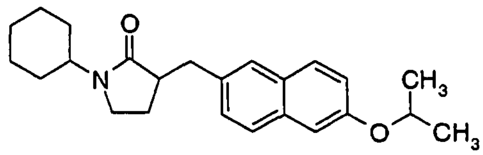

Add $Cs_2CO_3$ (0.7 g, 2.1 mmol) into a solution of 1-cyclohexyl-3-(6-hydroxy-naphthalen-2-ylmethyl)-pyrrolidin-2-one (300 mg, 0.93 mmol) and 2-idopropane (1 mL, 6 mmol) in DMF (10 mL). Stir for 12 hours. Pour the reaction mixture into water and extract with ethyl acetate. Dry, filtered and concentrate to afford the crude mixture. Purify the residue by column chromatography affords the title compound as a pale yellow solid (300 mg, 88%): MS (APCI-pos mode) m/z (rel intensity): 366.3 (M+H, 100%).

Example 29

1-Cyclohexyl-3-((7-isopropoxynaphthalen-2-yl)methyl)pyrrolidin-2-one

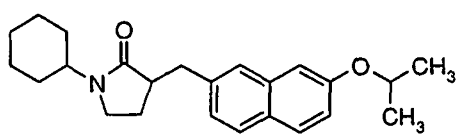

Using the procedure to synthesize Example 28 and using reagent 1-cyclohexyl-3-(7-hydroxy-naphthalen-2-ylmethyl)-pyrrolidin-2-one (300 mg, 0.93 mmol) affords the title compound (100 mg, 30%) as a white solid: MS (APCI-pos mode) m/z (rel intensity): 366.3. (M+H, 100%).

Example 30

1-Cyclohexyl-3-naphthalen-2-ylmethyl-piperidin-2-one

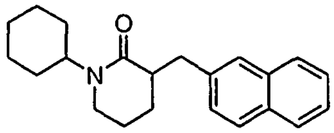

Dissolve 1-cyclohexyl-piperidin-2-one (0.135 g, 0.75 mmol) in dry THF (11 mL) cool to -78˚C and add *t*-butyl lithium (0.66 mL of 1.7 M solution in pentane, 1.12 mmol). After 15 min add 2-bromomethylnaphthalene (0.33 g, 1.5 mmol), and allow the mixture to warm to room temperature over 5 h. Wash the mixture with saturated ammonium chloride and brine, dry the organic layer over sodium sulfate and evaporate. Purify the residue by chromatography on silica gel (eluting 0 % to 50 % ethyl acetate in hexane) and then prep HPLC (10 % to 90 % acetonitrile in 0.1 % aqueous trifluoroacetic acid over 30 min, Sorbax SB-Phenyl 21.2 mm x 25 cm, 22 mL/min). The residue is partitioned between dichloromethane and saturated aqueous sodium bicarbonate and brine, the organic layer was dried over sodium sulfate and the evaporated to give the desired product as a white solid (60 mg, 25 %). MS(ES+) *m*/*z*=322.

Example 31

1-Cyclohexyl-3-methyl-3-naphthalen-1-ylmethyl-pyrrolidin-2-one

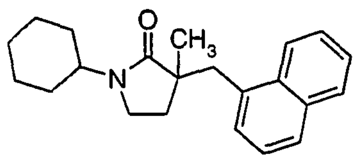

Dissolve 1-cyclohexyl-3-naphthalen-1-ylmethyl-pyrrolidin-2-one (200 mg, 0.65 mmol), in THF (6.5 mL), cool to -78˚C and add lithium diisopropylamide (0.87 mL of 1.5 M solution in cyclohexane, 1.3 mmol). After 9 minutes, add iodomethane (0.12 mL, 1.95 mmol). After 3 h, wash the mixture with ammonium chloride and brine, dry the organic layer over sodium sulfate and evaporate. Purify the residue by chromatography on silica gel (eluting 20 % to 60 % ethyl acetate in hexane) and then by prep HPLC (10 % to 99 % acetonitrile in 0.1 % aqueous trifluoroacetic acid over 30 min, Sorbax SB-Phenyl 21.2 mm x 25 cm, 22 mL/min) to obtain the desired product (126 mg, 60 %). MS(EI) *m*/*z*=321.

Example 32

*Trans*-3-(3-chloro-naphthalene-2-ylmethyl)-1-(4-hydroxy-cyclohexyl)-piperidine-2-one

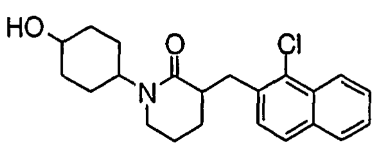

Dissolve *trans*-1-(4-[*tert*-butyl-dimethyl-silanyloxy)-cyclohexyl]-piperidin-2-one (0.3 g, 0.96 mmol) in anhydrous THF (6 mL) under nitrogen, cool to -78˚C and add LDA (2M solution in THF) (0.72 mL, 1.44 mmol) dropwise. Stir the reaction mixture for 5 minutes and add 2-bromomethyl-1-chloro-naphthylene (0.37 g, 1.44 mmol) in anhydrous THF (2 mL) dropwise. Stir the reaction mixture at -78˚C for 3 hours, slowly warm to room temperature, quench with saturated aqueous $NH_4Cl$ (5 mL) and extract with DCM (3 x 10 mL). Dry the organic layer over $Na_2SO_4$, remove the solvent and purify the residue by chromatography over silica gel (eluting with 0 to 20 % EtOAc in hexane) to obtain trans-1-[4-(*tert*-butyl-dimethyl-silanyloxy)-cyclohexyl]-3-(3-chloro-naphthalen-2-ylmethyl)-piperidin-2-one as a clear oil (0.2 g). Dissolve the *trans*-1-[4-(*tert*-butyl-dimethyl-silanyloxy)-cyclohexyl]-3-(3-chloro-naphthalen-2-ylmethyl)-piperidin-2-one

in ethanol containing concentrated hydrochloric acid (5% v/v) and heat at 40 ˚C for 1 hour. Evaporate the solvent, dissolve the residue in DCM (10 mL) and wash with saturated aqueous $NaHCO_3$ (5 mL). Dry the organic layer over anhydrous $Na_2SO_4$, remove the solvent and purify the residue by chromatography over silica gel (eluting with 0 to 50 % EtOAc in hexane) to obtain the title compound as a clear oil (120 mg, 33 %): MS (ES+) *m/z* = 372 $(M+H)^+$.

Examples 33-38 may be prepared essentially as described in Example 32, using appropriately cis or *trans*-1-(4-[*tert*-butyl-dimethyl-silanyloxy)-cyclohexyl]-piperidin-2-one (1 equivalent) and the appropriate bromide or chloride (1 to 1.5 equivalents).

Table 4

The following examples are prepared essentially as described in Example 30 except using the reagents in the "Reagents used" column.

| Example | Structure and name | Reagents used | Mass Spec (ES+) *m/z* |
|---|---|---|---|
| 33 | 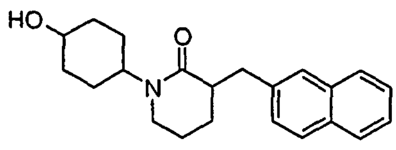 *Trans*-1-(4-hydroxy-cyclohexyl)-3-naphthalene-2-ylmethyl-piperidine-2-one | 2-bromomethyl-naphthylene | 338 $(M+H)^+$ |
| 34 | 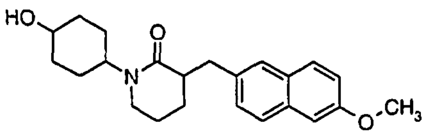 *Trans*-1-(4-hydroxy-cyclohexyl)-3-(6-methoxy-naphthalen-2-ylmethyl) piperidine-2-one | 2-bromomethyl-6-methoxy-naphthalene | 368 $(M+H)^+$ |
| 35 | 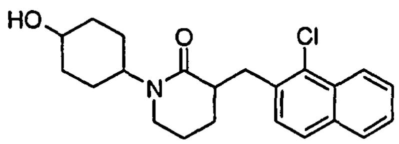 *Cis*-3-(1-chloro-naphthalen-2-ylmethyl)-1-(4-hydroxy-cyclohexyl)-piperidine-2-one | 2-bromomethyl-1-chloro-naphthylene | 372 $(M+H)^+$ |
| 36 | 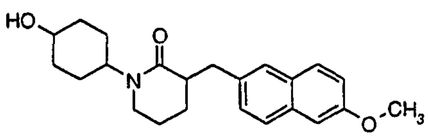 Cis-1-(4-hydroxy-cyclohexyl)-3-(6-methoxy-naphthalen-2-ylmethyl)-piperidine-2-one | 2-bromomethyl-6-methoxy-naphthalene | 368 $(M+H)^+$ |
| 37 | 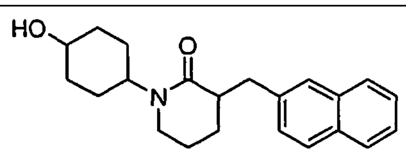 *Cis*-1-(4-hydroxy-cyclohexyl)-3-naphthalen-2-ylmethyl-piperidine-2-one | 2-bromomethyl-naphthylene | 338 $(M+H)^+$ |

(continued)

| The following examples are prepared essentially as described in Example 30 except using the reagents in the "Reagents used" column. | | | |
|---|---|---|---|
| Example | Structure and name | Reagents used | Mass Spec (ES+) *m/z* |
| 38 | 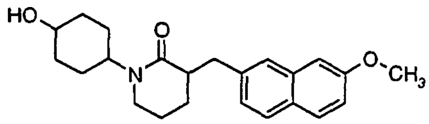 Cis-1-(4-hydroxy-cyclohexyl)-3-(7-methoxy-naphthalen-2-ylmethyl)-piperidine-2-one | 2-bromomethyl-7-methoxy-naphthalene | 368 $(M+H)^+$ |

Example 39

*Cis*-1-(4-hydroxy-cyclohexyl)-3-(3-methoxy-naphthalen-1-ylmethyl)-piperidin-2-one

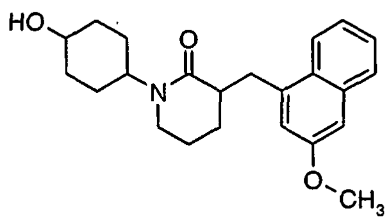

Dissolve *cis*-1-(4-[tert-butyl-dimethyl-silanyloxy)-cyclohexyl]-piperidin-2-one (0.3 g, 0.96 mmol) in anhydrous THF (6 mL) under nitrogen, cool to -78˚C and add LDA (2M solution in THF) (0.72 mL, 1.44 mmol) dropwise. Stir the reaction mixture for 5 minutes and add 1-bromomethyl-3-methoxy-naphthalene (0.24 g, 0.96 mmol) in anhydrous THF (2 mL) dropwise. Allow the mixture to warm over 16 h, quench with saturated aqueous $NH_4Cl$ (5 mL) and extract with DCM (3 x 10 mL). Dry the organic layer over $Na_2SO_4$, remove the solvent and purify the residue by chromatography over silica gel (eluting with 0 to 40 % EtOAc in hexane) to obtain cis-1-[4-(tert-butyl-dimethyl-silanyloxy)-cyclohexyl]-3-(3-methoxy-naphthalen-1-ylmethyl)-pyrrolidin-2-one as a white solid (0.290 g). Dissolve this solid in methanol containing concentrated hydrochloric acid (5% v/v) and heat at 40˚C for 1 hour. Evaporate the solvent, dissolve the residue in DCM (10 mL) and wash with saturated aqueous $NaHCO_3$ (5 mL). Dry the organic layer over anhydrous $Na_2SO_4$, remove the solvent and purify the residue by chromatography over silica gel (eluting with EtOAc) to obtain the title compound as a white solid (142 mg, 40 %): MS (ES+) m/z = 368 (M+H)+.

Example 40

1-Cyclohexyl-3-(2-methoxy-naphthalene-1-ylmethyl)-piperidin-2-one

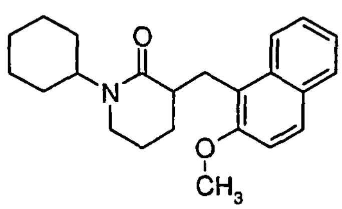

Dissolve 1-cyclohexyl-piperidine-2-one (0.3 g, 1.65 mmol) in anhydrous THF (6 mL) under nitrogen, cool to

-78°C and add LDA (2M solution in THF, 1.24 mL, 2.48 mmol) dropwise. Stir the reaction mixture for 5 minutes and add 1-chloromethyl-2-methoxy-naphthalene (0.3 g, 1.65 mmol) in anhydrous THF (2 mL) dropwise. Stir the reaction mixture at -78°C for 3 hours, slowly warm to room temperature, quench with saturated aqueous $NH_4Cl$ (5 mL) and extract with DCM (3 x 5 mL). Dry the organic layer over $Na_2SO_4$, remove the solvent and purify the residue by chromatography over silica gel (eluting with 0 to 40 % EtOAc in hexane) to obtain the title compound as a white solid (0.2 g, 31%). MS (ES+) *m*/*z* = 352 $(M+H)^+$.

Example 41

1-Cyclohexyl-3-(3-methoxy-naphthalene-1-ylmethyl)-piperidine-2-one

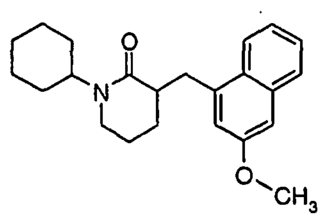

Prepare according to the procedure for Example 40 1-cyclohexyl-piperidine-2-one (0.3 g, 1.65 mmol) and 1-chloromethyl-3-methoxy-naphthalene (0.3 g, 1.65 mmol) to obtain the title compound as a white solid (0.2 g, 31%). MS (ES+) *m*/*z* = 352 $(M+H)^+$.

Example 42

3-((6-(3-Aminophenyl)naphthalen-2-yl)methyl)-1-cyclohexylpyrrolidin-2-one

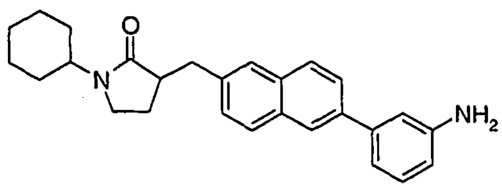

Heat a mixture of 3-(6-bromo-naphthalen-2-ylmethyl)-1-cyclohexyl-pyrrolidin-2-one (200 mg, 0.52 mmol), 3-aminophenylboronic acid monohydrate (200 mg, 1.46 mmol), LiCl (220 mg, 5.2 mmol), $Pd(PPh_3)_4$ (30 mg, 0.03 mmol), 2M $Na_2CO_3$ (2 mL) and dioxane (5 mL) to 80°C for 0.5 hour. Pour the reaction mixture into water and extract with EtOAc. Dry over sodium sulfate, filter and concentrate the organic layer. Purify the residue by column chromatography affords the title compound (165 mg, 76%) as a white solid: MS (APCI-pos mode) m/z (rel intensity): 399.3 (M+H, 100%).

Table 5

| The following examples are prepared essentially as described in Example 42 except using the reagents in the "Reagents used" column. | | | |
|---|---|---|---|
| Example | Structure and name | Reagents used | Mass Spec |
| 43 | 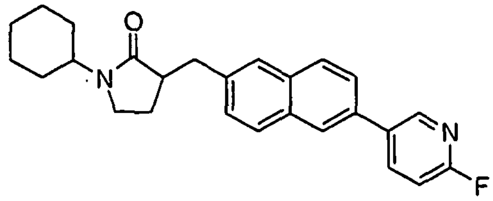 1-Cyclohexyl-3-((6-(6-fluoropyridin-3-yl)naphthalen-2-yl)methyl)pyrrolidin-2-one | 3-(6-bromo-naphthalen-2-ylmethyl)-1-cyclohexyl-pyrrolidin-2-one (250 mg, 0.65 mmol) and 2-fluoropyridine-5-boronic acid (200 mg, 1.3 mmol) | (APCI-pos mode) m/z (rel intensity): 403.2 (M+H, 100%) |
| 44 | 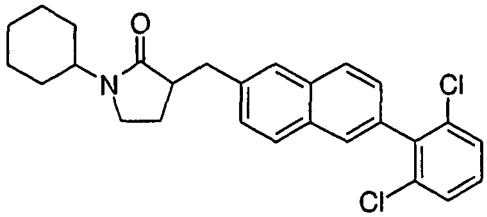 1-Cyclohexyl-3-((6-(2,6-dichlorophenyl)naphthalen-2-yl)methyl)pyrrolidin-2-one | 3-(6-bromo-naphthalen-2-ylmethyl)-1-cyclohexyl-pyrrolidin-2-one (200 mg, 0.65 mmol) and 2.6-dichlorobenzeneboronic acid (200 mg, 1.1 mmol) | (APCI-pos mode) m/z=452.1 (M+H, 100%), 454.2 (M+2+H, 80%) |
| 45 | 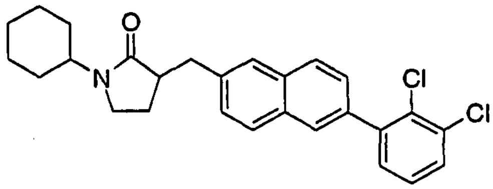 1-Cyclohexyl-3-((6-(2,3-dichlorophenyl)naphthalen-2-yl)methyl)pyrrolidin-2-one | 3-(6-bromo-naphthalen-2-ylmethyl)-1-cyclohexyl-pyrrolidin-2-one (200 mg, 0.52 mmol) and 2,3-dichlorobenzeneboronic acid (200 mg, 1.1 mmol) | (APCI-pos mode) m/z=370.1 (M-81, 40%), 452.2 (M+H, 100%), 454.2 (M+2+H, 60%) |
| 46 | 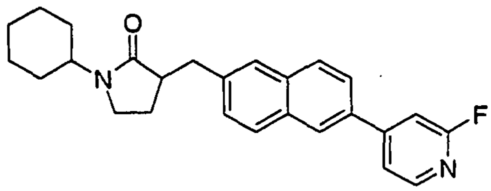 1-Cyclohexyl-3-((6-(2-fluoropyridin-4-yl)naphthalen-2-yl)methyllpyrrolidin-2-one | 3-(6-bromo-naphthalen-2-ylmethyl)-1-cyclohexyl-pyrrolidin-2-one (250 mg, 0.65 mmol) and 2-fluoropyridine-4-boronic acid (200 mg, 1.3 mmol) | (APCI-pos mode) m/z (rel intensity): 403.2 (M+H, 100%) |
| 47 | 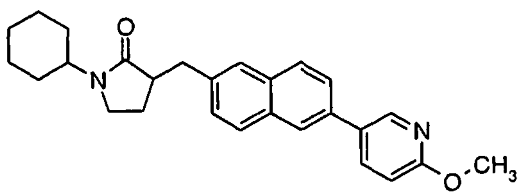 1-Cyclohexyl-3-((6-(6-methoxypyridin-3-yl) naphthalen-2-yl)methyl)pyrrolidin-2-one | 3-(6-bromo-naphthalen-2-ylmethyl)-1-cyclohexyl-pyrrolidin-2-one (250 mg, 0.65 mmol) and 2-methoxyl-5-pyridineboronic acid (200 mg, 1.3 mmol) | (APCI-pos mode) m/z (rel intensity): 415.3 (M+H, 100%) |

(continued)

| The following examples are prepared essentially as described in Example 42 except using the reagents in the "Reagents used" column. | | | |
|---|---|---|---|
| Example | Structure and name | Reagents used | Mass Spec |
| 48 | 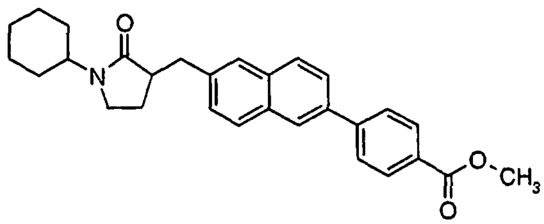 Methyl 4-(6-((1-cyclohexyl-2-oxopyrrolidin-3-yl) methyl)naphthalen-2-yl)benzoate | 3-(6-bromo-naphthalen-2-ylmethyl)-1-cyclohexyl-pyrrolidin-2-one (250 mg, 0.65 mmol) and 4-methoxylcarboylphenylboronic acid (200 mg, 0.52 mmol) | (APCI-pos mode) m/z (rel intensity): 442 (M+H, 100%) |
| 49 | 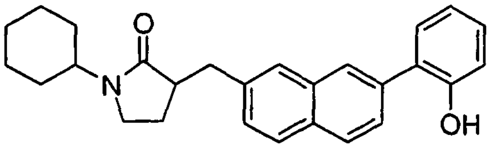 1-Cyclohexyl-3-((7-(2-hydroxyphenyl)naphthalen-2-yl)methyl)pyrrolidin-2-one | 7-((1-cyclohexyl-2-oxopyrrolidin-3-yl) methyl)naphthale n-2-yl trifluoromethanesulfonate (300 mg, 0.66 mmol) and 2-(4,4,5,5,-tetramethyl-1,3,2-dioxaborlan-2-yl) phenol (0.2 g, 0.91 mmol) | (APCI-pos mode) m/z (rel intensity): 400.3 (M+H, 100%) |
| 50 | 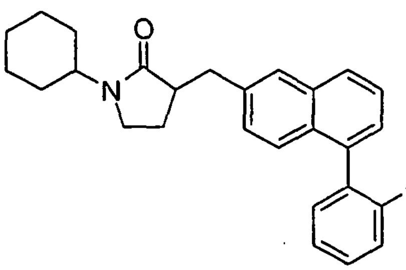 1-Cyclohexyl-3-((5-(2-hydroxyphenyl)naphthalen-2-yl)methyl)pyrrolidin-2-one | 3-((5-bromonaphthalen-2-yl)methyl)-1-cyclohexylpyrrolidi n-2-one (300 mg, 0.78 mmol) and 2-(4,4,5,5,-tetramethyl-1,3,2-dioxaborlan-2-yl) phenol (0.2 g, 0.91 mmol) | (APCI-pos mode) m/z (rel intensity): 400.3 (M+H, 100%) |

Example 51

1-Cyclohexyl-3-((5-vinylnaphthalen-2-yl)methyl)pyrrolidin-2-one

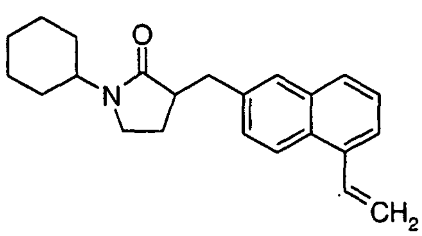

Using the procedure to synthesize Preparation 13 and using reagent 3-(5-bromo-naphthalen-2-ylmethyl)-1-cyclohexyl-pyrrolidin-2-one (2.5 g, 6.5 mmol) affords the title compound (2.0 g, 93%) as a white solid: NMR ($CDCl_3$): 8.04 (d, J=8.98 Hz, 1H), 7.72 (d, J=8.20 Hz, 1H), 7.64 (s, 1H), 7.58 (d, J=7.03Hz, 1H), 7.37-7.50 (m, 3H), 5.76-5.82 (m, 1H), 5.44-5.50 (m, 1H), 3.92-4.02 (m, 1H), 3.32-3.40 (m, 1H), 3.06-3.20 (m, 1H), 2.78-2.88 (m, 1H), 1.94-2.06 (m, 1H),

1.58-1.80 (m. 6H), 1.20-1.45 (m, 4H), 1.00-1.12 (m, 1H).

Example 52

1-Cyclohexyl-3-((7-ethylnaphthalen-2-yl)methyl)pyrrolidin-2-one

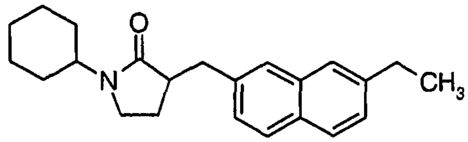

Stir a solution of 1-cyclohexyl-3-((7-vinylnaphthalen-2-yllmethyl)pyrrolidin-2-one (150 mg, 0.27 mmol) and 10 wt % Degussa type Pd/C (10 mg, 0.01 mmol) in a solution of methanol (5 mL) at room temperature for 30 minutes. Filter the mixture through a pad of celite. Concentrate the filtrate to afford the title compound as a white solid (50 mg, 33%): MS (APCI-pos mode) m/z (rel intensity): 336.3 (M+H, 100%).

Example 53

1-Cyclohexyl-3-((5-ethylnaphthalen-2-yl)methyl)pyrrolidin-2-one

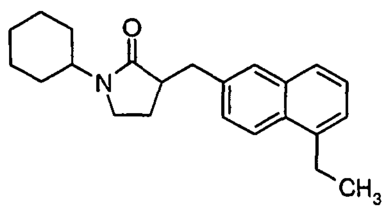

Using the procedure to synthesize Example 52 and using reagent 1-cyclohexyl-3-((5-vinylnaphthalen-2-yl) methyl)pyrrolidin-2-one (90 mg, 0.27 mmol) affords the title compound (60 mg, 67%) as a white solid: MS (APCI-pos mode) m/z (rel intensity): 336.2 (M+H, 100%).

Example 54

7-((1-Cyclohexyl-2-oxopyrrolidin-3-yl)methyl)naphthalen-2-yl trifluoromethanesulfonate

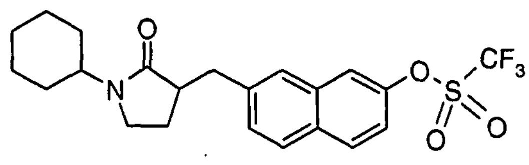

Add $Et_3N$ (3 mL, 21 mmol) into a solution of 1-cyclohexyl-3-(7-hydroxy-naphthalen-2-ylmethyl)-pyrrolidin-2-one (2.5 g, 7.7 mmol) and N-phenyl-bis(trifluoromethanesulfonimide) (3.3 g, 9.24 mmol) in THF (40 mL). Stir the reaction at room temperature for 2 hours. Pour the reaction mixture in water and extract with ethyl acetate. Dry the organic layer over $Na_2SO_4$, filter and concentrated. Purify the residue by column chromatography to afford the product as a white solid (3.16g, 90% yield): MS (APCI-pos mode) m/z (rel intensity): 456.1 (M+H, 100%).

Example 55

Methyl 4-(6-((1-cyclohexyl-2-oxopyrrolidin-3-yl)methyl)naphthalen-1-yl)benzoate

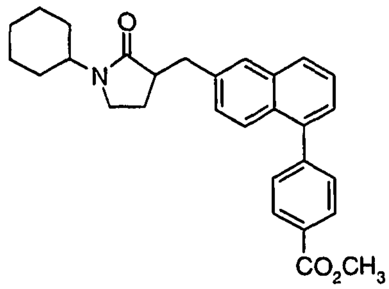

Add 3-((5-bromonaphthalen-2-yl)methyl)-1-cyclohexylpyrrolidin-2-one (150 mg, 0.39 mmol), 4-methoxycarbonylphenylboronic acid (0.2 g, 1 mmol), LiCl (450 mg, 10.7 mmoL) and $Pd(PPh_3)_4$ (60 mg, 0.05 mmol) into a solution of dioxane (20 mL) and 2M $Na_2CO_3$ (aq, 5 mL) and heat at 80˚C for 1 hour. Pour the reaction into water and extract with ethyl acetate. Wash the organic layer with $NaHCO_3$ and brine. Dry, filter and concentrated to obtain the crude mixture. Purify the residue by column chromatography to afford the title compound (100 mg, 75 %) as a pale brown power: MS (APCI-pos mode) m/z (rel intensity): 442.3 (M+H, 100%).

Example 56

4-(6-((1-cyclohexyl-2-oxopyrrolidin-3-yl)methyl)naphthalen-1-yl)benzoic acid

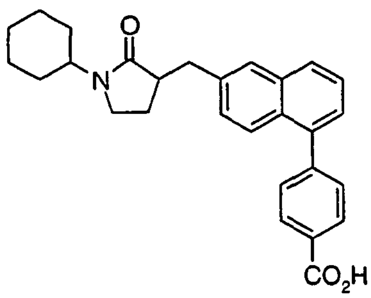

Heat a mixture of methyl 4-(6-((1-cyclohexyl-2-oxopyrrolidin-3-yl)methyl)naphthalen-1-yl)benzoate(100 mg, 0.23 mmol), $LiOH•H_2O$ (100 mg, 2.4 mmol) into a dioxane (5 mL) and H2O (2 mL) at 80˚C for 1 hour. Add 2 M HCl (1 mL) and extract the mixture with ethyl acetate. Dry over sodium sulfate, filter, and concentrate. Purify by silica gel (20-50% ethyl acetate in hexanes) to give the title compound as a white solid (80 mg, 83 %): MS (APCI-pos mode) m/z (rel intensity): 428.3 (M+H, 100%).

Example 57

Methyl 4-(7-((1-cyclohexyl-2-oxopyrrolidin-3-yl)methyl)naphthalen-2-yl)benzoate

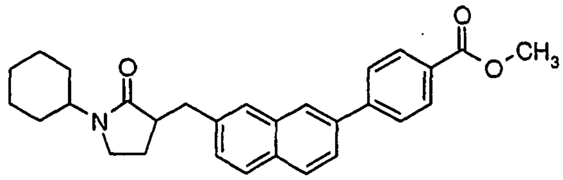

Add 7-((1-cyclohexyl-2-oxopyrrolidin-3-yl)methyl)naphthalen-2-yl-trifluoromethanesulfonate (150 mg, 0.33 mmol), 4-methoxycarbonylphenylboronic acid (0.2 g, 1.0 mmol), LiCl (450 mg, 10.7 mmoL) and $Pd(PPh_3)_4$ (60 mg, 0.05 mmol) into a solution of dioxane (20 mL) and 2M $Na_2CO_3$ (aq, 5 mL) and heat at 80°C for 1 hour. Pour the reaction into water and extract with ethyl acetate. Wash the organic layer with $NaHCO_3$ and brine. Dry, filter and concentrated to obtain the crude mixture. Purify the residue by column chromatography to afford the title compound (100 mg, 75 %): MS (APCI-pos mode) m/z (rel intensity): 442.3 (M+H, 100%).

Example 58

4-(7-((1-cyclohexyl-2-oxopyrrolidin-3-yl)methyl)naphthalen-2-yl)benzoic acid

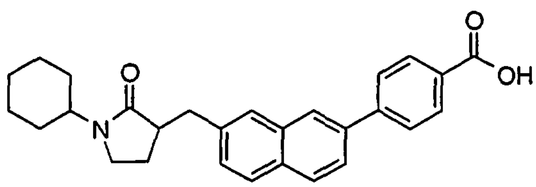

Heat a mixture of methyl 4-(7-((1-cyclohexyl-2-oxopyrrolidin-3-yl)methyl)naphthalen-2-yl)benzoate (100 mg, 0.23 mmol ), $LiOH \cdot H_2O$ (100 mg, 2.4 mmol) into a dioxane (5 mL) and $H_2O$ (2 mL) at 80°C for 1 hour. Add 2 M HCl (1 mL) and extract the mixture with ethyl acetate. Dry over sodium sulfate, filter, and concentrate. Purify by silica gel (20-50% ethyl acetate in hexanes) to give the title compound as a yellow solid (70 mg, 72 %): MS (APCI-pos mode) m/z (rel intensity): 428.2 (M+H, 100%).

Example 59

1-cyclohexyl-3-((5-(2-fluoropyridin-4-yl)naphthalen-2-yl)methyl)pyrrolidin-2-one

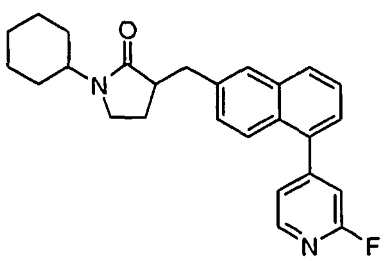

Add 3-((5-bromonaphthalen-2-yl)methyl)-1-cyclohexylpyrrolidin-2-one (300 mg, 0.66 mmol), 2-fluoropyridine-4-boronic acid (0.4 g, 1.1 mmol), LiCl (450 mg, 10.7 mmoL) and $Pd(PPh_3)_4$ (60 mg, 0.05 mmol) into a solution of dioxane (20 mL) and 2M $Na_2CO_3$ (aq, 5 mL) and heat at 80°C for 1 hour. Pour the reaction into water and extract with ethyl acetate. Wash the organic layer with $NaHCO_3$ and brine. Dry, filter and concentrated to obtain the crude mixture. Purify the residue by column chromatography to afford the title compound as a white solid (200 mg, 75 %): MS (APCI-pos

mode) m/z (rel intensity): 403.2 (M+H, 100%).

Example 60

1-cyclohexyl-3-((7-(2-fluoropyridin-4-yl)naphthalen-2-yl)methyl)pyrrolidin-2-one

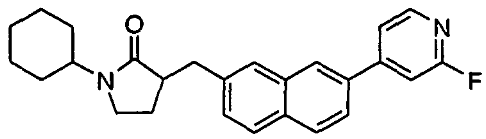

Add 7-((1-cyclohexyl-2-oxopyrrolidin-3-yl)methyl)naphthalen-2-yl trifluoromethanesulfonate (150 mg, 0.33 mmol), 2-fluoropyridine-4-boronic acid (0.2 g, 0.51 mmol), LiCl (450 mg, 10.7 mmoL) and $Pd(PPh_3)_4$ (60 mg, 0.05 mmol) into a solution of dioxane (20 mL) and 2M $Na_2CO_3$ (aq, 5 mL) and heat at 80˚C for 1 hour. Pour the reaction into water and extract with ethyl acetate. Wash the organic layer with $NaHCO_3$ and brine. Dry, filter and concentrated to obtain the crude mixture. Purify the residue by column chromatography to afford the title compound as a white solid (100 mg, 75 %): MS (APCI-pos mode) m/z (rel intensity): 403.2 (M+H, 100%).

Example 61

3-(4-Bromo-naphthalen-1-ylmethyl)-1-cyclohexyl-pyrrolidin-2-one

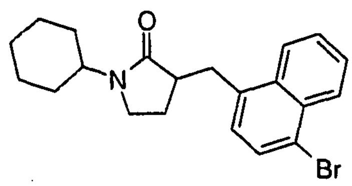

Place diisopropylamine (1.24 ml, 8.88 mmol) in 15 ml of dry tetrahydrofurane. Chill to -78˚ C, add *n*-butillithium (3.55 ml, 8.88 mmol) dropwise, and stir for 15 minutes. Add 1-cyclohexyl-pyrrolidin-2-one (0.97 ml, 5.92 mmol) in 3 ml of tetrahydrofurane and stir for 30 minutes. Add 1-bromo-4-bromomethyl-naphthalene (1.86 g, 6.21 mmol) and gradually warm the reaction mixture to room temperature. Quench with $NH_4Cl$ (saturated solution) and extract with ethyl acetate. Wash the extract with brine. Dry over magnesium sulfate, filter, and concentrate. Purify the residue by column chromatography to afford 1.86 g (81%) of the title compound: Mass spectrum (apci) m/z=287 (M+H).

Example 62

4-[4-(1-Cyclohexyl-2-oxo-pyrrolidin-3-ylmethyl)-naphthalen-1-yl]-benzoic acid methyl ester

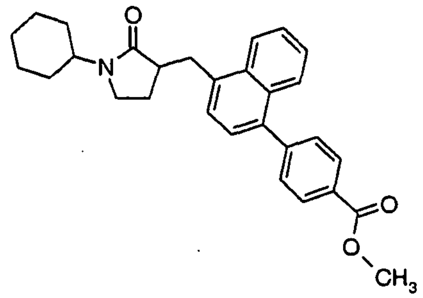

Mix 3-(4-bromo-naphthalen-1-ylmethyl)-1-cyclohexyl-pyrrolidin-2-one (1.20 g, 3.11 mmol), (4-methoxycarbonylphenyl)boronic acid (0.678 g, 3.73 mmol), tetrakis(triphenylphosphine)palladium(0) (0.359 g, 0.31 mmol), and 4.66ml of 2M solution of $K_2CO_3$ in DME (10 mL). Stir for 12 hours at 80° C. Quench the reaction with water and extract with ethyl acetate. Wash the extract with brine and dry over magnesium sulfate. Flash chromatography affords 1.09 g (79%) of the title compound: Mass spectrum (apci) m/z=442 (M+H).

Example 63

(R)-3-(4-Bromo-naphthalen-1-ylmethyl)-1-cyclohexyl-pyrrolidin-2-one

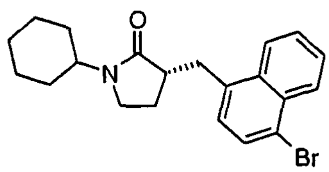

Mix 4-(4-benzyl-2-oxo-oxazolidin-3-yl)-3-(4-bromo-naphthalen-1-ylmethyl)-4-oxo-butyraldehyde (0.300 g, 0.63 mmol), cyclohexylamine (0.145 mL, 1.25 mmol), $NaBH(OAc)_3$ (0.556 g, 2.50 mmol) and acetic acid (0.05 mL, 0.75mmol) in 5 mL of 1,2-dichloroethane. Stir for 12 hours at room temperature. Quench with saturated solution of $NaHCO_3$ and extract with ethyl acetate. Wash the extract with brine. Dry over magnesium sulfate, filter, and concentrate. Flash column chromatography affords 0.156 g (65%) of the title compound: Mass spectrum (apci) m/z=387 (M+H).

Example 64

(R)-4-[4-(1-Cyclohexyl-2-oxo-pyrrolidin-3-ylmethyl)-naphthalen-1-yl]-benzoic acid methyl ester

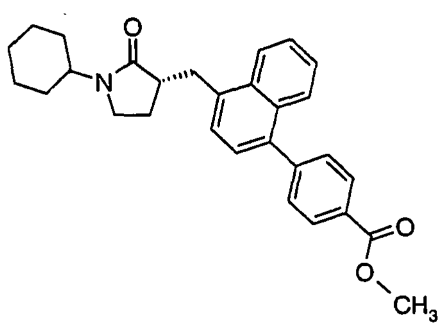

Using the procedure as described in Example 62 and using the reagent (R)-3-(4-bromo-naphthalen-1-ylmethyl)-1-cyclohexyl-pyrrolidin-2-one (0.163 g, 0.35 mmol) affords 94% of the title compound. Mass spectrum (apci) m/z=442

(M+H).

Example 65

(R)-4-[4-(1-Cyclohexyl-2-oxo-pyrrolidin-3-ylmethyl)-naphthalen-1-yl]-benzoic acid

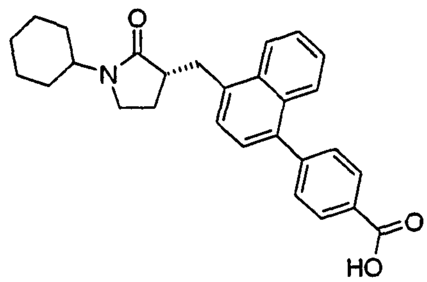

Place (R)-4-[4-(1-cyclohexyl-2-oxo-pyrrolidin-3-ylmethyl)-naphthalen-1-yl]-benzoic acid methyl ester (0.170 g, 0.38 mmol) in 10 ml of methanol and 10 ml of 1N aqueous NaOH. Stir at 80° C for 3 hours. Strip most of the solvent and quench the residue with 1N HCl. Filter precipitate and rinse with water on the filter. Drying affords the title compound in quantitative yield. Mass spectrum (apci) m/z=428 (M+H).

Example 66

3-((1-chloro-6-hydroxynaphthalen-2-yl)methyl)-1-cyclohexylpyrrolidin-2-one

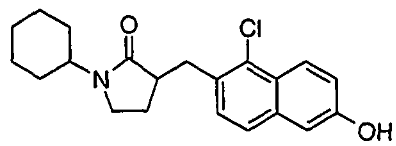

Add $BBr_3$ (1 ml, 10.6 mmol) into a solution of 3-((1-chloro-6-methoxynaphthalen-2-yl)methyl)-1-cyclohexylpyrrolidin-2-one (1.8 g, 4.8 mmoL) in $CH_2Cl_2$ (30 mL) at 0°C. Stir the solution at room temperature for 30 minutes. Pour the reaction mixture into ice and water. Add a few drop of methanol. Extract the reaction with $CH_2Cl_2$, wash with $NaHCO_3$ solution and brine. Dry with sodium sulfate, filtered and concentrated. Purify the residue with silica gel chromatography to afford the title compound (1.4g, 81%) as a white solid.: NMR (DMSO-d6) 9.98 (s, 1H), 8.00-8.20 (m, 1H), 7.60-7.70 (m, 1H), 7.30-7.40 (m, 1H), 7.10-7.25 (m, 2H), 3.60-3.80 (m, 1H), 3.30-3.40 (m, 2H), 3.05-3.25 (m, 2H), 2.70-2.80 (m, 2H), 1.00-1.95 (m, 11H).

**PHARMACOLOGICAL METHODS**

In the following section binding assays as well as functional assays useful for evaluating the efficiency of the compounds of the invention are described.

11β-HSD type 1 enzyme assay

Human 11β-HSD type 1 activity is measured by assaying NADPH production by fluorescence assay. Solid compounds are dissolved in DMSO to a concentration of 10 mM. Twenty microliters of each are then transferred to a column of a 96-well polypropylene Nunc plate where they are further diluted 50-fold followed by subsequent two-fold titration, ten times across the plate with additional DMSO using a Tecan Genesis 200 automated system. Plates are then transferred to a Tecan Freedom 200 system with an attached Tecan Temo 96-well head and an Ultra 384 plate reader. Reagents are supplied in 96-well polypropylene Nunc plates and are dispensed individually into black 96-well

Molecular Devices High Efficiency assay plates (40 μL/ well capacity) in the following fashion: 9 μL/well of substrate (2.22 mM NADP, 55.5 μM Cortisol, 10 mM Tris, 0.25% Prionex, 0.1% Triton X100), 3 μL/well of water to compound wells or 3 μL to control and standard wells, 6 μL/well recombinant human 11β-HSD type 1 enzyme, 2 μL/well of compound dilutions. For ultimate calculation of percent inhibition, a series of wells are added that represent assay minimum and maximum: one set containing substrate with 667 μM carbenoxolone (background), and another set containing substrate and enzyme without compound (maximum signal). Final DMSO concentration is 0.5% for all compounds, controls and standards. Plates are then placed on a shaker by the robotic arm of the Tecan for 15 seconds before being covered and stacked for a three hour incubation period at room temperature. Upon completion of this incubation, the Tecan robotic arm removes each plate individually from the stacker and places them in position for addition of 5 μL/well of a 250 μM carbenoxolone solution to stop the enzymatic reaction. Plates are then shaken once more for 15 seconds then placed into an Ultra 384 microplate reader (355EX/460EM) for detection of NADPH fluorescence.

Acute *In Vivo* Cortisone Conversion Assay

In general, compounds are dosed orally into mice, the mice are challenged with a subcutaneous injection of cortisone at a set timepoint after compound injection, and the blood of each animal is collected some time later. Separated serum is then isolated and analyzed for levels of cortisone and cortisol by LC-MS/MS, followed by calculation of mean cortisol and percent inhibition of each dosing group. Specifically, male C57BL/6 mice are obtained from Harlan Sprague Dawley at average weight of 25 grams. Exact weights are taken upon arrival and the mice randomized into groups of similar weights. Compounds are prepared in 1% w-w HEC, 0.25% w-w polysorbate 80, 0.05% w-w Dow Coming antifoam #1510-US at various doses based on assumed average weight of 25 grams. Compounds are dosed orally, 200 μl per animal, followed by a subcutaneous dose, 200 μl per animal, of 30 mg/kg cortisone at 1 to 24 hours post compound dose. At 10 minutes post cortisone challenge, each animal is euthanized for 1 minute in a $CO_2$ chamber, followed by blood collection via cardiac puncture into serum separator tubes. Once fully clotted, tubes are spun at 2500 x g, 4°C for 15 minutes, the serum is transferred to wells of 96-well plates (Coming Inc, Costar #4410, cluster tubes, 1.2 ml, polypropylene), and the plates frozen at -20°C until analysis by LC-MS/MS. For analysis, serum samples are thawed and the proteins are precipitated by the addition of acetonitrile containing $d_4$-cortisol internal standard. Samples are vortex mixed and centrifuged. The supernatant is removed and dried under a stream of warm nitrogen. Extracts are reconstituted in methanol/water (1:1) and injected onto the LC-MS/MS system. The levels of cortisone and cortisol are assayed by selective reaction monitoring mode following positive ACPI ionization on a triple quadrupole mass spectrophotometer.

All of the examples provided herein have activity in the 11β-HSD type 1 enzyme assay with $IC_{50}$ of less than 30 μM. The assay results are given below for the indicated compound in the 11β-HSD type 1 enzyme assay.

| Example | Structure | 11β-HSD type 1 enzyme assay $IC_{50}$ (nM) |
|---|---|---|
| 2 | 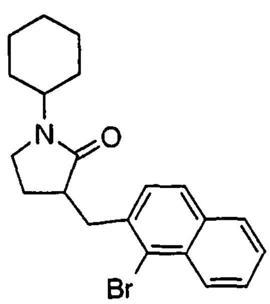 | 354 |
| 4 | 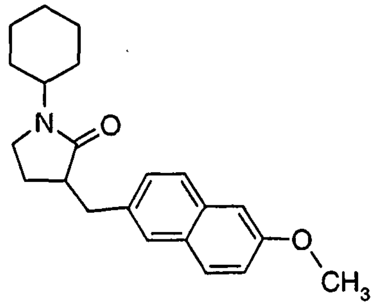 | 134 |

(continued)

| Example | Structure | 11β-HSD type 1 enzyme assay $IC_{50}$ (nM) |
|---|---|---|
| 8 | 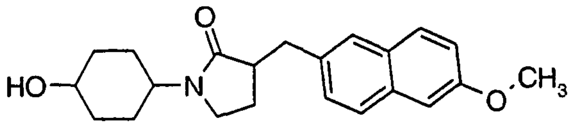 | 247 |
| 24 | 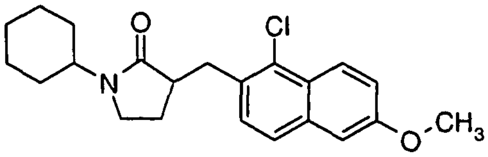 | 213 |
| 34 | 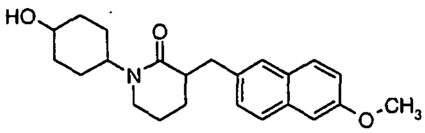 | 353 |
| 40 | 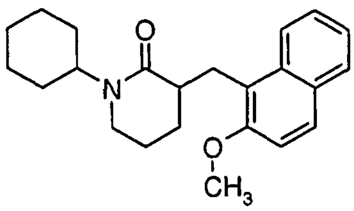 | 423 |
| 44 | 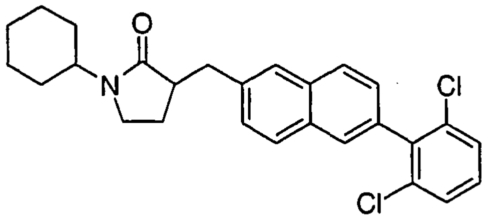 | 344 |

A compound of formula (I) can be formulated with common excipients, diluents, or carriers, and formed into tablets, capsules, and the like. Examples of excipients, diluents, and carriers that are suitable for formulation include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as agar, calcium carbonate, and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonire; and lubricants such as talc, calcium and magnesium stearate and solid polyethyl glycols. Final pharmaceutical forms may be: pills, tablets, powders, lozenges, syrups, aerosols, saches, cachets, elixirs, suspensions, emulsions, ointments, suppositories, sterile injectable solutions, or sterile packaged powders, depending on the type of excipient used.

Additionally, a compound of formula (I) or a pharmaceutically acceptable salt thereof, is suited to formulation as sustained release dosage forms. The formulations can also be so constituted that they release the active ingredient only or preferably in a particular part of the intestinal tract, possibly over a period of time. Such formulations would involve coatings, envelopes, or protective matrices that may be made from polymeric substances or waxes.

The particular dosage of a compound of formula (I) or a pharmaceutically acceptable salt thereof required to constitute an effective amount according to this invention will depend upon the particular circumstances of the conditions to be treated. Considerations such as dosage, route of administration, and frequency of dosing are best decided by the attending physician. Generally, accepted and effective dose ranges for oral or parenteral administration will be from about 0.1 mg/kg/day to about 10 mg/kg/day which translates into about 6 mg to 600 mg, and more typically between 30 mg and 200 mg for human patients. Such dosages will be administered to a patient in need of treatment from one to

three times each day or as often as needed to effectively treat a disease selected from (1) to (21) above.

The compounds of the present invention can be administered alone or in the form of a pharmaceutical composition, that is, combined with pharmaceutically acceptable carriers, or excipients, the proportion and nature of which are determined by the solubility and chemical properties of the compound selected, the chosen route of administration, and standard pharmaceutical practice. The compounds of the present invention, while effective themselves, may be formulated and administered in the form of their pharmaceutically acceptable salts, for purposes of stability, convenience of crystallization, increased solubility, and the like.

The compounds claimed herein can be administered by a variety of routes. In effecting treatment of a patient afflicted with or at risk of developing the disorders described herein, a compound of formula (I) or a pharmaceutically acceptable salt thereof can be administered in any form or mode that makes the compound bioavailable in an effective amount, including oral and parenteral routes. For example, the active compounds can be administered rectally, orally, by inhalation, or by the subcutaneous, intramuscular, intravenous, transdermal, intranasal, rectal, occular, topical, sublingual, buccal, or other routes. Oral administration may be preferred for treatment of the disorders described herein. However, oral administration is the preferred route. Other routes include the intravenous route as a matter of convenience or to avoid potential complications related to oral administration. One skilled in the art of preparing formulations can readily select the proper form and mode of administration depending upon the particular characteristics of the compound selected, the disorder or condition to be treated, the stage of the disorder or condition, and other relevant circumstances. (Remington's Pharmaceutical Sciences, 18th Edition, Mack Publishing Co. (1990)). In those instances where oral administration is impossible or not preferred, the composition may be made available in a form suitable for parenteral administration, e.g., intravenous, intraperitoneal or intramuscular.

## Claims

**1.** A compound of formula I:

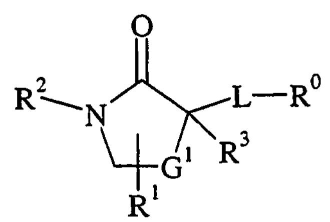

(I)

or a pharmaceutically acceptable salt thereof wherein

$R^0$ is

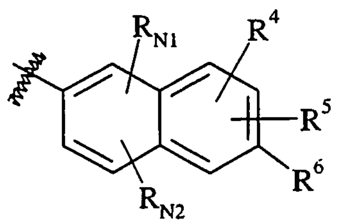

or

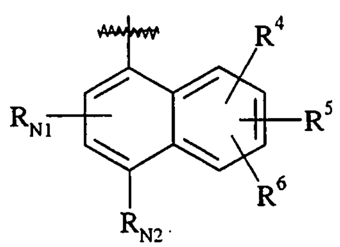

;

wherein the zigzag line represents the point of attachment to the $R^0$ position in formula I;
$G^1$ is methylene or ethylene;
L is $-(C_1-C_4)$alkylene-, -S-, -CH(OH)-, or -O-;
$R^1$ is hydrogen, hydroxy, $-(C_1-C_4)$alkyl(optionally substituted with one to three halogens), $-(C_1-C_4)$alkoxy(optionally substituted with one to three halogens), or
$-CH_2OR^7$, wherein $R^7$ is hydrogen or $-(C_1-C_4)$alkyl;
$R^2$ is

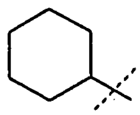,
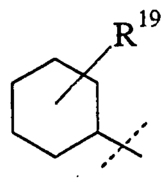,
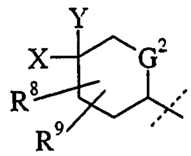,
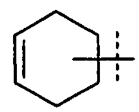,
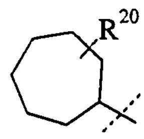,

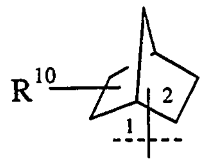,
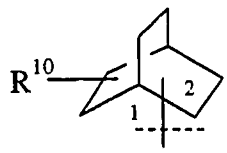,
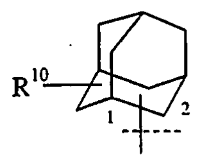,
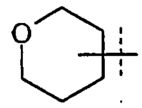,

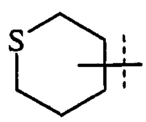,
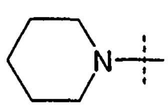,
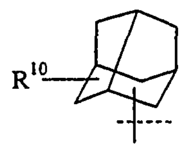,
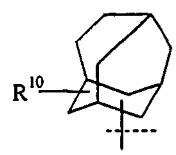, or
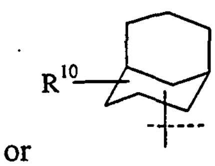;

wherein the dashed line represents the point of attachment to the $R^2$ position in formula I; wherein X is hydrogen, hydroxy, $-OCH_3$ or $-CH_2OH$; wherein Y is hydrogen or methyl, provided that at least one of X and Y is not hydrogen; and wherein optionally X and Y together with the carbon to which they are attached form carbonyl; and wherein $R^8$ is independently hydrogen, hydroxy, $-(C_1-C_4)$alkyl(optionally substituted with one to three halogens); $R^9$ is independently hydrogen, hydroxy, or $-(C_1-C_4)$alkyl(optionally substituted with one to three halogens); $R^{10}$ is independently at each occurrence hydrogen, hydroxy, or $-(C_1-C_4)$alkyl(optionally substituted with one to three halogens); and $G^2$ is methylene, ethylene, or 1-propylene;

$R^3$ is hydrogen, hydroxy, (provided that when L is -S- or -CH(OH)- then $R^3$ is not hydroxy), or $-(C_1-C_4)$alkyl (optionally substituted with one to three halogens);

$R_{N1}$ is hydrogen, hydroxy, halo, $-(C_1-C_4)$alkyl(optionally substituted with one to three halogens), or $-(C_1-C_4)$alkoxy(optionally substituted with one to three halogens);

$R_{N2}$ is hydrogen, hydroxy, halo, $-(C_1-C_4)$alkyl(optionally substituted with one to three halogens), $-(C_1-C_4)$alkoxy (optionally substituted with one to three halogens), $Ar^1$, or $Ar^2$;

$R^4$ is hydrogen, halo, hydroxy, $-(C_1-C_4)$alkyl(optionally substituted with one to three halogens), $-(C_1-C_4)$alkoxy (optionally substituted with one to three halogens), cyano;

$R^5$ is hydrogen, hydroxy, $-(C_1-C_4)$alkyl(optionally substituted with one to three halogens), $-(C_1-C_4)$alkoxy(optionally substituted with one to three halogens), halo, cyano, $-SCF_3$, $-(C_1-C_4)$alkyl-C(O)OH, $-(C_1-C_4)$alkyl-C(O)O$(C_1-C_4)$alkyl, $-(C_1-C_4)$alkyl-OH, -O-$(C_1-C_4)$alkyl-C(O)O$(C_1-C_4)$alkyl, -C(O)O$(C_1-C_4)$alkyl, $-OSO_2CF_3$, $-(C_2-C_4)$alkenyl, $Ar^1$, $Ar^2$, or $-(C_1-C_4)$alkyl-C(O)N$(R^{11})(R^{12})$; wherein $R^{11}$ and $R^{12}$ are each independently hydrogen or $-(C_1-C_4)$alkyl, or $R^{11}$ and $R^{12}$ taken together with the nitrogen to which they are attached form piperidinyl or pyrrolidinyl;

$R^6$ is hydrogen, hydroxy, halo, $-(C_1-C_4)$alkyl(optionally substituted with one to three halogens), $-(C_1-C_4)$alkoxy (optionally substituted with one to three halogens), -C(O)O$(C_1-C_4)$alkyl, $Ar^1$, $Het^1$, $Ar^2$, $Het^2$, $-Ar^1-(C_1-C_4)$alkyl,$-Het^1-(C_1-C_4)$alkyl,

-O-$(C_1-C_4$ alkyl)-$Ar^2$, $-Ar^2-(C_1-C_4)$alkyl, $-Het^2-(C_1-C_4)$alkyl, -C(O)-$Ar^2$, -C(O)-$Het^2$, or -O$(C_1-C_4)$alkyl-N$(R^{13})(R^{14})$; wherein $R^{13}$ and $R^{14}$ are each independently hydrogen or $-(C_1-C_4)$alkyl, or $R^{13}$ and $R^{14}$ taken together with the nitrogen to which they are attached form piperidinyl or pyrrolidinyl; or

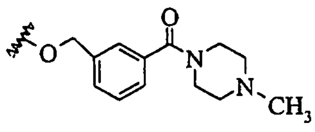;

wherein the zigzag line represents the point of attachment to $Ar^1$;

$Ar^1$ is phenyl or naphthyl;

$Ar^2$ is $Ar^1$ optionally substituted with from one to three moieties selected from halo, hydroxy, cyano, $-(C_1-C_4)$alkyl(optionally substituted with one to three halogens), $-(C_1-C_4$ alkyl)C(O)OH, $-O(C_1-C_4)$alkyl-C(O)OH, $-C(O)O-(C_1-C_4)$alkyl, $-NH_2$, -C(O)OH, $-(C_1-C_4)$alkyl-$N(R^{15})(R^{16})$, $-O(C_1-C_4)$alkyl-$N(R^{15})(R^{16})$, imidazolyl, pyridinyl, or $-(C_1-C_4)$alkyl-imidazolyl; wherein $R^{15}$ and $R^{16}$ are each independently hydrogen or $-(C_1-C_4)$alkyl or $R^{15}$ and $R^{16}$ taken together with the nitrogen to which they are attached form piperidinyl or pyrrolidinyl;

$Het^1$ is a heterocyclic radical selected from pyridinyl, piperidinyl, pyrimidinyl, pyrazinyl, piperazinyl, pyridazinyl, indolyl, isoindolyl, indolinyl, furanyl, benzofuranyl, thiazolyl, oxazolyl, isoxazolyl, isothiazolyl, benzothiophenyl, thiophenyl, quinolinyl, isoquinolinyl, quinoxalinyl, quinazolinyl, or phthalazinyl;

$Het^2$ is $Het^1$ optionally substituted with from one to three moieties selected from halo, hydroxy, cyano, $-CF_3$, $-(C_1-C_4)$alkyl, $-(C_1-C_4)$alkoxy, $-(C_1-C_4)$alkyl-C(O)OH, $-O(C_1-C_4)$alkyl-C(O)OH, $-C(O)O(C_1-C_4)$alkyl, $-(C_1-C_4)$alkyl-$N(R^{17})(R^{18})$, $-O(C_1-C_4)$alkyl-$N(R^{17})(R^{18})$, imidazolyl, pyridinyl, or $-(C_1-C_4)$alkyl-imidazolyl; wherein $R^{17}$ and $R^{18}$ are each independently hydrogen or $-(C_1-C_4)$alkyl or $R^{17}$ and $R^{18}$ taken together with the nitrogen to which they are attached form piperidinyl or pyrrolidinyl; and

$R^{19}$ and $R^{20}$ are each independently hydroxy, $-(C_1-C_4)$alkyl(optionally substituted with one to three halogens), or $-CH_2OH$.

**2.** A compound of claim 1, or a pharmaceutically acceptable salt thereof, wherein R1 is hydrogen and R3 is hydrogen.

**3.** A compound of claim 1 or 2, or a pharmaceutically acceptable salt thereof, wherein L is methylene.

**4.** A compound of any of claims 1 to 3, or a pharmaceutically acceptable salt thereof, wherein $G^1$ is methylene.

**5.** A compound of any of claims 1 to 3, or a pharmaceutically acceptable salt thereof, wherein $G^1$ is ethylene.

**6.** A compound of any of claims 1 to 5 or a pharmaceutically acceptable salt thereof, wherein $Ar^1$ is phenyl.

**7.** A compound of any of claims 1 to 6, or a pharmaceutically acceptable salt thereof wherein $R^2$ is

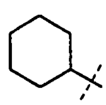 or 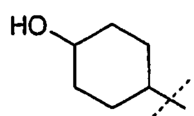

wherein the dashed line represents the point of attachment to the $R^2$ position in formula I.

**8.** A compound of any of claims 1 to 7, or a pharmaceutically acceptable salt thereof, wherein $R_{N1}$ is hydrogen.

**9.** A compound of claim 1, or a pharmaceutically acceptable salt thereof, wherein

$R^0$ is

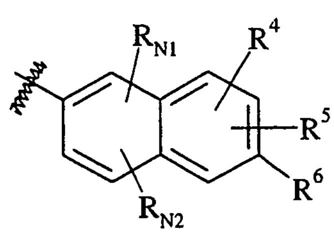 or 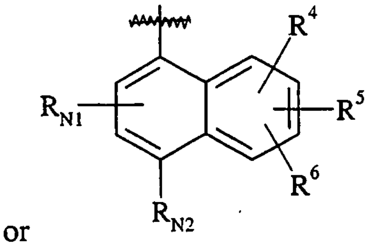 ;

wherein the zigzag line represents the point of attachment to the $R^0$ position in formula I;
$G^1$ is methylene;
L is $-CH_2-$;
$R^1$ is hydrogen;
$R^2$ is

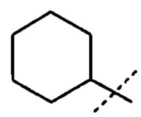 or 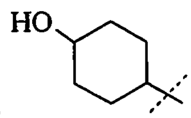 ;

wherein the dashed line represents the point of attachment to the $R^2$ position in formula I;
$R^3$ is hydrogen;
$R_{N1}$ is hydrogen, halo, $-CH_3$, or $-O-CH_3$;
$R_{N2}$ is hydrogen, hydroxy, halo, $-(C_1-C_4)$alkyl(optionally substituted with one to three halogens), $-(C_1-C_4)$alkoxy (optionally substituted with one to three halogens),
$Ar^1$, or $Ar^2$;
$R^4$ is hydrogen or halo;
$R^5$ is hydrogen, hydroxy, $-(C_1-C_4)$alkyl(optionally substituted with one to three halogens), $-(C_1-C_4)$alkoxy(optionally substituted with one to three halogens), halo, cyano, $-SCF_3$, $-(C_1-C_4)$alkyl-C(O)OH,- $(C_1-C_4)$alkyl-C(O)O$(C_1-C_4)$alkyl, $-(C_1-C_4)$alkyl-OH, -O-$(C_1-C_4)$alkyl-C(O)O$(C_1-C_4)$alkyl, -C(O)O$(C_1-C_4)$alkyl, $-OSO_2CF_3$, $-(C_2-C_4)$alkenyl, or $-(C_1-C_4)$alkyl-C(O)N($R^{11}$)($R^{12}$); wherein $R^{11}$ and $R^{12}$ are each independently hydrogen or $-(C_1-C_4)$alkyl, or $R^{11}$ and $R^{12}$ taken together with the nitrogen to which they are attached form piperidinyl or pyrrolidinyl;
$R^6$ is hydroxy, halo, $-(C_1-C_4)$alkyl(optionally substituted with one to three halogens), $-(C_1-C_4)$alkoxy(optionally substituted with one to three halogens), -C(O)O$(C_1-C_4)$alkyl, $Ar^1$, $Het^1$, $Ar^2$, $Het^2$, $-Ar^1-(C_1-C_4)$alkyl,$-Het^1-(C_1-C_4)$alkyl,
-O-$(C_1-C_4$ alkyl)-$Ar^2$, $-Ar^2-(C_1-C_4)$alkyl, $-Het^2-(C_1-C_4)$alkyl, -C(O)-$Ar^2$, -C(O)-$Het^2$, or -O$(C_1-C_4)$alkyl-N($R^{13}$)($R^{14}$); wherein $R^{13}$ and $R^{14}$ are each independently hydrogen or $-(C_1-C_4)$alkyl, or $R^{13}$ and $R^{14}$ taken together with the nitrogen to which they are attached form piperidinyl or pyrrolidinyl; or

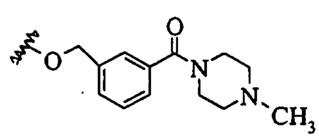 ;

wherein the zigzag line represents the point of attachment to $Ar^1$;
$Ar^1$ is phenyl;
$Ar^2$ is $Ar^1$ optionally substituted once or twice with moieties independently selected from halo, hydroxy, cyano, $-(C_1-C_4)$alkyl(optionally substituted with one to three halogens), $-(C_1-C_4$ alkyl)C(O)OH, -O$(C_1-C_4)$alkyl-C(O)OH, -C(O)O-$(C_1-C_4)$alkyl, $-NH_2$, -C(O)OH, $-(C_1-C_4)$alkyl-N($R^{15}$)($R^{16}$), -O$(C_1-C_4)$alkyl-N($R^{15}$)($R^{16}$); wherein $R^{15}$ and $R^{16}$ are each independently hydrogen or $-(C_1-C_4)$alkyl or $R^{15}$ and
$R^{16}$ taken together with the nitrogen to which they are attached form piperidinyl or pyrrolidinyl;
$Het^1$ is a heterocyclic radical selected from pyridinyl, piperidinyl, pyrimidinyl, pyrazinyl, piperazinyl, pyridazinyl, furanyl, thiazolyl, oxazolyl, isoxazolyl, isothiazolyl, thiophenyl; and
$Het^2$ is $Het^1$ optionally substituted once or twice with moieties independently selected from halo, hydroxy, cyano,

$-CF_3$, $-(C_1-C_4)$alkyl, $-(C_1-C_4)$alkoxy, $-(C_1-C_4)$alkyl-C(O)OH,- $O(C_1-C_4)$alkyl-C(O)OH, $-C(O)O(C_1-C_4)$alkyl, $-(C_1-C_4)$alkyl-$N(R^{17})(R^{18})$, $-O(C_1-C_4)$alkyl-$N(R^{17})(R^{18})$; wherein $R^{17}$ and $R^{18}$ are each independently hydrogen or $-(C_1-C_4)$alkyl or $R^{17}$ and $R^{18}$ taken together with the nitrogen to which they are attached form piperidinyl or pyrrolidinyl.

**10.** A compound according to claim 1, or a pharmaceutically acceptable salt thereof, wherein said compound is selected from the group consisting of:

1-Cyclohexyl-3-naphthalen-2-ylmethyl-pyrrolidin-2-one;
3-(1-Bromo-naphthalen-2-ylmethyl)-1-cyclohexyl-pyrrolidin-2-one;
1-Cyclohexyl-3-naphthalen-1-ylmethyl-pyrrolidin-2-one;
1-Cyclohexyl-3-(6-methoxy-naphthalen-2-ylmethyl)-pyrrolidin-2-one;
1-Cyclohexyl-3-(1,4-dimethyl-naphthalen-2-ylmethyl)-pyrrolidin-2-one;
3-(1-Chloro-naphthalen-2-ylmethyl)-1-cyclohexyl-pyrrolidin-2-one;
1-Cyclohexyl-3-(6-hydroxy-naphthalen-2-ylmethyl)-pyrrolidin-2-one;
1-(*cis*-4-Hydroxy-cyclohexyl)-3-(6-methoxy-naphthalen-2-ylmethyl)-pyrrolidin-2-one;
1-(4-Hydroxy-cyclohexyl)-3-(6-methoxy-naphthalen-2-ylmethyl)-pyrrolidin-2-one;
1-(*cis*)-(4-Hydroxy-cyclohexyl)-3-naphthalen-2-ylmethyl-pyrrolidin-2-one;
1-(4-Hydroxy-cyclohexyl)-3-naphthalen-2-ylmethyl-pyrrolidin-2-one;
1-Cyclohexyl-3-[6-(2-piperidin-1-yl-ethoxy)-naphthalen-2-ylmethyl]-pyrrolidin-2-one hydrochloride salt;
1-Cyclohexyl-3-[6-(3-dimethylamino-propoxy)-naphthalen-2-ylmethyl]-pyrrolidin-2-one hydrochloride salt;
3-[6-(1-Cyclohexyl-2-oxo-pyrrolidin-3-ylmethyl)-naphthalen-2-yloxymethyl]-benzoic acid methyl ester;
3-[6-(1-Cyclohexyl-2-oxo-pyrrolidin-3-ylmethyl)-naphthalen-2-yloxymethyl]-benzoic acid;
1-Cyclohexyl-3-{6-[3-(4-methyl-piperazine-1-carbonyl)-benzyloxy]-naphthalen-2-ylmethyl}-pyrrolidin-2-one trifluoroacetate salt;
1-Cyclohexyl-3-{6-[3-(4-methyl-piperazine-1-carbonyl)-benzyloxy]-naphthalen-2-ylmethyl}-pyrrolidin-2-one;
3-(1-Chloro-naphthalen-2-ylmethyl)-1-(*cis*-4-hydroxy-cyclohexyl)-pyrrolidin-2-one;
3-(1-Chloro-naphthalen-2-ylmethyl)-1-(4-hydroxy-cyclohexyl)-pyrrolidin-2-one;
1-Cyclohexyl-3-(4-methoxy-naphthalen-1-ylmethyl)-pyrrolidin-2-one;
1-Cyclohexyl-3-(2-methoxy-naphthalen-1-ylmethyl)-pyrrolidin-2-one;
3-(5-bromo-6-methoxy-naphthalen-2-ylmethyl)-1-cyclohexyl-pyrrolidin-2-one;
1-Cyclohexyl-3-(7-methoxy-naphthalen-2-ylmethyl)-pyrrolidin-2-one;
3-(6-Bromo-naphthalen-2-ylmethyl)-1-cyclohexyl-pyrrolidin-2-one;
3-((3-Chloronaphthalen-2-yl)methyl)-1-cyclohexylpyrrolidin-2-one;
1-Cyclohexyl-3-((3-methoxynaphthalen-1-yl)methyl)pyrrolidin-2-one;
3-((5-Bromonaphthalen-2-yl)methyl)-1-cyclohexylpyrrolidin-2-one;
3-((1-Chloro-6-methoxynaphthalen-3-yl)methyl)-1-cyclohexylpyrrolidin-2-one;
1-Cyclohexyl-3-(2-hydroxy-naphthalen-1-ylmethyl)-pyrrolidin-2-one;
3-(5-Bromo-6-hydroxy-naphthalen-2-ylmethyl)-1-cyclohexyl-pyrrolidin-2-one;
1-Cyclohexyl-3-(7-hydroxy-naphthalen-2-ylmethyl)-pyrrolidin-2-one;
1-Cyclohexyl-3-(6-isopropoxy-naphthalen-2-ylmethyl)-pyrrolidin-2-one;
1-Cyclohexyl-3-((7-isopropoxynaphthalen-2-yl)methyl)pyrrolidin-2-one;
1-Cyclohexyl-3-naphthalen-2-ylmethyl-piperidin-2-one;
1-Cyclohexyl-3-methyl-3-naphthalen-1-ylmethyl-pyrrolidin-2-one;
*Trans*-3-(3-chloro-naphthalene-2-ylmethyl)-1-(4-hydroxy-cyclohexyl)-piperidine-2-one;
3-(3-chloro-naphthalene-2-ylmethyl)-1-(4-hydroxy-cyclohexyl)-piperidine-2-one;
*Trans*-1-(4-hydroxy-cyclohexyl)-3-naphthalene-2-ylmethyl-piperidine-2-one;
1-(4-hydroxy-cyclohexyl)-3-naphthalene-2-ylmethyl-piperidine-2-one;
*Trans*-1-(4-hydroxy-cyclohexyl)-3-(6-methoxy-naphthalen-2-ylmethyl)-piperidine-2-one;
1-(4-hydroxy-cyclohexyl)-3-(6-methoxy-naphthalen-2-ylmethyl)-piperidine-2-one;
*Cis*-3-(1-chloro-naphthalen-2-ylmethyl)-1-(4-hydroxy-cyclohexyl)-piperidine-2-one;
3-(1-chloro-naphthalen-2-ylmethyl)-1-(4-hydroxy-cyclohexyl)-piperidine-2-one;
Cis-1-(4-hydroxy-cyclohexyl)-3-(6-methoxy-naphthalen-2-ylmethyl)-piperidine-2-one;
1-(4-hydroxy-cyclohexyl)-3-(6-methoxy-naphthalen-2-ylmethyl)-piperidine-2-one;
*Cis*-1-(4-hydroxy-cyclohexyl)-3-naphthalen-2-ylmethyl-piperidine-2-one;
1-(4-hydroxy-cyclohexyl)-3-naphthalen-2-ylmethyl-piperidine-2-one;
*Cis*-1-(4-hydroxy-cyclohexyl)-3-(7-methoxy-naphthalen-2-ylmethyl)-piperidine-2-one;
1-(4-hydroxy-cyclohexyl)-3-(7-methoxy-naphthalen-2-ylmethyl)-piperidine-2-one;

*Cis*-1-(4-hydroxy-cyclohexyl)-3-(3-methoxy-naphthalen-1-ylmethyl)-piperidin-2-one;
1-(4-hydroxy-cyclohexyl)-3-(3-methoxy-naphthalen-1-ylmethyl)-piperidin-2-one;
1-Cyclohexyl-3-(2-methoxy-naphthalene-1-ylmethyl)-piperidin-2-one;
1-Cyclohexyl-3-(3-methoxy-naphthalene-1-ylmethyl)-piperidine-2-one;
3-((6-(3-Aminophenyl)naphthalen-2-yl)methyl)-1-cyclohexylpyrrolidin-2-one;
1-Cyclohexyl-3-((6-(6-fluoropyridin-3-yl)naphthalen-2-yl)methyl)pyrrolidin-2-one;
1-Cyclohexyl-3-((6-(2,6-dichlorophenyl)naphthalen-2-yl)methyl)pyrrolidin-2-one;
1-Cyclohexyl-3-((6-(2,3-dichlorophenyl)naphthalen-2-yl)methyl)pyrrolidin-2-one;
1-Cyclohexyl-3-((6-(2-fluoropyridin-4-yl)naphthalen-2-yl)methyl)pyrrolidin-2-one;
1-Cyclohexyl-3-((6-(6-methoxypyridin-3-yl)naphthalen-2-yl)methyl)pyrrolidin-2-one;
Methyl 4-(6-((1-cyclohexyl-2-oxopyrrolidin-3-yl)methyl)naphthalen-2-yl)benzoate;
1-Cyclohexyl-3-((7-(2-hydroxyphenyl)naphthalen-2-yl)methyl)pyrrolidin-2-one;
1-Cyclohexyl-3-((5-(2-hydroxyphenyl)naphthalen-2-yl)methyl)pyrrolidin-2-one;
1-Cyclohexyl-3-((5-vinylnaphthalen-2-yl)methyl)pyrrolidin-2-one;
1-Cyclohexyl-3-((7-ethylnaphthalen-2-yl)methyl)pyrrolidin-2-one;
1-Cyclohexyl-3-((5-ethylnaphthalen-2-yl)methyl)pyrrolidin-2-one;
7-((1-Cyclohexyl-2-oxopyrrolidin-3-yl)methyl)naphthalen-2-yl trifluoromethanesulfonate;
Methyl 4-(6-((1-cyclohexyl-2-oxopyrrolidin-3-yl)methyl)naphthalen-1-yl)benzoate;
4-(6-((1-cyclohexyl-2-oxopyrrolidin-3-yl)methyl)naphthalen-1-yl)benzoic acid;
Methyl 4-(7-((1-cyclohexyl-2-oxopyrrolidin-3-yl)methyl)naphthalen-2-yl)benzoate;
4-(7-((1-cyclohexyl-2-oxopyrrolidin-3-yl)methyl)naphthalen-2-yl)benzoic acid;
1-cyclohexyl-3-((5-(2-fluoropyridin-4-yl)naphthalen-2-yl)methyl)pyrrolidin-2-one;
1-cyclohexyl-3-((7-(2-fluoropyridin-4-yl)naphthalen-2-yl)methyl)pyrrolidin-2-one;
3-(4-Bromo-naphthalen-1-ylmethyl)-1-cyclohexyl-pyrrolidin-2-one;
4-[4-(1-Cyclohexyl-2-oxo-pyrrolidin-3-ylmethyl)-naphthalen-1-yl]-benzoic acid methyl ester;
(R)-3-(4-Bromo-naphthalen-1-ylmethyl)-1-cyclohexyl-pyrrolidin-2-one;
3-(4-Bromo-naphthalen-1-ylmethyl)-1-cyclohexyl-pyrrolidin-2-one;
(R)-4-[4-(1-Cyclohexyl-2-oxo-pyrrolidin-3-ylmethyl)-naphthalen-1-yl]-benzoic acid methyl ester;
4-[4-(1-Cyclohexyl-2-oxo-pyrrolidin-3-ylmethyl)-naphthalen-1-yl]-benzoic acid methyl ester;
(R)-4-[4-(1-Cyclohexyl-2-oxo-pyrrolidin-3-ylmethyl)-naphthalen-1-yl]-benzoic acid;
4-[4-(1-Cyclohexyl-2-oxo-pyrrolidin-3-ylmethyl)-naphthalen-1-yl]-benzoic acid; and
3-((1-chloro-6-hydroxynaphthalen-2-yl)methyl)-1-cyclohexylpyrrolidin-2-one.

**11.** A pharmaceutical composition which comprises a compound of any of claims 1-10 and a pharmaceutically acceptable carrier.

**12.** A compound of formula I, or a pharmaceutically acceptable salt thereof, as claimed in any one of claims 1-10, for use as a medicament.

**13.** The use of a compound of any one of claims 1-10 or a pharmaceutically acceptable salt thereof, for the manufacture of a medicament for the treatment of metabolic syndrome.

**14.** The use of a compound of any one of claims 1-10, or a pharmaceutically acceptable salt thereof, for the manufacture of a medicament for the treatment of diabetes.

**15.** The use of a compound of any one of claims 1-10, or a pharmaceutically acceptable salt thereof, for the manufacture of a medicament for the treatment of(1) hyperglycemia, (2) low glucose tolerance, (3) insulin resistance, (4) obesity, (5) lipid disorders, (6) dyslipidemia, (7) hyperlipidemia, (8) hypertriglyceridemia, (9) hypercholesterolemia, (10) low HDL levels, (11) high LDL levels, (12) atherosclerosis and its sequelae, (13) vascular restenosis, (14) pancreatitis, (15) abdominal obesity, (16) neurodegenerative disease, (17) retinopathy, (18) nephropathy, (19) neuropathy, (20) metabolic syndrome, and other conditions and disorders where insulin resistance is a component.

**Patentansprüche**

**1.** Verbindung der Formel

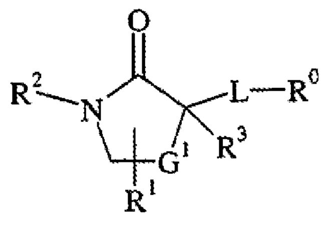

(I)

oder ein pharmazeutisch akzeptables Salz hiervon,
worin R° für die folgenden Gruppen steht

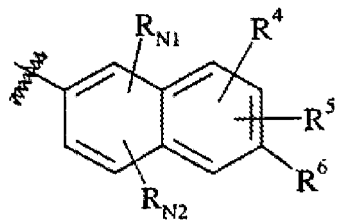 oder 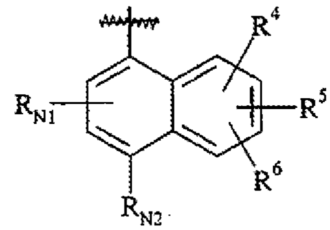 ,

die Zickzacklinie für den Befestigungspunkt an der Position $R^0$ in Formel I steht,

$G^1$ für Methylen oder Ethylen steht,
L für -($C_1$-$C_4$)-Alkylen-, -S-, -CH(OH)- oder -O- steht,
$R^1$ für Wasserstoff, Hydroxy, -($C_1$-$C_4$)-Alkyl (das optional substituiert ist mit ein bis drei Halogenen), -($C_1$-$C_4$)-Alkoxy (das optional substituiert ist mit ein bis drei Halogenen) oder -$CH_2OR^7$ steht, worin $R^7$ für Wasserstoff oder -($C_1$-$C_4$)-Alkyl steht,
$R^2$ für die folgenden Gruppen steht

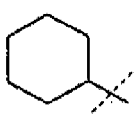 , 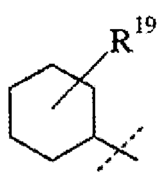 , 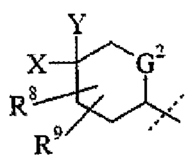 , 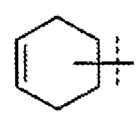 , 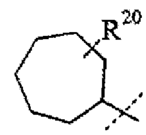 ,

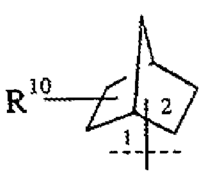 , 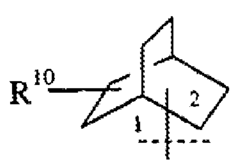 , 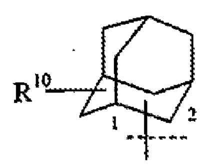 , 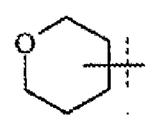 ,

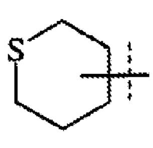 , 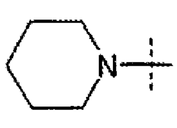 , 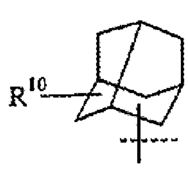 , 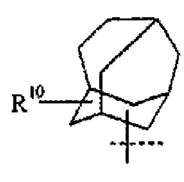 , oder 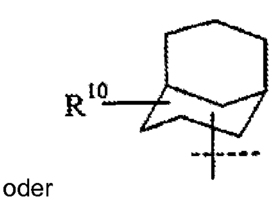 ,

worin

die gestrichelte Linie den Punkt der Befestigung an der Position von $R^2$ bedeutet,

X für Wasserstoff, Hydroxy, $-OCH_3$- oder $-CH_2OH$ steht,
Y für Wasserstoff oder Methyl steht, mit der Maßgabe, dass wenigstens eines von X und Y nicht für Wasserstoff steht, oder
X und Y zusammen mit dem Kohlenstoffatom, an das sie gebunden sind, optional für Carbonyl stehen, $R^8$ unabhängig für Wasserstoff, Hydroxy, $-(C_1-C_4)$-Alkyl (das optional substituiert ist mit ein bis drei Halogenen) steht,
$R^9$ unabhängig für Wasserstoff, Hydroxy oder $-(C_1-C_4)$-Alkyl (das optional substituiert ist mit ein bis drei Halogenen) steht,
$R^{10}$ unabhängig an jedem Vorkommen für Wasserstoff, Hydroxy oder $-(C_1-C_4)$-Alkyl (das optional substituiert ist mit ein bis drei Halogenen) steht, und
$G^2$ für Methylen, Ethylen oder 1-Propylen steht,
$R^3$ für Wasserstoff, Hydroxy (mit der Maßgabe, dass, falls L für -S- oder -CH(OH)- steht, dann $R^3$ nicht Hydroxy ist) oder für $-(C_1-C_4)$-Alkyl (das optional substituiert ist mit ein bis drei Halogenen) steht,
$R_{N1}$ für Wasserstoff, Hydroxy, Halogen, $-(C_1-C_4)$-Alkyl (das optional mit ein bis drei Halogenen substituiert ist) oder für $-(C_1-C_4)$-Alkoxy (das optional substituiert ist mit ein bis drei Halogenen) steht,
$R_{N2}$ für Wasserstoff, Hydroxy, Halogen, $-(C_1-C_4)$-Alkyl (das optional substituiert ist mit ein bis drei Halogenen), für $-(C_1-C_4)$-Alkoxy (das optional substituiert ist mit ein bis drei Halogenen), für $Ar^1$ oder $Ar^2$ steht,
$R^4$ für Wasserstoff, Halogen, Hydroxy, $-(C_1-C_4)$-Alkyl (das optional substituiert ist mit ein bis drei Halogenen), für $-(C_1-C_4)$-Alkoxy (das optional substituiert ist mit ein n bis drei Halogenen) oder für Cyano steht,
$R^5$ steht für Wasserstoff, Hydroxy, $-(C_1-C_4)$-Alkyl (das optional substituiert ist mit ein bis drei Halogenen), für-$(C_1-C_4)$-Alkoxy (das optional substituiert ist mit ein bis drei Halogenen), für Halogen, Cyano, $-SCF_3$, $-(C_1-C_4)$-Alkyl-C(O)OH, $-(C_1-C_4)$-Alkyl-C(O)O-$(C_1-C_4)$-alkyl, $-(C_1-C_4)$-Alkyl-OH, -O-$(C_1-C_4)$-Alkyl-C(O)O$(C_1-C_4)$-alkyl, -C(O)O-$(C_1-C_4)$-Alkyl, $-OSO_2CF_3$, $-(C_2-C_4)$-Alkenyl, $Ar^1$, $Ar^2$ oder-$(C_1-C_4)$-Alkyl-C(O)N($R^{11}$)($R^{12}$),

worin

$R^{11}$ und $R^{12}$ jeweils unabhängig für Wasserstoff oder $-(C_1-C_4)$-Alkyl stehen, oder
$R^{11}$ und $R^{12}$ zusammen mit dem Stickstoffatom, an das sie gebunden sind, für Piperidinyl oder Pyrrolidinyl stehen,
$R^6$ steht für Wasserstoff, Hydroxy, Halogen, $-(C_1-C_4)$-Alkyl (das optional substituiert ist mit ein bis drei Halogenen), für $-(C_1-C_4)$-Alkoxy (das optional substituiert ist mit ein bis drei Halogenen), für -C(O)O-$(C_1-C_4)$-Alkyl, $Ar^1$, $Het^1$,$Ar^2$,$Het^2$,-$Ar^1$-$(C_1-C_4)$-Alkyl,-$Het^1$-$(C_1-C_4)$-Alkyl,-O-($C_1$-$C_4$-Alkyl)-$Ar^2$,-$Ar^2$-$(C_1-C_4)$-Alkyl,-$Het^2$-$(C_1-C_4)$-Alkyl, -C(O)-$Ar^2$, -C(O)-$Het^2$ oder für -O-$(C_1-C_4)$-Alkyl-N($R^{13}$)($R^{14}$), worin
$R^{13}$ und $R^{14}$ jeweils unabhängig für Wasserstoff oder $-(C_1-C_4)$-Alkyl stehen, oder
$R^{13}$ und $R^{14}$ zusammen mit dem Stickstoffatom, an das sie gebunden sind, für Piperidinyl, Pyrrolidinyl oder den folgenden Rest stehen

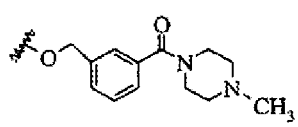,

worin
die Zickzacklinie für den Punkt der Befestigung an $Ar^1$ steht,

$Ar^1$ für Phenyl oder Naphthyl steht,
$Ar^2$ für $Ar^1$ steht, das optional substituiert ist mit ein bis drei Gruppen, die ausgewählt sind aus Halogen, Hydroxy, Cyano, $-(C_1-C_4)$-Alkyl (das optional substituiert ist mit ein bis drei Halogenen), aus -($C_1$-$C_4$-Alkyl)-C(O)OH, -O-$(C_1-C_4)$-Alkyl-C(O)OH, -C(O)O-$(C_1-C_4)$-Alkyl, $-NH_2$, -C(O)OH, $-(C_1-C_4)$-Alkyl-N($R^{15}$)($R^{16}$), -O$(C_1-C_4)$-Alkyl-N($R^{15}$)($R^{16}$), Imidazolyl, Pyridinyl oder aus $-(C_1-C_4)$-Alkylimidazolyl, worin
$R^{15}$ und $R^{16}$ jeweils unabhängig für Wasserstoff oder $-(C_1-C_4)$-Alkyl stehen, oder
$R^{15}$ oder $R^{16}$ zusammen mit dem Stickstoffatom, an das sie gebunden sind, für Piperidinyl oder Pyrrolidinyl stehen,
$Het^1$ für einen heterocyclischen Rest steht, der ausgewählt ist aus Pyridinyl, Piperidinyl, Pyrimidinyl, Pyrazinyl, Piperazinyl, Pyridazinyl, Indolyl, Isoindolyl, Indolinyl, Furanyl, Benzofuranyl, Thiazolyl, Oxazolyl, Isoxazolyl,

Isothiazolyl, Benzothiophenyl, Thiophenyl, Chinolinyl, Isochinolinyl, Chinoxalinyl, Chinazolinyl oder Phthalazinyl,
$Het^2$ für $Het^1$ steht, das optional substituiert ist mit ein bis drei Resten, die ausgewählt sind aus Halogen, Hydroxy, Cyano, $-CF_3$, $-(C_1-C_4)$-Alkyl, $-(C_1-C_4)$-Alkoxy, $-(C_1-C_4)$-Alkyl-C(O)OH, -O-$(C_1-C_4)$-Alkyl-C(O)OH, -C(O)O-$(C_1-C_4)$-Alkyl, $-(C_1-C_4)$-Alkyl-N($R^{17}$)($R^{18}$), -O-$(C_1-C_4)$-Alkyl-N($R^{17}$)($R^{18}$), Imidazolyl, Pyridinyl oder aus $-(C_1-C_4)$-Alkylimidazolyl, worin
$R^{17}$ und $R^{18}$ jeweils unabhängig für Wasserstoff oder $-(C_1-C_4)$-Alkyl stehen, oder
$R^{17}$ und $R^{18}$ zusammen mit dem Stickstoffatom, an das sie gebunden sind, für Piperidinyl oder Pyrrolidinyl stehen, und
$R^{19}$ und $R^{20}$ jeweils unabhängig für Hydroxy, $-(C_1-C_4)$-Alkyl (das optional substituiert ist mit ein bis drei Halogenen) oder für $-CH_2OH$ stehen.

**2.** Verbindung nach Anspruch 1 oder ein pharmazeutisch akzeptables Salz hiervon, worin $R^1$ für Wasserstoff steht und $R^3$ für Wasserstoff steht.

**3.** Verbindung nach Anspruch 1 oder 2 oder ein pharmazeutisch akzeptables Salz hiervon, worin L für Methylen steht.

**4.** Verbindung nach einem der Ansprüche 1 bis 3 oder ein pharmazeutisch akzeptables Salz hiervon, worin $G^1$ für Methylen steht.

**5.** Verbindung nach einem der Ansprüche 1 bis 3 oder ein pharmazeutisch akzeptables Salz hiervon, worin $G^1$ für Ethylen steht.

**6.** Verbindung nach einem der Ansprüche 1 bis 5 oder ein pharmazeutisch akzeptables Salz hiervon, worin $Ar^1$ für Phenyl steht.

**7.** Verbindung nach einem der Ansprüche 1 bis 6 oder ein pharmazeutisch akzeptables Salz hiervon, worin $R^2$ für die folgenden Gruppen steht

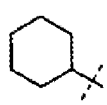
oder
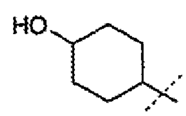
,

worin
die gestrichelte Linie den Befestigungspunkt an der Position von $R^2$ in der Formel I bedeutet.

**8.** Verbindung nach einem der Ansprüche 1 bis 7 oder ein pharmazeutisch akzeptables Salz hiervon, worin $R_{N1}$ für Wasserstoff steht.

**9.** Verbindung nach Anspruch 1 oder ein pharmazeutisch akzeptables Salz hiervon,
worin

R° für die folgenden Gruppen steht

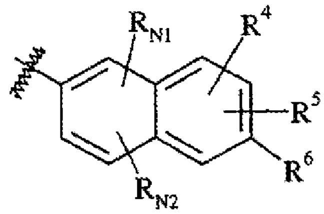
oder
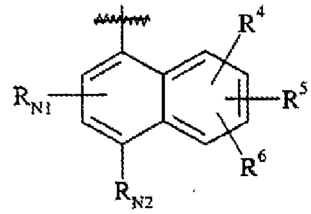
,

worin
die Zickzacklinie den Befestigungspunkt an der Position von R° in Formel bedeutet,

$G^1$ für Methylen steht,
L für $-CH_2-$ steht,
$R^1$ für Wasserstoff steht,
$R^2$ für die folgenden Gruppen steht

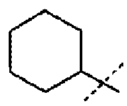 oder 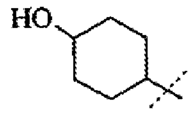,

worin
die gestrichelte Linie den Befestigungspunkt an der Position von $R^2$ in Formel I bedeutet,

$R^3$ für Wasserstoff steht,
$R_{N1}$ für Wasserstoff, Halogen, $-CH_3$ oder $-O-CH_3$ steht,
$R_{N2}$ für Wasserstoff, Hydroxy, Halogen, $-(C_1-C_4)$-Alkyl (das optional substituiert ist mit ein bis drei Halogenen), für $-(C_1-C_4)$-Alkoxy (das optional substituiert ist mit ein bis drei Halogenen), für $Ar^1$ oder $Ar^2$ steht,
$R^4$ für Wasserstoff oder Halogen steht,
$R^5$ steht für Wasserstoff, Hydroxy, $-(C_1-C_4)$-Alkyl (das optional substituiert ist mit ein bis drei Halogenen), für-$(C_1-C_4)$-Alkoxy (das optional substituiert ist mit ein bis drei Halogenen), für Halogen, Cyano, $-SCF_3$, $-(C_1-C_4)$-Alkyl-C(O)OH, $-(C_1-C_4)$-Alkyl-C(O)O-$(C_1-C_4)$-alkyl, $-(C_1-C_4)$-Alkyl-OH, -O-$(C_1-C_4)$-Alkyl-C(O)O-$(C_1-C_4)$-alkyl, -C(O)O-$(C_1-C_4)$-Alkyl, $-OSO_2CF_3$, $-(C_2-C_4)$-Alkenyl oder für-$(C_1-C_4)$-Alkyl-C(O)N($R^{11}$)($R^{12}$),

worin

$R^{11}$ und $R^{12}$ jeweils unabhängig für Wasserstoff oder $-(C_1-C_4)$-Alkyl stehen, oder
$R^{11}$ und $R^{12}$ zusammen mit dem Stickstoffatom, an das sie gebunden sind, für Pipieridinyl oder Pyrrolidinyl stehen,
$R^6$ steht für Hydroxy, Halogen, $-(C_1-C_4)$-Alkyl (das optional substituiert ist mit ein bis drei Halogenen), für $-(C_1-C_4)$-Alkoxy (das optional substituiert ist mit ein bis drei Halogenen), für -C(O)O-$(C_1-C_4)$-Alkyl, $Ar^1$, $Het^1$, $Ar^2$, $Het^2$, $-Ar^1-(C_1-C_4)$-Alkyl, $-Het^1-(C_1-C_4)$-Alkyl, -O-$(C_1-C_4$-Alkyl)-$Ar^2$, $-Ar^2-(C_1-C_4)$-Alkyl, $-Het^2-(C_1-C_4)$-Alkyl, -C(O)-$Ar^2$, -C(O)-$Het^2$ oder für -O-$(C_1-C_4)$-Alkyl-N($R^{13}$)($R^{14}$), worin
$R^{13}$ und $R^{14}$ jeweils unabhängig für Wasserstoff oder $-(C_1-C_4)$-Alkyl stehen, oder worin
$R^{13}$ und $R^{14}$ zusammen mit dem Stickstoffatom, an das sie gebunden sind, für Piperidinyl, Pyrrolidinyl oder den folgenden Rest stehen

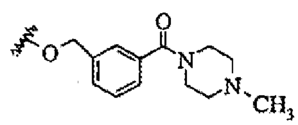,

worin
die Zickzacklinie für den Punkt der Befestigung an $Ar^1$ steht,

$Ar^1$ für Phenyl steht,
$Ar^2$ für $Ar^1$ steht, das optional substituiert ist mit ein oder zwei Gruppen, die unabhängig ausgewählt sind aus Halogen, Hydroxy, Cyano, $-(C_1-C_4)$-Alkyl (das optional substituiert ist mit ein bis drei Halogenen), aus -$(C_1-C_4$-Alkyl)-C(O)OH, -O-$(C_1-C_4)$-Alkyl-C(O)OH, -C(O)O-$(C_1-C_4)$-Alkyl, $-NH_2$, -C(O)OH, $-(C_1-C_4)$-Alkyl-N($R^{15}$)($R^{16}$) oder aus -O$(C_1-C_4)$-Alkyl-N($R^{15}$)($R^{16}$), worin
$R^{15}$ und $R^{16}$ jeweils unabhängig für Wasserstoff oder $-(C_1-C_4)$-Alkyl stehen, oder worin
$R^{15}$ oder $R^{16}$ zusammen mit dem Stickstoffatom, an das sie gebunden sind, für Piperidinyl oder Pyrrolidinyl stehen,
$Het^1$ für einen heterocyclischen Ring steht, der ausgewählt ist aus Pyridinyl, Piperidinyl, Pyrimidinyl, Pyrazinyl, Piperazinyl, Pyridazinyl, Furanyl, Thiazolyl, Oxazolyl, Isoxazolyl, Isothiazolyl oder Thiophenyl, und
$Het^2$ für $Het^1$ steht, das optional substituiert ist durch ein oder zwei Gruppen, die unabhängig ausgewählt sind aus Halogen, Hydroxy, Cyano, $-CF_3$, $-(C_1-C_4)$-Alkyl, $-(C_1-C_4)$-Alkoxy, $-(C_1-C_4)$-Alkyl-C(O)OH, -O-$(C_1-C_4)$-Alkyl-

C(O)OH, -C(O)O-($C_1$-$C_4$)-Alkyl, -($C_1$-$C_4$)-Alkyl-N($R^{17}$)($R^{18}$) oder -O-($C_1$-$C_4$)-Alkyl-N($R^{17}$)($R^{18}$),

worin

$R^{17}$ und $R^{18}$ jeweils unabhängig für Wasserstoff oder -($C_1$-$C_4$)-Alkyl stehen, oder
$R^{17}$ und $R^{18}$ zusammen mit dem Stickstoffatom, an das sie gebunden sind, für Piperidinyl oder Pyrrolidinyl stehen.

**10.** Verbindung nach Anspruch 1 oder ein pharmazeutisch akzeptables Salz hiervon, worin diese Verbindung ausgewählt ist aus der Gruppe, die besteht aus:

1-Cyclohexyl-3-naphthalin-2-ylmethylpyrrolidin-2-on;
3-(1-Bromnaphthalin-2-ylmethyl)-1-cyclohexylpyrrolidin-2-on;
1-Cyclohexyl-3-naphthalin-1-ylmethyl-pyrrolidin-2-on;
1-Cyclohexyl-3-(6-methoxynaphthalin-2-ylmethyl)-pyrrolidin-2-on;
1-Cyclohexyl-3-(1,4-dimethylnaphthalin-2-ylmethyl)-pyrrolidin-2-on;
3-(1-Chlornaphthalin-2-ylmethyl)-1-cyclohexylpyrrolidin-2-on;
1-Cyclohexyl-3-(6-hydroxynaphthalin-2-ylmethyl)-pyrrolidin-2-on;
1-(cis-4-Hydroxycyclohexyl)-3-(6-methoxynaphthalin-2-ylmethyl)-pyrrolidin-2-on;
1-(4-Hydroxycyclohexyl)-3-(6-methoxynaphthalin-2-ylmethyl)-pyrrolidin-2-on;
1-(cis)-(4-Hydroxycyclohexyl)-3-naphthalin-2-ylmethylpyrrolidin-2-on;
1-(4-Hydroxycyclohexyl)-3-naphthalin-2-ylmethylpyrrolidin-2-on;
1-Cyclohexyl-3-[6-(2-piperidin-1-ylethoxy)-naphthalin-2-ylmethyl]-pyrrolidin-2-onhydrochloridsalz;
1-Cyclohexyl-3-[6-(3-dimethylaminopropoxy)-naphthalin-2-ylmethyl]-pyrrolidin-2-onhydrochloridsalz;
3-[6-(1-Cyclohexyl-2-oxopyrrolidin-3-ylmethyl)-naphthalin-2-yloxymethyl]-benzoesäuremethylester;
3-[6-(1-Cyclohexyl-2-oxopyrrolidin-3-ylmethyl)-naphthalin-2-yloxymethyl]-benzoesäure;
1- Cyclohexyl- 3- {6-[3-(4- methylpiperazin- 1- carbonyl)-benzyloxy]-naphthalin- 2- ylmethyl}-pyrrolidin- 2- ontrifluoracetatsalz;
1-Cyclohexyl-3-{6-[3-(4-methylpiperazin-1-carbonyl)-benzyloxy]-naphthalin-2-ylmethyl}-pyrrolidin-2-on;
3-(1-Chlornaphthalin-2-ylmethyl)-1-(cis-4-hydroxycyclohexyl)-pyrrolidin-2-on;
3-(1-Chlornaphthalin-2-ylmethyl)-1-(4-hydroxycyclohexyl)-pyrrolidin-2-on;
1-Cyclohexyl-3-(4-methoxynaphthalin-1-ylmethyl)-pyrrolidin-2-on;
1-Cyclohexyl-3-(2-methoxynaphthalin-1-ylmethyl)-pyrrolidin-2-on;
3-(5-Brom-6-methoxynaphthalin-2-ylmethyl)-1-cyclohexylpyrrolidin-2-on;
1-Cyclohexyl-3-(7-methoxynaphthalin-2-ylmethyl)-pyrrolidin-2-on;
3-(6-Bromnaphthalin-2-ylmethyl)-1-cyclohexylpyrrolidin-2-on;
3-((3-Chlornaphthalin-2-yl)-methyl)-1-cyclohexylpyrrolidin-2-on;
1-Cyclohexyl-3-((3-methoxynaphthalin-1-yl)-methyl)-pyrrolidin-2-on;
3-((5-Bromnaphthalin-2-yl)-methyl)-1-cyclohexylpyrrolidin-2-on;
3-((1-Chlor-6-methoxynaphthalin-2-yl)-methyl)-1-cyclohexylpyrrolidin-2-on;
1-Cyclohexyl-3-(2-hydroxynaphthalin-1-ylmethyl)-pyrrolidin-2-on;
3-(5-Brom-6-hydroxynaphthalin-2-ylmethyl)-1-cyclohexylpyrrolidin-2-on;
1-Cyclohexyl-3-(7-hydroxynaphthalin-2-ylmethyl)-pyrrolidin-2-on;
1-Cyclohexyl-3-(6-isopropoxynaphthalin-2-ylmethyl)-pyrrolidin-2-on;
1-Cyclohexyl-3-((7-isopropoxynaphthalin-2-yl)-methyl)-pyrrolidin-2-on;
1-Cyclohexyl-3-naphthalin-2-ylmethylpiperidin-2-on;
1-Cyclohexyl-3-methyl-3-naphthalin-1-ylmethylpyrrolidin-2-on;
trans-3-(3-Chlornaphthalin-2-ylmethyl)-1-(4-hydroxycyclohexyl)-piperidin-2-on;
3-(3-Chlornaphthalin-2-ylmethyl)-1-(4-hydroxycyclohexyl)-piperidin-2-on;
trans-1-(4-Hydroxycyclohexyl)-3-naphthalin-2-ylmethylpiperidin-2-on;
1-(4-Hydroxycyclohexyl)-3-naphthalin-2-ylmethylpiperidin-2-on;
trans-1-(4-Hydroxycyclohexyl)-3-(6-methoxynaphthalin-2-ylmethyl)-piperidin-2-on;
1-(4-Hydroxycyclohexyl)-3-(6-methoxynaphthalin-2-ylmethyl)-piperidin-2-on;
cis-3-(1-Chlornaphthalin-2-ylmethyl)-1-(4-hydroxycyclohexyl)-piperidin-2-on;
3-(1-Chlornaphthalin-2-ylmethyl)-1-(4-hydroxycyclohexyl)-piperidin-2-on;
cis-1-(4-Hydroxycyclohexyl)-3-(6-methoxynaphthalin-2-ylmethyl)-piperidin-2-on;
1-(4-Hydroxycyclohexyl)-3-(6-methoxynaphthalin-2-ylmethyl)-piperidin-2-on;
cis-1-(4-Hydroxycyclohexyl)-3-naphthalin-2-ylmethylpiperidin-2-on;

1-(4-Hydroxycyclohexyl)-3-naphthalin-2-ylmethylpiperidin-2-on;
cis-1-(4-Hydroxycyclohexyl)-3-(7-methoxynaphthalin-2-ylmethyl)-piperidin-2-on;
1-(4-Hydroxycyclohexyl)-3-(7-methoxynaphthalin-2-ylmethyl)-piperidin-2-on;
cis-1-(4-Hydroxycyclohexyl)-3-(3-methoxynaphthalin-1-ylmethyl)-piperidin-2-on;
1-(4-Hydroxycyclohexyl)-3-(3-methoxynaphthalin-1-ylmethyl)-piperidin-2-on;
1-Cyclohexyl-3-(2-methoxynaphthalin-1-ylmethyl)-piperidin-2-on;
1-Cyclohexyl-3-(3-methoxynaphthalin-1-ylmethyl)-piperidin-2-on;
3-((6-(3-Aminophenyl)-naphthalin-2-yl)-methyl)-1-cyclohexylpyrrolidin-2-on;
1-Cyclohexyl-3-((6-(6-fluorpyridin-3-yl)-naphthalin-2-yl)-methyl)-pyrrolidin-2-on;
1-Cyclohexyl-3-((6-(2,6-dichlorphenyl)-naphthalin-2-yl)-methyl)-pyrrolidin-2-on;
1-Cyclohexyl-3-((6-(2,3-dichlorphenyl)-naphthalin-2-yl)-methyl)-pyrrolidin-2-on;
1-Cyclohexyl-3-((6-(2-fluorpyridin-4-yl)-naphthalin-2-yl)-methyl)-pyrrolidin-2-on;
1-Cyclohexyl-3-((6-(6-methoxypyridin-3-yl)-naphthalin-2-yl)-methyl)-pyrrolidin-2-on;
Methyl-4-(6-((1-cyclohexyl-2-oxopyrrolidin-3-yl)-methyl)-naphthalin-2-yl)-benzoat;
1-Cyclohexyl-3-((7-(2-hydroxyphenyl)-naphthalin-2-yl)-methyl)-pyrrolidin-2-on;
1-Cyclohexyl-3-((5-(2-hydroxyphenyl)-naphthalin-2-yl)-methyl)-pyrrolidin-2-on;
1-Cyclohexyl-3-((5-vinylnaphthalin-2-yl)-methyl)-pyrrolidin-2-on;
1-Cyclohexyl-3-((7-ethylnaphthalin-2-yl)-methyl)-pyrrolidin-2-on;
1-Cyclohexyl-3-((5-ethylnaphthalin-2-yl)-methyl)-pyrrolidin-2-on;
7-((1-Cyclohexyl-2-oxopyrrolidin-3-yl)-methyl)-naphthalin-2-yltrifluormethansulfonat;
Methyl-4-(6-((1-cyclohexyl-2-oxopyrrolidin-3-yl)-methyl)-naphthalin-1-yl)-benzoat;
4-(6-((1-Cyclohexyl-2-oxopyrrolidin-3-yl)-methyl)-naphthalin-1-yl)-benzoesäure;
Methyl-4-(7-((1-cyclohexyl-2-oxopyrrolidin-3-yl)-methyl)-naphthalin-2-yl)-benzoat;
4-(7-((1-Cyclohexyl-2-oxopyrrolidin-3-yl)-methyl)-naphthalin-2-yl)-benzoesäure;
1-Cyclohexyl-3-((5-(2-fluorpyridin-4-yl)-naphthalin-2-yl)-methyl)-pyrrolidin-2-on;
1-Cyclohexyl-3-((7-(2-fluorpyridin-4-yl)-naphthalin-2-yl)-methyl)-pyrrolidin-2-on;
3-(4-Bromnaphthalin-1-ylmethyl)-1-cyclohexylpyrrolidin-2-on;
4-[4-(1-Cyclohexyl-2-oxopyrrolidin-3-ylmethyl)-naphthalin-1-yl]-benzoesäuremethylester;
(R)-3-(4-Bromnaphthalin-1-ylmethyl)-1-cyclohexylpyrrolidin-2-on;
3-(4-Bromnaphthalin-1-ylmethyl)-1-cyclohexylpyrrolidin-2-on;
(R)-4-[4-(1-Cyclohexyl-2-oxopyrrolidin-3-ylmethyl)-naphthalin-1-yl]-benzoesäuremethylester;
4-[4-(1-Cyclohexyl-2-oxopyrrolidin-3-ylmethyl)-naphthalin-1-yl]-benzoesäuremethylester;
(R)-4-[4-(1-Cyclohexyl-2-oxopyrrolidin-3-ylmethyl)-naphthalin-1-yl]-benzoesäure;
4-[4-(1-Cyclohexyl-2-oxopyrrolidin-3-ylmethyl)-naphthalin-1-yl]-benzoesäure; und
3-((1-Chlor-6-hydroxynaphthalin-2-yl)-methyl)-1-cyclohexylpyrrolidin-2-on.

**11.** Pharmazeutische Zusammensetzung, umfassend eine Verbindung nach einem der Ansprüche 1 bis 10 und einen pharmazeutisch akzeptablen Träger.

**12.** Verbindung der Formel I oder ein pharmazeutisch akzeptables Salz hiervon nach einem der Ansprüche 1 bis 10 zur Verwendung als ein Arzneimittel.

**13.** Verwendung einer Verbindung nach einem der Ansprüche 1 bis 10 oder eines pharmazeutisch akzeptablen Salzes hiervon zur Herstellung eines Arzneimittels für die Behandlung von metabolischem Syndrom.

**14.** Verwendung einer Verbindung nach einem der Ansprüche 1 bis 10 oder eines pharmazeutisch akzeptablen Salzes hiervon zur Herstellung eines Arzneimittels für die Behandlung von Diabetes.

**15.** Verwendung einer Verbindung nach einem der Ansprüche 1 bis 10 oder eines pharmazeutisch akzeptablen Salzes hiervon zur Herstellung eines Arzneimittels für die Behandlung von (1) Hyperglykämie, (2) niedriger Glucosetoleranz, (3) Insulinresistenz, (4) Obesität, (5) Fettstoffwechselstörung, (6) Dyslipidämie, (7), Hyperlipidämie, (8) Hypertriglyceridämie, (9) Hypercholesterinämie, (10) niedrigen HDL Spiegeln, (11) hohen LDL Spiegeln, (12) Atherosklerose und ihren Folgekrankheiten, (13) Vaskularrestenose, (14) Pankreatitis, (15) Abodminalobesität, (16) neurodegenerativer Krankheit, (17) Retinopathie, (18) Nephropathie, (19) Neuropathie, (20) metabolischem Syndrom und anderen Zuständen und Störungen, wo eine Insulinresistenz eine Komponente ist.

## Revendications

**1.** Composé de formule 1 :

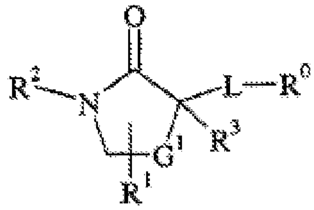

(I)

ou un sel pharmaceutiquement acceptable de celui-ci dans lequel

$R^\circ$ représente

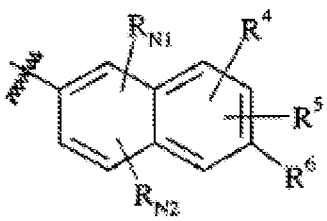 ou 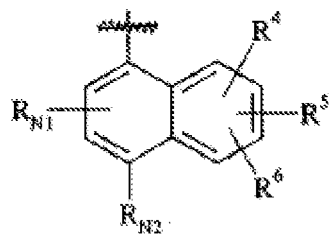 ;

dans lequel la ligne en zigzag représente le point d'attachement sur la position de $R^\circ$ dans la formule 1 ;
$G^1$ représente un groupe méthylène ou éthylène ;
L représente un groupe -alkylène en ($C_1$-$C_4$)-, -S-, -CH(OH)-, ou -O- ;
$R^1$ représente
un atome d'hydrogène, un groupe hydroxy, -alkyle en ($C_1$-$C_4$) (éventuellement substitué par un à trois atome (s) d'halogène), -alcoxy en ($C_1$-$C_4$) (éventuellement substitué par un à trois atome(s) d'halogène), ou $-CH_2OR^7$, dans lequel $R^7$ représente un atome d'hydrogène ou un groupe -alkyle en ($C_1$-$C_4$) ;
$R^2$ représente

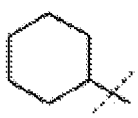 , 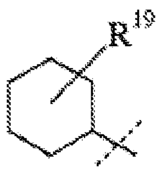 , 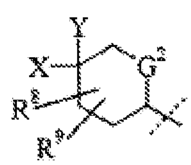 , 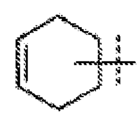 , 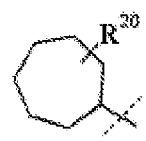 ,

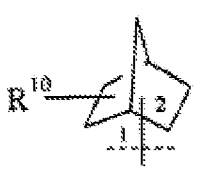 , 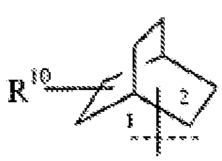 , 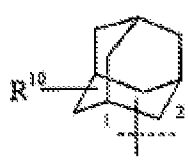 , 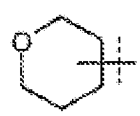 ,

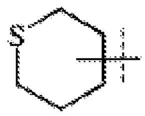 , 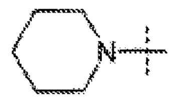 , 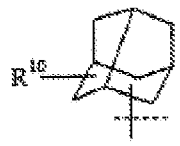 , 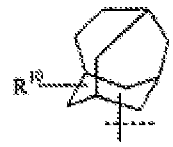 , ou 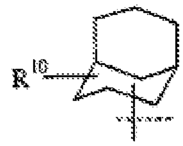 ;

dans lequel la ligne pointillée représente le point d'attachement sur la position de $R^2$ dans la formule 1 ; dans lequel X représente un atome d'hydrogène, un groupe hydroxy, $-OCH_3$ ou $-CH_2OH$ ; dans lequel Y représente un atome d'hydrogène ou un groupe méthyle, à condition qu'au moins l'un parmi X et Y ne soit pas un atome d'hydrogène ; et dans lequel, éventuellement, X et Y conjointement au carbone auquel ils sont attachés forment un groupe carbonyle ; et dans lequel $R^8$ représente, de manière indépendante, un atome d'hydrogène, un groupe hydroxy, -alkyle en ($C_1$-$C_4$) (éventuellement substitué par un à trois atome(s) d'halogène) ; $R^9$ représente, de manière indépendante, un atome d'hydrogène, un groupe hydroxy, ou -alkyle en ($C_1$-$C_4$) (éventuellement substitué par un à trois atome(s) d'halogène) ; $R^{10}$ représente, de manière indépendante, à chaque occurrence, un atome d'hydrogène, un groupe hydroxy, ou -alkyle en ($C_1$-$C_4$) (éventuellement substitué par un à trois atome(s) d'halogène) ; et $G^2$ représente un groupe méthylène, éthylène, ou 1-propylène ;

$R^3$ représente

un atome d'hydrogène, un groupe hydroxy, (à condition que lorsque L représente -S- ou -CH(OH)- alors $R^3$ ne soit pas un groupe hydroxy), ou -alkyle en ($C_1$-$C_4$) (éventuellement substitué par un à trois atome(s) d'halogène) ;

$R_{N1}$ représente

un atome d'hydrogène, un groupe hydroxy, un atome d'halogène, un groupe -alkyle en ($C_1$-$C_4$) (éventuellement substitué par un à trois atome(s) d'halogène), ou -alcoxy en ($C_1$-$C_4$) (éventuellement substitué par un à trois atome(s) d'halogène) ;

$R_{N2}$ représente

un atome d'hydrogène, un groupe hydroxy, un atome d'halogène, un groupe -alkyle en ($C_1$-$C_4$) (éventuellement substitué par un à trois atome(s) d'halogène), -alcoxy en ($C_1$-$C_4$) (éventuellement substitué par un à trois atome (s) d'halogène), $Ar^1$, ou $Ar^2$ ;

$R^4$ représente

un atome d'hydrogène, d'halogène, un groupe hydroxy, -alkyle en ($C_1$-$C_4$) (éventuellement substitué par un à trois atome(s) d'halogène), -alcoxy en ($C_1$-$C_4$) (éventuellement substitué par un à trois atome(s) d'halogène), cyano ;

$R^5$ représente

un atome d'hydrogène, un groupe hydroxy, -alkyle en ($C_1$-$C_4$) (éventuellement substitué par un à trois atome (s) d'halogène), -alcoxy en ($C_1$-$C_4$) (éventuellement substitué par un à trois atome(s) d'halogène), un atome d'halogène, un groupe cyano, $-SCF_3$, -alkyle en ($C_1$-$C_4$)-C(O)OH, -alkyle en ($C_1$-$C_4$)-C(O)Oalkyle en ($C_1$-$C_4$), -alkyle en ($C_1$-$C_4$)-OH, -O-alkyle en ($C_1$-$C_4$)-C(O)Oalkyle en ($C_1$-$C_4$), -C(O)Oalkyle en ($C_1$-$C_4$), $-OSO_2CF_3$, -alcényle en ($C_2$-$C_4$), $Ar^1$, $Ar^2$, ou -alkyle en ($C_1$-$C_4$)-C(O)N($R^{11}$)($R^{12}$) ; dans lequel $R^{11}$ et $R^{12}$ représentent chacun, de manière indépendante, un atome d'hydrogène ou un groupe -alkyle en ($C_1$-$C_4$), ou bien $R^{11}$ et $R^{12}$ pris conjointement avec l'atome d'azote auquel ils sont attachés forment un groupe pipéridinyle ou pyrrolidinyle ;

$R^6$ représente

un atome d'hydrogène, un groupe hydroxy, un atome d'halogène, un groupe -alkyle en ($C_1$-$C_4$) (éventuellement substitué par un à trois atome(s) d'halogène), -alcoxy en ($C_1$-$C_4$) (éventuellement substitué par un à trois atome (s) d'halogène), -C(O)Oalkyle en ($C_1$-$C_4$), $Ar^1$, $Het^1$, $Ar^2$, $Het^2$, -$Ar^1$-alkyle en ($C_1$-$C_4$), -$Het^1$-alkyle en ($C_1$-$C_4$), -O-alkyle en ($C_1$-$C_4$)-$Ar^2$, -$Ar^2$-alkyle en ($C_1$-$C_4$), -$Het^2$-alkyle en ($C_1$-$C_4$), -C(O)-$Ar^2$, -C(O)-$Het^2$, ou -Oalkyle en ($C_1$-$C_4$)-N($R^{13}$)($R^{14}$) ; dans lequel $R^{13}$ et $R^{14}$ représentent chacun, de manière indépendante, un atome hydrogène ou un groupe -alkyle en ($C_1$-$C_4$), ou bien $R^{13}$ et $R^{14}$ pris conjointement avec l'atome d'azote auquel ils sont attachés forment un groupe pipéridinyle ou pyrrolidinyle ; ou

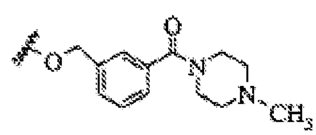 ;

dans lequel la ligne en zigzag représente le point d'attachement sur $Ar^1$ ;

$Ar^1$ représente un groupe phényle ou naphtyle ;
$Ar^2$ représente
$Ar^1$ éventuellement substitué par une à trois fraction(s) choisie(s) parmi un atome d'halogène, un groupe hydroxy, cyano, -alkyle en ($C_1$-$C_4$) (éventuellement substitué par un à trois atome(s) d'halogène), -alkyle en ($C_1$-$C_4$)C(O)OH, -Oalkyle en ($C_1$-$C_4$)-C(O)OH, -C(O)O-alkyle en ($C_1$-$C_4$), -$NH_2$, -C(O)OH, -alkyle en ($C_1$-$C_4$)-N($R^{15}$)($R^{16}$), -Oalkyle en ($C_1$-$C_4$)-N($R^{15}$)($R^{16}$), imidazolyle, pyridinyle, ou -alkyle en ($C_1$-$C_4$)-imidazolyle ; dans lequel $R^{15}$ et $R^{16}$ représentent chacun, de manière indépendante, un atome d'hydrogène ou un groupe -alkyle en ($C_1$-$C_4$) ou bien $R^{15}$ et $R^{16}$ pris conjointement avec l'atome d'azote auquel ils sont attachés forment un groupe pipéridinyle ou pyrrolidinyle ;
$Het^1$ représente
un radical hétérocyclique choisi parmi un groupe pyridinyle, pipéridinyle, pyrimidinyle, pyrazinyle, pipérazinyle, pyridazinyle, indolyle, isoindolyle, indolinyle, furanyle, benzofuranyle, thiazolyle, oxazolyle, isoxazolyle, isothiazolyle, benzothiophényle, thiophényle, quinolinyle, isoquinolinyle, quinoxalinyle, quinazolinyle, ou phtalazinyle ;
$Het^2$ représente
$Het^1$ éventuellement substitué par une à trois fraction(s) choisie(s) parmi un atome d'halogène, un groupe hydroxy, cyano, -$CF_3$, -alkyle en ($C_1$-$C_4$), -alcoxy en ($C_1$-$C_4$), -alkyle en ($C_1$-$C_4$)-C(O)OH, -Oalkyle en ($C_1$-$C_4$)-C(O)OH, -C(O)Oalkyle en ($C_1$-$C_4$), -alkyle en ($C_1$-$C_4$)-N($R^{17}$)($R^{18}$), -Oalkyle en ($C_1$-$C_4$)-N($R^{17}$)($R^{18}$), imidazolyle, pyridinyle, ou -alkyle en ($C_1$-$C_4$)-imidazolyle ; dans lequel $R^{17}$ et $R^{18}$ représentent chacun, de manière indépendante, un atome d'hydrogène ou un groupe -alkyle en ($C_1$-$C_4$) ou bien $R^{17}$ et $R^{18}$ pris conjointement avec l'atome d'azote auquel ils sont attachés forment un groupe pipéridinyle ou pyrrolidinyle, et
$R^{19}$ et $R^{20}$ représentent chacun, de manière indépendante,
un groupe hydroxy, -alkyle en ($C_1$-$C_4$) (éventuellement substitué par un à trois atome(s) d'halogène), ou -$CH_2$OH.

2. Composé selon la revendication 1, ou un sel pharmaceutiquement acceptable de celui-ci, dans lequel $R^1$ représente un atome d'hydrogène et $R^3$ représente un atome d'hydrogène.

3. Composé selon la revendication 1 ou 2, ou un sel pharmaceutiquement acceptable de celui-ci, dans lequel L représente un groupe méthylène.

4. Composé selon l'une quelconque des revendications 1 à 3, ou un sel pharmaceutiquement acceptable de celui-ci, dans lequel $G^1$ représente un groupe méthylène.

5. Composé selon l'une quelconque des revendications 1 à 3, ou un sel pharmaceutiquement acceptable de celui-ci, dans lequel $G^1$ représente un groupe éthylène.

6. Composé selon l'une quelconque des revendications 1 à 5, ou un sel pharmaceutiquement acceptable de celui-ci, dans lequel $Ar^1$ représente un groupe phényle.

7. Composé selon l'une quelconque des revendications 1 à 6, ou un sel pharmaceutiquement acceptable de celui-ci, dans lequel $R^2$ représente

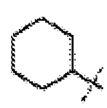 ou 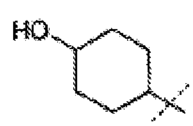

dans lequel la ligne pointillée représente le point d'attachement sur la position de $R^2$ dans la formule I.

8. Composé selon l'une quelconque des revendications 1 à 7, ou un sel pharmaceutiquement acceptable de celui-ci, dans lequel $R_{N1}$ représente un atome d'hydrogène.

9. Composé selon la revendication 1, ou un sel pharmaceutiquement acceptable de celui-ci, dans lequel R° représente

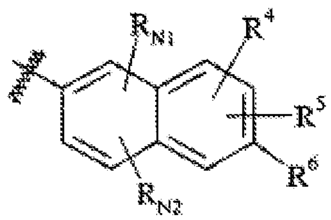 ou 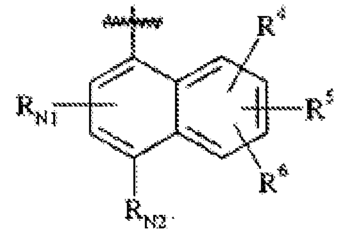 ;

dans lequel la ligne en zigzag représente le point d'attachement sur la position de R° dans la formule 1 ;

$G^1$ représente un groupe méthylène ;
L représente $-CH_2-$ ;
$R^1$ représente un atome d'hydrogène ;
$R^2$ représente

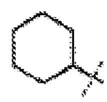 ou 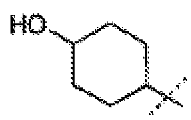 ;

dans lequel la ligne pointillée représente le point d'attachement sur la position de $R^2$ dans la formule 1 ;
$R^3$ représente un atome d'hydrogène ;
$R_{N1}$ représente un atome d'hydrogène, d'halogène, $-CH_3-$, ou $-O-CH_3-$ ;
$R_{N2}$ représente un atome d'hydrogène, un groupe hydroxy, un atome d'halogène, un groupe -alkyle en $(C_1-C_4)$ (éventuellement substitué par un à trois atome(s) d'halogène), -alcoxy en $(C_1-C_4)$ (éventuellement substitué par un à trois atome(s) d'halogène), $Ar^1$, ou $Ar^2$ ;
$R^4$ représente un atome d'hydrogène ou d'halogène ;
$R^5$ représente
un atome d'hydrogène, un groupe hydroxy, -alkyle en $(C_1-C_4)$ (éventuellement substitué par un à trois atome (s) d'halogène), -alcoxy en $(C_1-C_4)$ (éventuellement substitué par un à trois atome(s) d'halogène), un atome d'halogène, un groupe cyano, $-SCF_3$, -alkyle en $(C_1-C_4)$-C(O)OH, -alkyle en $(C_1-C_4)$-C-(O)Oalkyle en $(C_1-C_4)$, -alkyle en $(C_1-C_4)$-OH, -O-alkyle en $(C_1-C_4)$-C(O)Oalkyle en $(C_1-C_4)$, -C(O)Oalkyle en $(C_1-C_4)$, $-OSO_2CF_3$, -alcényle en $(C_2-C_4)$, ou -alkyle en $(C_1-C_4)$-C(O)N($R^{11}$)($R^{12}$); dans lequel $R^{11}$ et $R^{12}$ représentent chacun, de manière indépendante, un atome d'hydrogène ou un groupe -alkyle en $(C_1-C_4)$, ou bien $R^{11}$ et $R^{12}$ pris conjointement avec l'atome d'azote auquel ils sont attachés forment un groupe pipéridinyle ou pyrrolidinyle ;
$R^6$ représente
un groupe hydroxy, un atome d'halogène, un groupe -alkyle en $(C_1-C_4)$ (éventuellement substitué par un à trois atome(s) d'halogène), -alcoxy en $(C_1-C_4)$ (éventuellement substitué par un à trois atome(s) d'halogène), -C(O) Oalkyle en $(C_1-C_4)$, $Ar^1$, $Het^1$, $Ar^2$, $Het^2$, -$Ar^1$-alkyle en $(C_1-C_4)$, -$Het^1$-alkyle en $(C_1-C_4)$, -O-alkyle en $(C_1-C_4)$-$Ar^2$, -$Ar^2$-alkyle en $(C_1-C_4)$, -$Het^2$-alkyle en $(C_1-C_4)$, -C(O)-$Ar^2$, -C(O)-$Het^2$, ou -Oalkyle en $(C_1-C_4)$-N($R^{13}$)($R^{14}$) ; dans lequel $R^{13}$ et $R^{14}$ représentent chacun, de manière indépendante, un atome d'hydrogène ou un groupe -alkyle en $(C_1-C_4)$, ou bien $R^{13}$ et $R^{14}$ pris conjointement avec l'atome d'azote auquel ils sont attachés forment un groupe pipéridinyle ou pyrrolidinyle ; ou

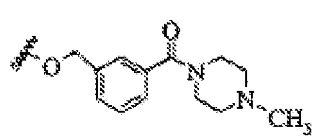 ;

dans lequel la ligne en zigzag représente le point d'attachement sur $Ar^1$ ;
$Ar^1$ représente un groupe phényle ;
$Ar^2$ représente $Ar^1$ éventuellement substitué une ou deux fois par des fractions choisies, de manière indépendante, parmi un atome d'halogène, un groupe hydroxy, cyano, -alkyle en $(C_1-C_4)$ (éventuellement substitué

par un à trois atome(s) d'halogène), -alkyle en ($C_1$-$C_4$)C(O)OH, -Oalkyle en ($C_1$-$C_4$)-C(O)OH, -C(O)O-alkyle en ($C_1$-$C_4$), -$NH_2$, -C(O)OH, -alkyle en ($C_1$-$C_4$)-N($R^{15}$)($R^{16}$), -Oalkyle en ($C_1$-$C_4$)-N($R^{15}$)($R^{16}$) ; dans lequel $R^{15}$ et $R^{16}$ représentent chacun, de manière indépendante, un atome d'hydrogène ou un groupe -alkyle en ($C_1$-$C_4$) ou bien $R^{15}$ et $R^{16}$ pris conjointement avec l'atome d'azote auquel ils sont attachés forment un groupe pipéridinyle ou pyrrolidinyle ;

$Het^1$ représente un radical hétérocyclique choisi parmi un groupe pyridinyle, pipéridinyle, pyrimidinyle, pyrazinyle, pipérazinyle, pyridazinyle, furanyle, thiazolyle, oxazolyle, isoxazolyle, isothiazolyle, thiophényle ; et

$Het^2$ représente $Het^1$ éventuellement substitué une ou deux fois par des fractions choisies, de manière indépendante, parmi un atome d'halogène, un groupe hydroxy, cyano, -$CF_3$, -alkyle en ($C_1$-$C_4$), -alcoxy en ($C_1$-$C_4$), -alkyle en ($C_1$-$C_4$)-C(O)OH, -Oalkyle en ($C_1$-$C_4$)-C(O)OH, -C(O)Oalkyle en ($C_1$-$C_4$), -alkyle en ($C_1$-$C_4$)-N($R^{17}$)($R^{18}$), -Oalkyle en ($C_1$-$C_4$)-N($R^{17}$)($R^{18}$) ; dans lequel $R^{17}$ et $R^{18}$ représentent chacun, de manière indépendante, un atome d'hydrogène ou un groupe -alkyle en ($C_1$-$C_4$) ou bien $R^{17}$ et $R^{18}$ pris conjointement avec l'atome d'azote auquel ils sont attachés forment un groupe pipéridinyle ou pyrrolidinyle.

**10.** Composé selon la revendication 1, ou un sel pharmaceutiquement acceptable de celui-ci, dans lequel ledit composé est choisi dans le groupe constitué par :

la 1-cyclohexyl-3-naphtalén-2-ylméthyl-pyrrolidin-2-one ;
la 3-(1-bromo-naphtalén-2-ylméthyl)-1-cyclohexyl-pyrrolidin-2-one ;
la 1-cyclohexyl-3-naphtalén-1-ylméthyl-pyrrolidin-2-one ;
la 1-cyclohexyl-3-(6-méthoxy-naphtalén-2-ylméthyl)-pyrrolidin-2-one ;
la 1-cyclohexyl-3-(1,4-diméthyl-naphtalén-2-ylméthyl)-pyrrolidin-2-one ;
la 3-(1-chloro-naphtalén-2-ylméthyl)-1-cyclohexyl-pyrrolidin-2-one ;
la 1-cyclohexyl-3-(6-hydroxy-naphtalén-2-ylméthyl)-pyrrolidin-2-one ;
la 1-(cis-4-hydroxy-cyclohexyl)-3-(6-méthoxy-naphtalén-2-ylméthyl)-pyrrolidin-2-one ;
la 1-(4-hydroxy-cyclohexyl)-3-(6-méthoxy-naphtalén-2-ylméthyl)-pyrrolidin-2-one ;
la 1-(*cis*)-(4-hydroxy-cyclohexyl)-3-naphtalén-2-ylméthyl-pyrrolidin-2-one ;
la 1-(4-hydroxy-cyclohexyl)-3-naphtalén-2-ylméthyl-pyrrolidin-2-one ;
le sel chlorhydrate de 1-cyclohexyl-3-[6-(2-pipéridin-1-yl-éthoxy)-naphtalén-2-ylméthyl]-pyrrolidin-2-one ;
le sel chlorhydrate de 1-cyclohexyl-3-[6-(3-diméthylamino-propoxy)-naphtalén-2-ylméthyl]-pyrrolidin-2-one ;
l'ester méthylique d'acide 3-[6-(1-cyclohexyl-2-oxo-pyrrolidin-3-ylméthyl)-naphtalén-2-yloxyméthyl]-benzoïque ;
l'acide 3-[6-(1-cyclohexyl-2-oxo-pyrrolidin-3-ylméthyl)-naphtalén-2-yloxyméthyl]-benzoïque ;
le sel trifluoroacétate de 1-cyclohexyl-3-{6-[3-(4-méthyl-pipérazine-1-carbonyl)-benzyloxy]-naphtalén-2-ylméthyl}-pyrrolidin-2-one ;
la 1-cyclohexyl-3-{6-[3-(4-méthyl-pipérazine-1-carbonyl)-benzyloxy]-naphtalén-2-ylméthyl}-pyrrolidin-2-one ;
la 3-(1-chloro-naphtalén-2-ylméthyl)-1-(*cis*-4-hydroxy-cyclohexyl)-pyrrolidin-2-one ;
la 3-(1-chloro-naphtalén-2-ylméthyl)-1-(4-hydroxy-cyclohexyl)-pyrrolidin-2-one ;
la 1-cyclohexyl-3-(4-méthoxy-naphtalén-1-ylméthyl)-pyrrolidin-2-one ;
la 1-cyclohexyl-3-(2-méthoxy-naphtalén-1-ylméthyl)-pyrrolidin-2-one ;
la 3-(5-bromo-6-méthoxy-naphtalén-2-ylméthyl)-1-cyclohexyl-pyrrolidin-2-one ;
la 1-cyclohexyl-3-(7-méthoxy-naphtalén-2-ylméthyl)-pyrrolidin-2-one ;
la 3-(6-bromo-naphtalén-2-ylméthyl)-1-cyclohexyl-pyrrolidin-2-one ;
la 3-((3-chloronaphtalén-2-yl)méthyl)-1-cyclohexylpyrrolidin-2-one ;
la 1-cyclohexyl-3-((3-méthoxynaphtalén-1-yl)méthyl)pyrrolidin-2-one ;
la 3-((5-bromonaphtalén-2-yl)méthyl)-1-cyclohexylpyrrolidin-2-one ;
la 3-((1-chloro-6-méthoxynaphtalén-2-yl)méthyl)-1-cyclohexylpyrrolidin-2-one ;
la 1-cyclohexyl-3-(2-hydroxy-naphtalén-1-ylméthyl)-pyrrolidin-2-one ;
la 3-(5-bromo-6-hydroxy-naphtalén-2-ylméthyl)-1-cyclohexyl-pyrrolidin-2-one ;
la 1-cyclohexyl-3-(7-hydroxy-naphtalén-2-ylméthyl)-pyrrolidin-2-one ;
la 1-cyclohexyl-3-(6-isopropoxy-naphtalén-2-ylméthyl)-pyrrolidin-2-one ;
la 1-cyclohexyl-3-((7-isopropoxynaphtalén-2-yl)méthyl)pyrrolidin-2-one ;
la 1-cyclohexyl-3-naphtalén-2-ylméthyl-pipéridin-2-one ;
la 1-cyclohexyl-3-méthyl-3-naphtalén-1-ylméthyl-pyrrolidin-2-one ;
la *trans*-3-(3-chloro-naphtalén-2-ylméthyl)-1-(4-hydroxy-cyclohexyl)-pipéridin-2-one ;
la 3-(3-chloro-naphtalén-2-ylméthyl)-1-(4-hydroxy-cyclohexyl)-pipéridin-2-one ;
la *trans*-1-(4-hydroxy-cyclohexyl)-3-naphtalén-2-ylméthyl-pipéridin-2-one ;
la 1-(4-hydroxy-cyclohexyl)-3-naphtalén-2-ylméthyl-pipéridin-2-one ;
la *trans*-1-(4-hydroxy-cyclohexyl)-3-(6-méthoxy-naphtalén-2-ylméthyl)-pipéridin-2-one ;

la 1-(4-hydroxy-cyclohexyl)-3-(6-méthoxy-naphtalén-2-ylméthyl)-pipéridin-2-one ;
la *cis*-3-(1-chloro-naphtalén-2-ylméthyl)-1-(4-hydroxy-cyclohexyl)-pipéridin-2-one ;
la 3-(1-chloro-naphtalén-2-ylméthyl)-1-(4-hydroxy-cyclohexyl)-pipéridin-2-one ;
la *cis*-1-(4-hydroxy-cyclohexyl)-3-(6-méthoxy-naphtalén-2-ylméthyl)-pipéridin-2-one ;
la 1-(4-hydroxy-cyclohexyl)-3-(6-méthoxy-naphtalén-2-ylméthyl)-pipéridin-2-one ;
la *cis*-1-(4-hydroxy-cyclohexyl)-3-naphtalén-2-ylméthyl-pipéridin-2-one ;
la 1-(4-hydroxy-cyclohexyl)-3-naphtalén-2-ylméthyl-pipéridin-2-one ;
la *cis*-1-(4-hydroxy-cyclohexyl)-3-(7-méthoxy-naphtalén-2-ylméthyl)-pipéridin-2-one ;
la 1-(4-hydroxy-cyclohexyl)-3-(7-méthoxy-naphtalén-2-ylméthyl)-pipéridin-2-one ;
la *cis*-1-(4-hydroxy-cyclohexyl)-3-(3-méthoxy-naphtalén-1-ylméthyl)-pipéridin-2-one ;
la 1-(4-hydroxy-cyclohexyl)-3-(3-méthoxy-naphtalén-1-ylméthyl)-pipéridin-2-one ;
la 1-cyclohexyl-3-(2-méthoxy-naphtalén-1-ylméthyl)-pipéridin-2-one ;
la 1-cyclohexyl-3-(3-méthoxy-naphtalén-1-ylméthyl)-pipéridin-2-one ;
la 3-((6-(3-aminophényl)naphtalén-2-yl)méthyl)-1-cyclohexylpyrrolidin-2-one ;
la 1-cyclohexyl-3-((6-(6-fluoropyridin-3-yl)naphtalén-2-yl)méthyl)pyrrolidin-2-one ;
la 1-cyclohexyl-3-((6-(2,6-dichlorophényl)naphtalén-2-yl)méthyl)pyrrolidin-2-one ;
la 1-cyclohexyl-3-((6-(2,3-dichlorophényl)naphtalén-2-yl)méthyl)pyrrolidin-2-one ;
la 1-cyclohexyl-3-((6-(2-fluoropyridin-4-yl)naphtalén-2-yl)méthyl)pyrrolidin-2-one ;
la 1-cyclohexyl-3-((6-(6-méthoxypyridin-3-yl)naphtalén-2-yl)méthyl)pyrrolidin-2-one ;
le 4-(6-((1-cyclohexyl-2-oxopyrrol idin-3-yl)méthyl)naphtalén-2-yl)benzoate de méthyle ;
la 1-cyclohexyl-3-((7-(2-hydroxyphényl)naphtalén-2-yl)méthyl)pyrrolidin-2-one ;
la 1-cyclohexyl-3-((5-(2-hydroxyphényl)naphtalén-2-yl)méthyl)pyrrolidin-2-one ;
la 1-cyclohexyl-3-((5-vinylnaphtalén-2-yl)méthyl)pyrrolidin-2-one ;
la 1-cyclohexyl-3-((7-éthylnaphtalén-2-yl)méthyl)pyrrolidin-2-one ;
la 1-cyclohexyl-3-((5-éthylnaphtalén-2-yl)méthyl)pyrrolidin-2-one ;
le trifluorométhanesulfonate de 7-((1-cyclohexyl-2-oxopyrrolidin-3-yl)méthyl)naphtalén-2-yle ;
le 4-(6-((1-cyclohexyl-2-oxopyrrolidin-3-yl)méthyl)naphtalén-1-yl)benzoate de méthyle;
l'acide 4-(6-((1-cyclohexyl-2-oxopyrrolidin-3-yl)méthyl)naphtalén-1-yl)benzoïque ;
le 4-(7-((1-cyclohexyl-2-oxopyrrol idin-3-yl)méthyl)naphtalén-2-yl)benzoate de méthyle;
l'acide 4-(7-((1-cyclohexyl-2-oxopyrrolidin-3-yl)méthyl)naphtalén-2-yl)benzoïque ;
la 1-cydohexyl-3-((5-(2-fluoropyridin-4-yl)naphtalén-2-yl)méthyl)pyrrolidin-2-one ;
la 1-cyclohexyl-3-((7-(2-fluoropyridin-4-yl)naphtalén-2-yl)méthyl)pyrrolidin-2-one ;
la 3-(4-bromo-naphtalén-1-ylméthyl)-1-cyclohexyl-pyrrolidin-2-one ;
l'ester méthylique d'acide 4-[4-(1-cyclohexyl-2-oxo-pyrrolidin-3-ylméthyl)naphtalén-1-yl]-benzoïque ;
la (R)-3-(4-bromo-naphtalén-1-ylméthyl)-1-cyclohexyl-pyrrolidin-2-one ;
la 3-(4-bromo-naphtalén-1-ylméthyl)-1-cyclohexyl-pyrrolidin-2-one ;
l'ester méthylique d'acide (R)-4-[4-(1-cyclohexyl-2-oxo-pyrrolidin-3-ylméthyl)-naphtalén-1-yl]-benzoïque ;
l'ester méthylique d'acide 4-[4-(1-cyclohexyl-2-oxo-pyrrolidin-3-ylméthyl)-naphtalén-1-yl]-benzoïque ;
l'acide (R)-4-[4-(1-cyclohexyl-2-oxo-pyrrolidin-3-ylméthyl)-naphtalén-1-yl]-benzoïque ;
l'acide 4-[4-(1-cyclohexyl-2-oxo-pyrrolidin-3-ylméthyl)-naphtalén-1-yl]-benzoïque ; et
la 3-((1-chloro-6-hydroxynaphtalén-2-yl)méthyl)-1-cyclohexylpyrrolidin-2-one.

**11.** Composition pharmaceutique qui comprend un composé selon l'une quelconque des revendications 1 à 10 et un support pharmaceutiquement acceptable.

**12.** Composé selon la formule 1, ou un sel pharmaceutiquement acceptable de celui-ci, selon l'une quelconque des revendications 1 à 10, pour une utilisation en tant que médicament.

**13.** Utilisation d'un composé selon l'une quelconque des revendications 1 à 10 ou d'un sel pharmaceutiquement acceptable de celui-ci, pour la fabrication d'un médicament destiné au traitement d'un syndrome métabolique.

**14.** Utilisation d'un composé selon l'une quelconque des revendications 1 à 10, ou d'un sel pharmaceutiquement acceptable de celui-ci, pour la fabrication d'un médicament destiné au traitement du diabète.

**15.** Utilisation d'un composé selon l'une quelconque des revendications 1 à 10, ou d'un sel pharmaceutiquement acceptable de celui-ci, pour la fabrication d'un médicament destiné au traitement de (1) l'hyperglycémie, (2) une faible tolérance au glucose, (3) une résistance à l'insuline, (4) l'obésité, (5) des troubles lipidiques, (6) une dyslipidémie, (7) une hyperlipidémie, (8) une hypertriglycéridémie, (9) une hypercholestérolémie, (10) des taux faibles de HDL,

(11) des taux élevés de LDL, (12) une athérosclérose et ses séquelles, (13) une resténose vasculaire, (14) une pancréatite, (15) une obésité abdominale, (16) une maladie neurodégénérative, (17) une rétinopathie, (18) une néphropathie, (19) une neuropathie, (20) un syndrome métabolique, et d'autres maladies et troubles où une résistance à l'insuline est une composante.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63748804 P
- EP 838448 A


### Non-patent literature cited in the description


- **J. JACQUES et al.** Enantiomers, Racemates, and Resolutions. John Wiley and Sons, Inc, 1981
- **E.L. ELIEL ; S.H. WILEN.** Stereochemistry of Organic Compounds. Wiley-Interscience, 1994
- Nomenclature of Organic Compounds: Principles and Practice. 1974, 103-120
- **HULLET, P. et al.** *Can. J. Chem.,* 1976, vol. 54, 1098-1104
- **GOUMRI-MAGNET et al.** *J. Org. Chem.,* 1999, vol. 64, 3741-3744
- *J. Med Chem.,* 1988, vol. 31, 72-83
- *J. Med. Chem.,* 1991, vol. 34, 887-900
- **LIEBIGS.** *Ann. Chemie.,* 1983, 165-180
- *Chem. Pharm. Bull.,* 1990, vol. 38, 393-399
- *J. Am. Chem. Soc.,* 1947, vol. 69, 715-716
- *Syn Lett.,* 1994, 81-83
- *Bioorg. Med. Chem. Lett.,* 2003, 2035-2040
- **GREENE.** Protective Groups in Organic Synthesis. John Wiley & Sons
- *J. Med. Chem,* 1987, vol. 30, 1995-1998
- *Tetrahedron,* 1982, 2347
- *J. Org. Chem.,* 2002, 6216-6219
- *Chem. Ber.,* 1958, 1354-1356
- **J. LABEL.** *Compounds and Radiopharm.,* 1987, 851-858
- **JOHN L. KICE ; HARVINDER LOTEY.** *J. Org. Chem,* 1989, vol. 54 (15), 3596-602
- *J. Med. Chem.,* 1996, vol. 39 (16), 3089-3095
- *J. Am. Chem. Soc.,* 2000, vol. 122 (29), 6935-6949
- *J. Org. Chem.,* 1957, 1473
- Remington's Pharmaceutical Sciences. Mack Publishing Co, 1990